# EUROPEAN PATENT SPECIFICATION

(11) **EP 2 470 173 B1**
(45) Date of publication and mention of the grant of the patent: **27.04.2016**
(21) Application number: 10812593.1
(22) Date of filing: 25.08.2010
(51) Int. Cl.: A61K 31/337, A61K 9/20

(54) **COMBINATION THERAPY WITH NANOPARTICLE COMPOSITIONS OF TAXANE AND HEDGEHOG INHIBITORS**
KOMBINATIONSTHERAPIE MIT NANOPARTIKELZUSAMMENSETZUNGEN AUS TAXAN UND HEDGEHOG-HEMMERN
PLURITHÉRAPIE AVEC DES COMPOSITIONS NANOPARTICULAIRES DE TAXANE ET DES INHIBITEURS HEDGEHOG

(30) Priority: 25.08.2009 US 236813 P
(43) Date of publication of application: 04.07.2012
(73) Proprietor: Abraxis BioScience, LLC, Los Angeles, CA 90025 (US)
(72) Inventor: TAO, Chunlin, Los Angeles CA 90025 (US); DESAI, Neil, P., Los Angeles CA 90025 (US); SOON-SHIONG, Patrick, Los Angeles CA 90049 (US)
(74) Representative: Jones Day
(86) International application number: PCT/US2010/046684
(87) International publication number: WO 2011/025838

(56) References cited:
- WO-A1-2006/039569
- WO-A1-2009/086324
- US-A1- 2007 116 761
- US-A1- 2007 281 040
- US-A1- 2009 123 932
- HAWKINS ET AL: "Protein Nanoparticles as drug carriers in clinical medicine.", ADVANCED DRUG DELIVERY, vol. 60, 7 February 2008 (2008-02-07), - 7 February 2008 (2008-02-07), pages 876-885, XP002696596,
- LOBO ET AL: "Paclitaxel albumin bound particles in combination with bevacizumab with or without gemcitabine.", BIOMEDICINE AND PHARMACOTHERAPY., vol. 61, 12 September 2007 (2007-09-12), - 12 September 2007 (2007-09-12), pages 531-533, XP002696597,
- LI ET AL: ADV. DRUG. DELIV. REV, vol. 60, no. 8, 22 May 2008 (2008-05-22), - 22 May 2008 (2008-05-22), pages 886-898,
- LANGER ET AL: J.THORACIC ONCOL., vol. 3, no. 6, 1 June 2008 (2008-06-01), - 1 June 2008 (2008-06-01), pages 623-630,

## Description

### CROSS REFERENCE TO RELATED APPLICATIONS

The patent application claims the benefit of priority under 35 U.S.C. § 119(e) of U.S. Provisional Application No. 61/236,813 filed August 25, 2009.

### TECHNICAL FIELD

The present invention relates to methods and compositions for the treatment of proliferative diseases comprising the administration of a taxane, specifically a nanoparticle composition of taxane, and another therapeutic agent for the treatment of proliferative diseases.

### BACKGROUND

About 1.4 million new cases of cancer will be diagnosed in the United States in 2005, and more than 550,000 people will die of the disease. American Cancer Society, Inc. Cancer Facts and Figures 2005. Atlanta: American Cancer Society, Inc., 2005. Cancer is a leading cause of death world wide. Cancer is the second leading cause of death in this country. About 64 percent of all people diagnosed with cancer will be alive 5 years after diagnosis. The most common types of cancer, as defined by an estimate annual incidence of 40,000 cases or more for 2010 as reported by American Cancer Society: Cancer Facts and Figures 2010. Atlanta, Ga: American Cancer Society, 2010, are bladder cancer, lung cancer, breast cancer, melanoma, colorectal cancer, non-Hodgkin lymphoma, endometrial cancer, pancreatic cancer, kidney (renal cell) cancer, prostate cancer, leukemia, and thyroid cancer. Despite significant advances in the field of chemotherapy, many of the most prevalent forms of cancer still resist chemotherapeutic intervention.

Among all the cancers, pancreatic cancer ranks number four as a leading cause of death in the United States. It was estimated that, in 2009 alone, about 42,470 individuals in the United States will be diagnosed with pancreatic cancer. Because it is usually diagnosed at an advanced stage, the survival rate is poor compared with that of other types of cancer. Less than 5 percent of those diagnosed with pancreatic cancer are still alive five years after diagnosis. Complete remission of the disease is extremely rare. Despite effort in developing therapeutics for pancreatic cancer, the overall pancreatic cancer incidence and mortality rates have changed very little in the past three decades.

Taxanes (such as paclitaxel and docetaxel) have been shown to have significant antineoplastic and anticancer effects in a wide variety of cancers. For example, paclitaxel acts by interfering with the normal function of microtubule breakdown. Paclitaxel binds to the β subunit of tubulin, the building blocks of microtubules, causing hyper-stabilization of the microtubule structures. The resulting paclitaxel/microtubule structure is unable to disassemble, thereby arresting mitosis and inhibiting angiogenesis. The poor aqueous solubility for the taxanes, however, presents significant challenges for developing effective taxane-based cancer therapeutics. Furthermore, the interaction of different taxane formulations with other therapeutic agents in the combination therapy context remains to be studied.

Albumin-based nanoparticle compositions have been developed as a drug delivery system for delivering substantially water insoluble drugs such as taxanes. See, for example, U.S. Pat. Nos. 5,916,596; 6,506,405; 6,749,868, and 6,537,579 and also in U.S. Pat. Pub. Nos. 2005/0004002 and 2007/0082838. The albumin-based nanoparticle technology utilizes the natural properties of the protein albumin to transport and deliver substantially water insoluble drugs to the site of disease. These nanoparticles are readily incorporated into the body's own transport processes and are able to exploit the tumors' attraction to albumin, enabling the delivery of higher concentrations of the active drug encapsulated in the nanoparticles to the target site. In addition, the albumin-based nanoparticle technology offers the ability to improve a drug's solubility by avoiding the need for toxic chemicals, such as solvents, in the administration process, thus potentially improving safety through the elimination of solvent-related side effects.

More effective treatments for cancer, such as pancreatic cancer, are needed.

### BRIEF SUMMARY OF THE INVENTION

The present invention provides methods for the treatment of proliferative diseases such as cancer. The invention provides combination therapy methods of treating a proliferative disease (such as cancer), comprising administering to the individual a) an effective amount of a composition comprising nanoparticles comprising a taxane and a carrier protein (such as albumin), and b) an effective amount of a hedgehog inhibitor. In some embodiments, the invention provides a method of treating a proliferative disease (such as cancer) in an individual comprising administering to the individual a) an effective amount of a composition comprising nanoparticles comprising paclitaxel and an albumin (such as Abraxane®), and b) an effective amount of a hedgehog inhibitor.

The methods described here are particularly useful for individuals having cancer, including for example any one of the following cancers: basal cell carcinoma, medulloblastoma, glioblastoma, multiple myeloma, chronic myelogenous leukemia (CML), acute myelogenous leukemia, pancreatic cancer, lung cancer (small cell lung cancer and non-small cell lung cancer), esophageal cancer, stomach cancer, biliary cancer, prostate cancer, liver cancer, hepatocellular cancer, gastrointestinal cancer, gastric cancer, and ovarian and bladder cancer. In some embodiments, the cancer is selected from the group consisting of pancreas ductal adenocarcinoma, colon adenocarcinoma, and ovary cystadenocarcinoma.

In some embodiments, the cancer is pancreatic cancer, including for example pancreatic adenocarcinoma, pancreatic adenosquamous carcinoma, pancreatic squamous cell carcinoma, and pancreatic giant cell carcinoma. Thus, for example, in some embodiments, there is provided a method of treating pancreatic cancer, comprising administering to the individual a) an effective amount of a composition comprising nanoparticles comprising a taxane and a carrier protein (such as albumin), and b) an effective amount of a hedgehog inhibitor. In some embodiments, there is provided a method treating pancreatic cancer, comprising administering to the individual a) an effective amount of a composition comprising nanoparticles comprising paclitaxel and an albumin (such as Abraxane®), and b) an effective amount of a hedgehog inhibitor. In some embodiments, the pancreatic cancer is exocrine pancreatic cancer. In some embodiments, the pancreatic cancer is endocrine pancreatic cancer (such as islet cell carcinoma). In some embodiments, the pancreatic cancer is advanced metastatic pancreatic cancer.

The methods described herein generally require the administration of a hedgehog inhibitor. In some embodiments, the hedgehog inhibitor directly targets Smoothened. In some embodiments, the hedgehog inhibitor is an isoquinoline or quinazoline compound, such as a compound according to any of the general formulas (I), (Ia), (Ib), or (Ic). In some embodiments, the hedgehog inhibitor is selected from the compounds provided in Table 1. In some embodiments, the hedgehog inhibitor is selected from the compounds provided in Table 4. In some embodiments, the hedgehog inhibitor is a tetrazine compound, such as a compound according to any of the general formulas (II), (IIa), (IIb), or (IIc). In some embodiments, the inhibitor is selected from the compounds provided in Table 2. In some embodiments, the inhibitor is selected from the compounds provided in Table 5. In some embodiments, the hedgehog inhibitor is selected from the compounds provided in Table 3. In some embodiments, the hedgehog inhibitor is Jervine, GANT61, purmorphamine, SAG, SANT-2, tomatidine, zerumbone, or derivatives thereof. In some embodiments, the hedgehog inhibitor is GDC-0449, XL139, IPI926, IPI609 (IPI269609), or LDE225. In some embodiments, the hedgehog inhibitor is a cyclopamine or derivative thereof. In some embodiments, the hedgehog inhibitor is provided in the form of nanoparticles (such as nanoparticles comprising a hedgehog inhibitor and a carrier protein (such as albumin)).

The composition comprising nanoparticles of taxane (also referred to as "nanoparticle taxane composition") and the hedgehog inhibitor can be administered simultaneously, either in the same composition or in separate compositions. Alternatively, the nanoparticle taxane composition and the hedgehog inhibitor are administered sequentially, i.e., the nanoparticle taxane composition is administered either prior to or after the administration of the hedgehog inhibitor. In some embodiments, the nanoparticle taxane composition is administered prior to the administration of the hedgehog inhibitor. In some embodiments, the nanoparticle taxane composition is administered after the administration of the hedgehog inhibitor.

In some embodiments, the administration of the nanoparticle taxane composition and the hedgehog inhibitor are concurrent, i.e., the administration period of the nanoparticle taxane composition and that of the hedgehog inhibitor overlap with each other. In some embodiments, the nanoparticle taxane composition is administered for at least one cycle (for example, at least any of 2, 3, or 4 cycles) prior to the administration of the hedgehog inhibitor. In some embodiments, the hedgehog inhibitor is administered for at least any of one, two, three, or four weeks.

In some embodiments, the administrations of the nanoparticle taxane composition and the hedgehog inhibitor are non-concurrent. For example, in some embodiments, the administration of the nanoparticle taxane composition is terminated before the hedgehog inhibitor is administered. In some embodiments, the administration of the hedgehog inhibitor is terminated before the nanoparticle taxane composition is administered.

In some embodiments, the method further comprises administering an effective amount of an antimetabolite (such as gemcitabine). Thus, for example, in some embodiments, there is provided a method of treating a proliferative disease (such as cancer), comprising administering to the individual a) an effective amount of a composition comprising nanoparticles comprising a taxane and a carrier protein (such as albumin), b) an effective amount of a hedgehog inhibitor; and c) an effective amount of an antimetabolite (such as gemcitabine). In some embodiments, there is provided a method treating a proliferative disease (such as cancer), comprising administering to the individual a) an effective amount of a composition comprising nanoparticles comprising paclitaxel and an albumin (such as Abraxane®), b) an effective amount of a hedgehog inhibitor, and c) an effective amount of gemcitabine.

In some embodiments, there is provided a method of treating pancreatic cancer, comprising administering to the individual a) an effective amount of a composition comprising nanoparticles comprising a taxane and a carrier protein (such as albumin), b) an effective amount of a hedgehog inhibitor; and c) an effective amount of an antimetabolite (such as gemcitabine). In some embodiments, there is provided a method of treating pancreatic cancer, comprising administering to the individual a) an effective amount of a composition comprising nanoparticles comprising paclitaxel and an albumin (such as Abraxane®), b) an effective amount of a hedgehog inhibitor, and c) an effective amount of gemcitabine.

In some embodiments, there is provided a method of treating pancreatic cancer, comprising administering to the individual a) an effective amount of a composition comprising nanoparticles comprising paclitaxel and albumin (such as Abraxane®), b) an effective amount of an isoquinoline or quinazoline compound (such as a compound of any of general formulas (I), (Ia), (Ib), or (Ic), or a compound provided in Table 1, or a compound provided in Table 4), and c) an effective amount of gemcitabine. In some embodiments, there is provided a method of treating pancreatic cancer, comprising administering to the individual a) an effective amount of a composition comprising nanoparticles comprising paclitaxel and albumin (such as Abraxane®), b) an effective amount of a tetrazine compound (such as a compound of any of general formulas (II), (IIa), (IIb), or (IIc), or a compound provided in Table 2, or a compound provided in Table 5), and c) an effective amount of gemcitabine. In some embodiments, there is provided a method of treating pancreatic cancer, comprising administering to the individual a) an effective amount of a composition comprising nanoparticles comprising paclitaxel and albumin (such as Abraxane®), b) an effective amount of a compound provided in Table 3, and c) an effective amount of gemcitabine. In some embodiments, there is provided a method of treating pancreatic cancer, comprising administering to the individual a) an effective amount of a composition comprising nanoparticles comprising paclitaxel and albumin (such as Abraxane®), b) an effective amount of a compound selected from Jervine, GANT61, purmorphamine, SAG, SANT-2, tomatidine, zerumbone, and derivatives thereof, and c) an effective amount of gemcitabine. In some embodiments, there is provided a method of treating pancreatic cancer, comprising administering to the individual a) an effective amount of a composition comprising nanoparticles comprising paclitaxel and albumin (such as Abraxane®), b) an effective amount of a compound selected from GDC-0449, XL139, IPI926, IPI609 (IPI269609), and LDE225, and c) an effective amount of gemcitabine. In some embodiments, there is provided a method of treating pancreatic cancer, comprising administering to the individual a) an effective amount of a composition comprising nanoparticles comprising paclitaxel and albumin (such as Abraxane®), b) an effective amount of a cyclopamine or derivative thereof (such as IPI926), and c) an effective amount of gemcitabine.

The methods described herein generally comprise administration of a composition comprising nanoparticles comprising a taxane and a carrier protein. In some embodiments, the nanoparticle taxane composition comprises nanoparticles comprising paclitaxel and an albumin. In some embodiments, the nanoparticles in the composition described herein have an average diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least about any one of 60%, 70%, 80%, 90%, 95%, or 99%) of all the nanoparticles in the composition have a diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least any one of 60%, 70%, 80%, 90%, 95%, or 99%) of all the nanoparticles in the composition fall within the range of about 20 to about 200 nm, including for example any one of about 30 to about 180 nm, about 40 to about 150, about 50 to about 120, and about 60 to about 100 nm.

In some embodiments, the carrier protein has sulfhydryl groups that can form disulfide bonds. In some embodiments, at least about 5% (including for example at least about any one of 10%, 15%, 20%, 25%, 30%, 40%, 50%, 60%, 70%, 80%, or 90%) of the carrier protein in the nanoparticle portion of the composition are crosslinked (for example crosslinked through one or more disulfide bonds).

In some embodiments, the nanoparticles comprise the taxane (such as paclitaxel) coated with a carrier protein, such as albumin (e.g., human serum albumin). In some embodiments, the composition comprises taxane in both nanoparticle and non-nanoparticle forms, wherein at least about any one of 50%, 60%, 70%, 80%, 90%, 95%, or 99% of the taxane in the composition are in nanoparticle form. In some embodiments, the taxane in the nanoparticles constitutes more than about any one of 50%, 60%, 70%, 80%, 90%, 95%, or 99% of the nanoparticles by weight. In some embodiments, the nanoparticles have a non-polymeric matrix. In some embodiments, the nanoparticles comprise a core of taxane that is substantially free of polymeric materials (such as polymeric matrix).

In some embodiments, the nanoparticle taxane composition is substantially free (such as free) of surfactants (such as Cremophor®, Tween 80, or other organic solvents used for the administration of taxanes). In some embodiments, the nanoparticle taxane composition contains less than about any one of 20%, 15%, 10%, 7.5%, 5%, 2.5%, or 1% organic solvent. In some embodiments, the weight ratio of carrier protein (such as albumin) and taxane in the nanoparticle taxane composition is about 18:1 or less, such as about 15:1 or less, for example about 10:1 or less. In some embodiments, the weight ratio of carrier protein (such as albumin) and taxane in the composition falls within the range of any one of about 1:1 to about 18:1, about 1:1 to 9:1, about 2:1 to about 15:1, about 3:1 to about 13:1, about 4:1 to about 12:1, about 5:1 to about 10:1. In some embodiments, the weight ratio of carrier protein and taxane in the nanoparticle portion of the composition is about any one of 1:2, 1:3, 1:4, 1:5, 1:6, 1:7, 1:8, 1:9, 1:10, 1:11, 1:12, 1:13, 1:14, 1:15, or less.

In some embodiments, the particle composition comprises one or more of the above characteristics.

In some embodiments, the nanoparticle taxane composition is Abraxane®. Nanoparticle taxane compositions comprising other taxanes (such as docetaxel and ortataxel) may also comprise one or more of the above characteristics.

Also provided are kits and compositions useful for methods described herein.

These and other aspects and advantages of the present invention will become apparent from the subsequent detailed description and the appended claims. It is to be understood that one, some, or all of the properties of the various embodiments described herein may be combined to form other embodiments of the present invention.

### BRIEF DESCRIPTION OF FIGURES

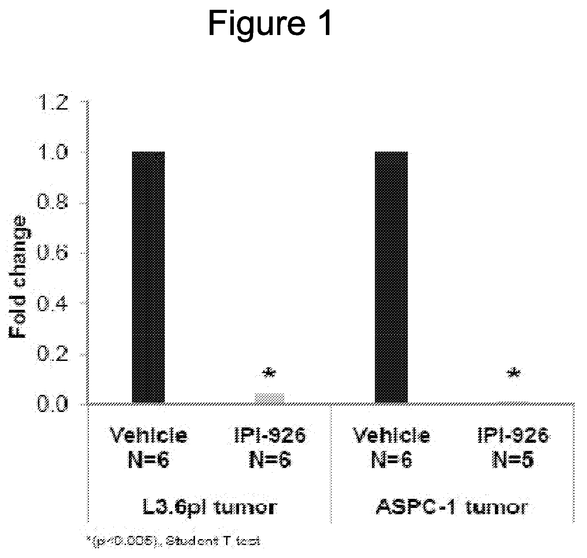

Figure 1 shows the effect of Hedgehog inhibitor IPI-926 on murine Gli1 mRNA expression in pancreatic cancer xenograft models of L3.6pl and ASPC-1.

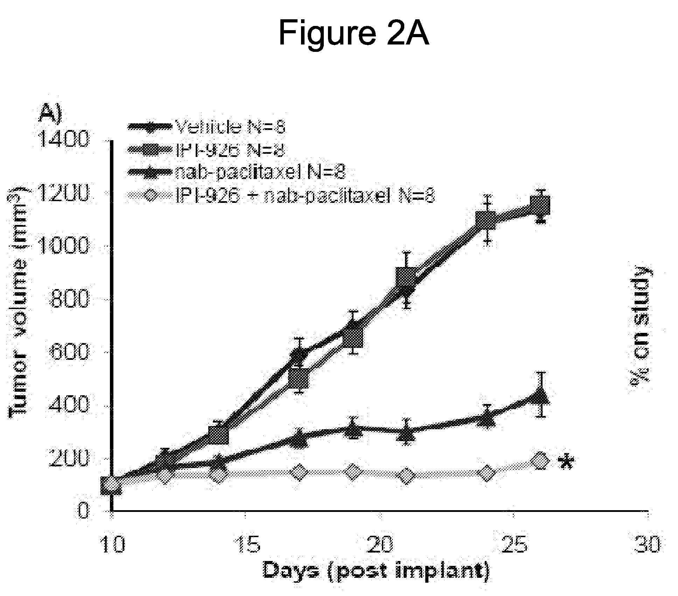

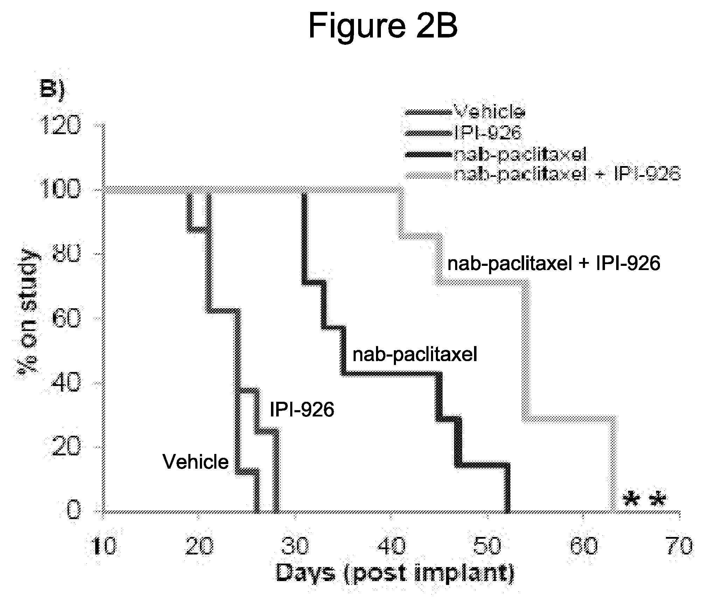

Figure 2A shows the tumor volume (mm³) with the treatment of vehicle alone (N=8), IPI-926 (N=8), Abraxane® (also known as "nab-paclitaxel") (N=8) and Abraxane® + IPI-926 (N=8) in L3.6pl pancreatic xenograft model. Figure 2B shows the % of mice with L3.6pl pancreatic xenograft remaining on study with the treatment vehicle alone, IPI-926, Abraxane® and Abraxane® + IPI-926. Mice were taken off the study when the tumors reached 1000 mm³.

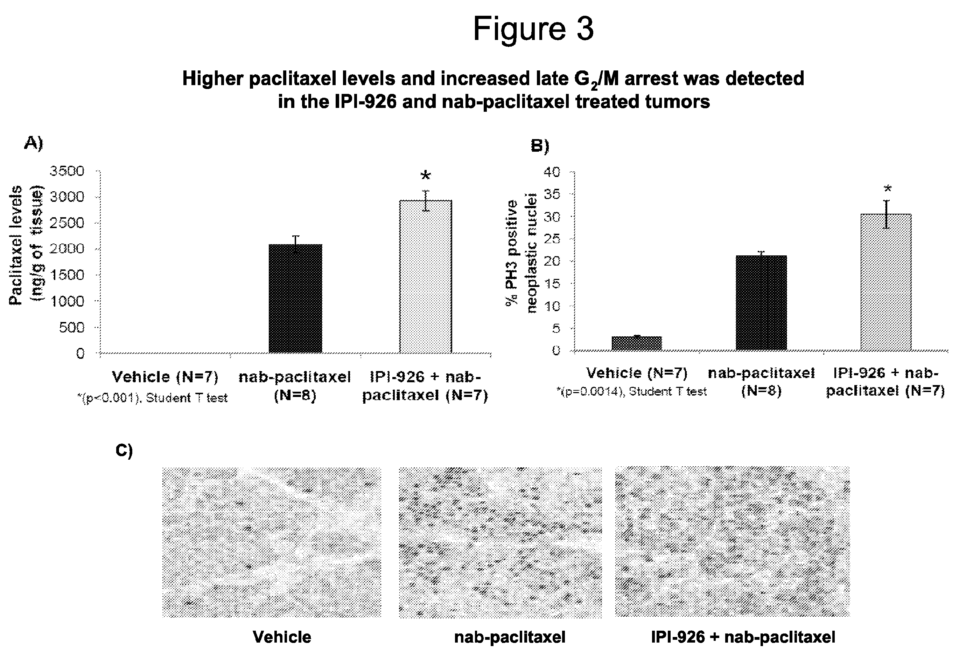

Figure 3 shows higher paclitaxel levels and increased late G2/M arrest in the IPI-926 and nab-paclitaxel treated tumors. Figure 3A shows the levels of paclitaxel in the L3.6pl tumors treated with vehicle, Abraxane® alone, and the combination of IPI-926 and Abraxane®. Figures 3B show the % phosphorylated histone H3 ("PH3") positive tumor cells treated with vehicle, Abraxane® alone, and the combination of IPI-926 and Abraxane®. Figure 3C shows the PH3 immunostaining of tumor cells treated with vehicle, Abraxane® alone, and the combination of IPI-926 and Abraxane® (200x).

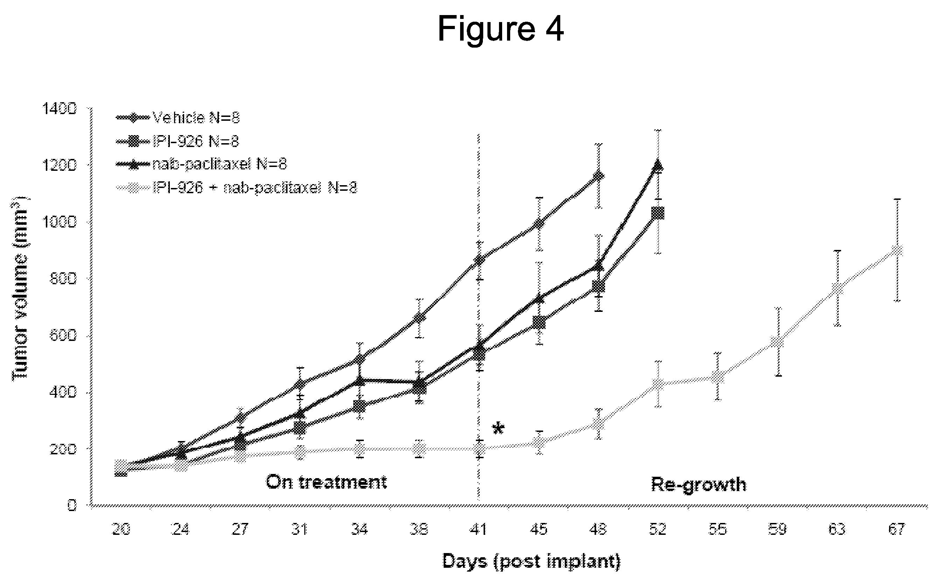

Figure 4 shows the tumor volume (mm³) with the treatment of vehicle alone (N=8), IPI-926 (N=8), Abraxane® (N=8) and Abraxane® + IPI-926 (N=8) in ASPC-1 pancreatic tumor model.

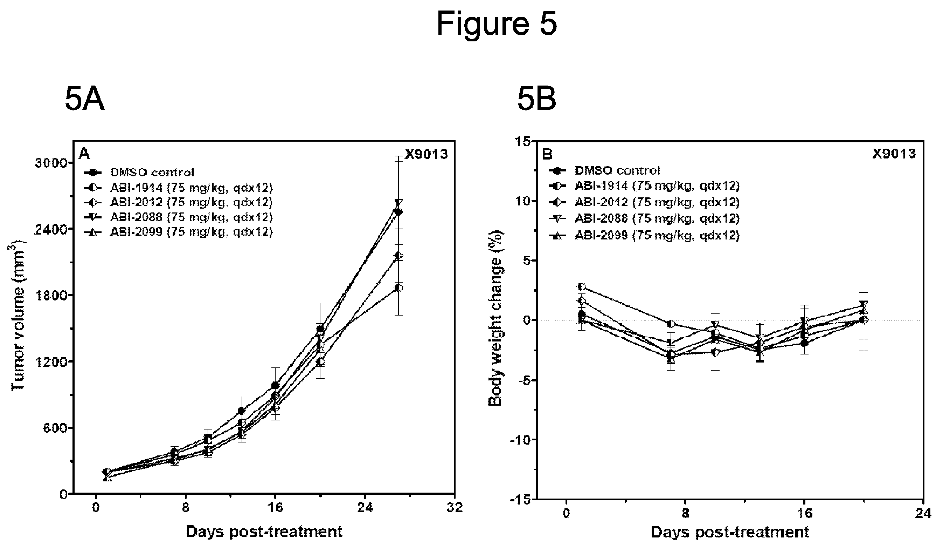

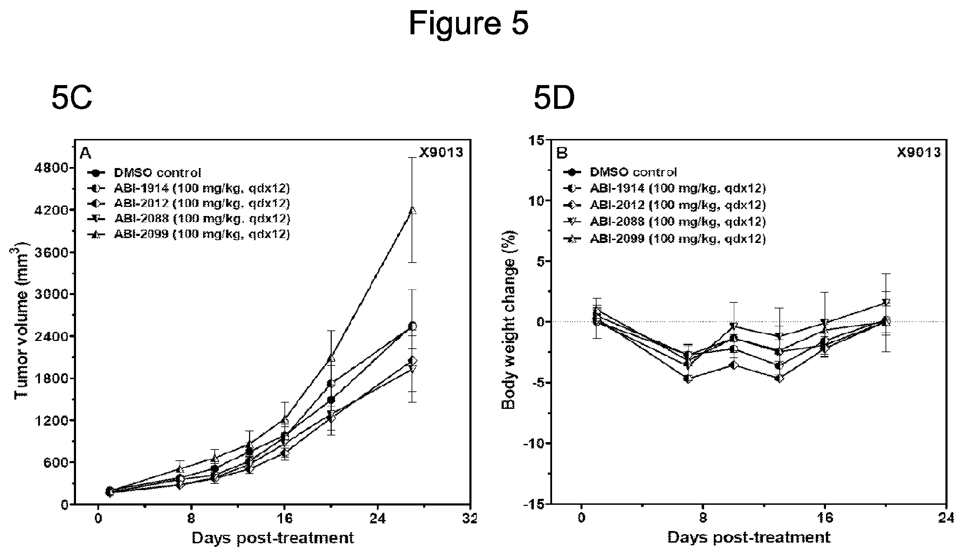

Figures 5A and 5B show the tumor volume (mm³) (Fig. 5A) and % body weight change (Fig. 5B) with the treatment of DMSO, ABI1914 (75 mg/kg, qdx12), ABI2012 (75 mg/kg, qdx12), ABI2088 (75 mg/kg, qdx12), and ABI2099 (75 mg/kg, qdx12). Figures 5C and 5D show the tumor volume (mm³) (Fig. 5C) and % body weight change (Fig. 5D) with the treatment of DMSO, ABI1914 (100 mg/kg, qdx12), ABI2012 (100 mg/kg, qdx12), ABI2088 (100 mg/kg, qdx12), and ABI2099 (100 mg/kg, qdx12) in HT29 xenograft model.

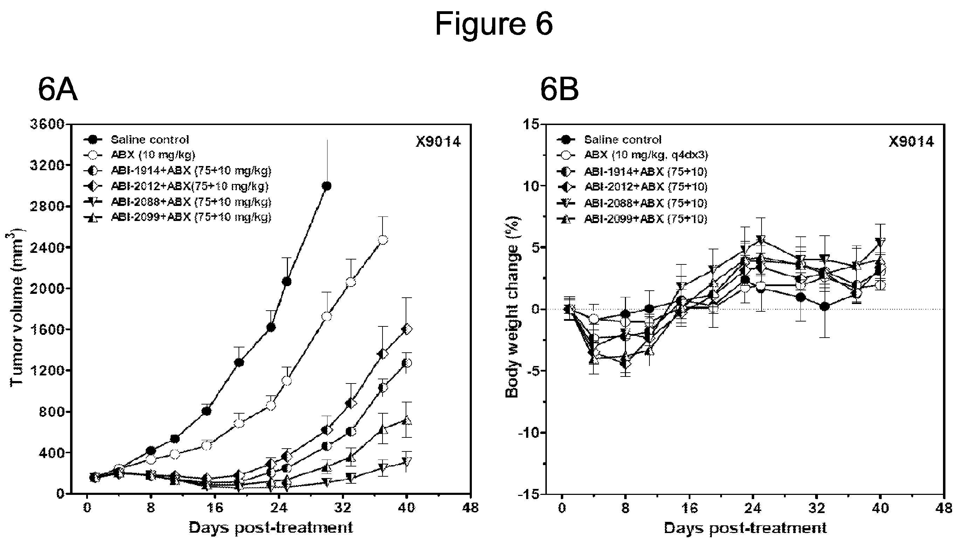

Figures 6A and 6B show the tumor volume (mm³) (Fig. 6A) and % body weight change (Fig. 6B) with the treatment of saline (q4dx3), Abraxane® (10 mg/kg, q4dx3), ABI1914 (75 mg/kg, qdx12) + Abraxane® (10 mg/kg, q4dx3), ABI2012 (75 mg/kg, qdx12) + Abraxane® (10 mg/kg, q4dx3), ABI2088 (75 mg/kg, qdx12) + Abraxane® (10 mg/kg, q4dx3), and ABI2099 (75 mg/kg, qdx12) + Abraxane® (10 mg/kg, q4dx3) in HT29 xenograft model.

### DETAILED DESCRIPTION OF THE INVENTION

The present invention provides methods of combination therapy comprising administration of nanoparticles comprising a taxane and a carrier protein (such as albumin) in conjunction with a hedgehog inhibitor, and optionally with an antimetabolite (such as gemcitabine). The methods described herein are generally useful for treatment of proliferative diseases, particularly cancer.

It is understood that aspect and embodiments of the invention described herein include "consisting" and/or "consisting essentially of" aspects and embodiments.

### Definitions

As used herein, "treatment" is an approach for obtaining beneficial or desired clinical results. For purposes of this invention, beneficial or desired clinical results include, but are not limited to, any one or more of: alleviation of one or more symptoms, diminishment of extent of disease, preventing or delaying spread (e.g., metastasis, for example metastasis to the lung or to the lymph node) of disease, preventing or delaying recurrence of disease, delay or slowing of disease progression, amelioration of the disease state, and remission (whether partial or total). Also encompassed by "treatment" is a reduction of pathological consequence of a proliferative disease. The methods of the invention contemplate any one or more of these aspects of treatment.

As used herein, a "proliferative disease" includes tumor disease (including benign or cancerous) and/or any metastases. A proliferative disease may include hyperproliferative conditions such as hyperplasias, fibrosis (especially pulmonary, but also other types of fibrosis, such as renal fibrosis), angiogenesis, psoriasis, atherosclerosis and smooth muscle proliferation in the blood vessels, such as stenosis or restenosis following angioplasty. In some embodiments, the proliferative disease is cancer. In some embodiments, the proliferative disease is a non-cancerous disease. In some embodiments, the proliferative disease is a benign or malignant tumor.

The term "effective amount" used herein refers to an amount of a compound or composition sufficient to treat a specified disorder, condition or disease such as ameliorate, palliate, lessen, and/or delay one or more of its symptoms. In reference to cancers or other unwanted cell proliferation, an effective amount comprises an amount sufficient to cause a tumor to shrink and/or to decrease the growth rate of the tumor (such as to suppress tumor growth) or to prevent or delay other unwanted cell proliferation. In some embodiments, an effective amount is an amount sufficient to delay development. In some embodiments, an effective amount is an amount sufficient to prevent or delay recurrence. An effective amount can be administered in one or more administrations.

The term "individual" refers to a mammal, including humans. An individual includes, but is not limited to, human, bovine, horse, feline, canine, rodent, or primate.

"Adjuvant setting" refers to a clinical setting in which an individual has had a history of a proliferative disease, particularly cancer, and generally (but not necessarily) been responsive to therapy, which includes, but is not limited to, surgery (such as surgical resection), radiotherapy, and chemotherapy. However, because of their history of the proliferative disease (such as cancer), these individuals are considered at risk of development of the disease. Treatment or administration in the "adjuvant setting" refers to a subsequent mode of treatment. The degree of risk (i.e., when an individual in the adjuvant setting is considered as "high risk" or "low risk") depends upon several factors, most usually the extent of disease when first treated.

"Neoadjuvant setting" refers to a clinical setting in which a method is carried out before the primary/definitive therapy.

"Hedgehog inhibitor" used herein refers to an agent that inhibits a hedgehog signaling pathway, for example by affecting the activity of one or more components of the hedgehog signaling pathway, either directly or indirectly.

As used herein, "hedgehog" refers generically to any of the mammalian homologs of the Drosophila hedgehog protein, and includes at least Sonic hedgehog (Shh), Desert hedgehog (Dhh) and Indian hedgehog (Ihh).

The terms "hedgehog signaling pathway" as used herein refers to the signaling cascade mediated by (or downstream of) hedgehog and its receptors which results in changes of gene expression and other phenotypic changes typical of hedgehog activity.

The term "component of the hedgehog signaling pathway" or "hedgehog signaling component" used interchangeably refers to molecules that participate in the hedgehog signaling pathway. A hedgehog signaling component frequently affects the transmission of the hedgehog signal in cells or tissues, thereby affecting the downstream gene expression levels and/or other phenotypic changes associated with hedgehog pathway activation.

The term "proteins" refers to polypeptides or polymers of amino acids of any length (including full length or fragments), which may be linear or branched, comprise modified amino acids, and/or be interrupted by non-amino acids. The term also encompasses an amino acid polymer that has been modified naturally or by intervention; for example, disulfide bond formation, glycosylation, lipidation, acetylation, phosphorylation, or any other manipulation or modification. Also included within this term are, for example, polypeptides containing one or more analogs of an amino acid (including, for example, unnatural amino acids, etc.), as well as other modifications known in the art.

A composition is "substantially free of Cremophor" or "substantially free of surfactant" if the amount of Cremophor or surfactant in the composition is not sufficient to cause one or more side effect(s) in an individual when the nanoparticle composition is administered to the individual.

"In conjunction with" used herein refers to administration of one treatment modality in addition to another treatment modality, such as administration of a nanoparticle composition described herein in addition to administration of the other agent to the same individual. As such, "in conjunction with" refers to administration of one treatment modality before, during, or after delivery of the other treatment modality to the individual.

Reference to "about" a value or parameter herein includes (and describes) embodiments that are directed to that value or parameter per se. For example, description referring to "about X" includes description of "X".

As used herein and in the appended claims, the singular forms "a," "or," and "the" include plural referents unless the context clearly dictates otherwise. It is understood that aspect and embodiments of the invention described herein include "consisting" and/or "consisting essentially of" aspects and embodiments.

### Methods of the present invention

The present invention provides methods of treating a proliferative disease (such as cancer) in an individual, comprising administering to the individual: a) an effective amount of a composition comprising nanoparticles comprising a taxane and a carrier protein (such as albumin); and b) an effective amount of a hedgehog inhibitor. In some embodiments, the taxane is paclitaxel (for example, in some embodiments, the nanoparticle composition is Abraxane®). In some embodiments, the proliferative disease is cancer. In some embodiments, there is provided a method of treating a tumor that is poorly perfused and/or poorly vascularized. In some embodiments, the cancer is pancreatic cancer.

The present invention provides methods for the treatment of proliferative diseases such as cancer. The invention provides combination therapy methods of treating a proliferative disease (such as cancer), comprising administering to the individual a) an effective amount of a composition comprising nanoparticles comprising a taxane and a carrier protein (such as albumin), and b) an effective amount of a hedgehog inhibitor. In some embodiments, the invention provides a method of treating a proliferative disease (such as cancer) in an individual comprising administering to the individual a) an effective amount of a composition comprising nanoparticles comprising paclitaxel and an albumin (such as Abraxane®), and b) an effective amount of a hedgehog inhibitor.

In some embodiments, there is provided a method of treating a proliferative disease (such as cancer), comprising administering to the individual a) an effective amount of a composition comprising nanoparticles comprising a taxane coated with a carrier protein (such as albumin), and b) an effective amount of a hedgehog inhibitor. In some embodiments, the invention provides a method of treating a proliferative disease (such as cancer) in an individual comprising administering to the individual a) an effective amount of a composition comprising nanoparticles comprising paclitaxel coated with an albumin (such as Abraxane®), and b) an effective amount of a hedgehog inhibitor. In some embodiments, the nanoparticles have an average diameter of no greater than about 200 nm. In some embodiments, at least about 50% (for example at least about any one of 60%, 70%, 80%, 90%, or more) of all the nanoparticles in the composition have a diameter of no greater than about 200 nm.

The methods described here are particularly useful for individuals having cancer, including for example any one of the following cancers: pancreatic cancer, colon cancer, basal cell carcinoma, medulloblastoma, glioblastoma, multiple myeloma, chronic myelogenous leukemia (CML), acute myelogenous leukemia, lung cancer (small cell lung cancer and non-small cell lung cancer), esophageal cancer, stomach cancer, biliary cancer, prostate cancer, liver cancer, hepatocellular cancer, gastrointestinal cancer, gastric cancer, and ovarian and bladder cancer. In some embodiments, the cancer is selected from the group consisting of pancreas ductal adenocarcinoma, colon adenocarcinoma, and ovary cystadenocarcinoma. In some embodiments, the cancer is selected from the group consisting of medulloblastoma, rhabdomyosarcoma, melanoma, basal cell carcinoma, breast cancer, lung cancer, liver cancer, stomach cancer, prostate cancer, colon cancer, and pancreatic cancers. In some embodiments, the cancer is pancreatic cancer. In some embodiments, the cancer is colon cancer. In some embodiments, the cancer is a tumor that is poorly perfused and/or poorly vascularized.

In some embodiments, the cancer is pancreatic cancer, including for example pancreatic adenocarcinoma, pancreatic adenosquamous carcinoma, pancreatic squamous cell carcinoma, or pancreatic giant cell carcinoma. Thus, for example, in some embodiments, there is provided a method of treating pancreatic cancer, comprising administering to the individual a) an effective amount of a composition comprising nanoparticles comprising a taxane and a carrier protein (such as albumin), and b) an effective amount of a hedgehog inhibitor. In some embodiments, there is provided a method treating pancreatic cancer, comprising administering to the individual a) an effective amount of a composition comprising nanoparticles comprising paclitaxel and an albumin (such as Abraxane®), and b) an effective amount of a hedgehog inhibitor. In some embodiments, the pancreatic cancer is exocrine pancreatic cancer. In some embodiments, the pancreatic cancer is endocrine pancreatic cancer (such as islet cell carcinoma). In some embodiments, the pancreatic cancer is advanced metastatic pancreatic cancer.

In some embodiments, there is provided a method of treating pancreatic cancer, comprising administering to the individual a) an effective amount of a composition comprising nanoparticles comprising a taxane coated with a carrier protein (such as albumin), and b) an effective amount of a hedgehog inhibitor. In some embodiments, there is provided a method treating pancreatic cancer, comprising administering to the individual a) an effective amount of a composition comprising nanoparticles comprising paclitaxel coated with an albumin (such as Abraxane®), and b) an effective amount of a hedgehog inhibitor. In some embodiments, the nanoparticles have an average diameter of no greater than about 200 nm. In some embodiments, at least about 50% (for example at least about any one of 60%, 70%, 80%, 90%, or more) of all the nanoparticles in the composition have a diameter of no greater than about 200 nm.

In some embodiments, the cancer is colon cancer, including for example colon adenocarcinoma, colon melanoma, colon lymphoma, or colon carcinoid. In some embodiments, the colon cancer is stage I, stage II, stage III, or stage IV. In some embodiments, the colon cancer is locally advanced or metastatic. Thus, for example, in some embodiments, there is provided a method of treating colon cancer, comprising administering to the individual a) an effective amount of a composition comprising nanoparticles comprising a taxane and a carrier protein (such as albumin), and b) an effective amount of a hedgehog inhibitor. In some embodiments, there is provided a method treating colon cancer, comprising administering to the individual a) an effective amount of a composition comprising nanoparticles comprising paclitaxel and an albumin (such as Abraxane®), and b) an effective amount of a hedgehog inhibitor. In some embodiments, the colon cancer is colon adenocarcinoma. In some embodiments, the colon cancer is advanced metastatic colon cancer.

In some embodiments, there is provided a method of treating colon cancer, comprising administering to the individual a) an effective amount of a composition comprising nanoparticles comprising a taxane coated with a carrier protein (such as albumin), and b) an effective amount of a hedgehog inhibitor. In some embodiments, there is provided a method of treating colon cancer, comprising administering to the individual a) an effective amount of a composition comprising nanoparticles comprising paclitaxel coated with an albumin (such as Abraxane®), and b) an effective amount of a hedgehog inhibitor. In some embodiments, the nanoparticles have an average diameter of no greater than about 200 nm. In some embodiments, at least about 50% (for example at least about any one of 60%, 70%, 80%, 90%, or more) of all the nanoparticles in the composition have a diameter of no greater than about 200 nm.

In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of an isoquinoline or quinazoline compound, such as a compound according to any of the general formulas (I), (Ia), (Ib), or (Ic). In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of a hedgehog inhibitor, wherein the hedgehog inhibitor is a tetrazine compound, such as a compound according to any of the general formulas (II), (IIa), (IIb), or (IIc). In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of a hedgehog inhibitor selected from the compounds provided in Table 1 and/or Table 4. In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of a hedgehog inhibitor selected from the compounds provided in Table 2 and/or Table 5. In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of a hedgehog inhibitor selected from the compounds provided in Table 3.

In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of a hedgehog inhibitor, wherein the hedgehog inhibitor is a cyclopamine or derivative thereof (such as IPI926). In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of a hedgehog inhibitor, wherein the hedgehog inhibitor is a pyridine or derivative thereof (such as GDC-0449 or NVP-LDE225). In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of a hedgehog inhibitor, wherein the hedgehog inhibitor is Jervine, GANT61, purmorphamine, SAG, SANT-2, tomatidine, zerumbone, or a derivative thereof. In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of a hedgehog inhibitor, wherein the hedgehog inhibitor is GDC-0449, XL139, IPI926, IPI609 (IPI269609), or LDE225. In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of a hedgehog inhibitor, wherein the hedgehog inhibitor is GDC-0449, IPI926, NVP-LDE225, or BMS-833923/XL139. In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of IPI926.

In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of ABI1C4. In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of ABI1C5. In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of ABI1C6. In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of ABI1C7. In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of ABI2C4. In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of ABI2C5. In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of ABI2C6. In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of ABI2C7. In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of ABI2012. In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of ABI1914. In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of ABI2088. In some embodiments, there is provided a method of treating a proliferative disease (such as cancer) in an individual comprising administering to the individual a) an effective amount of a composition comprising nanoparticles comprising a taxane and a carrier protein (such as albumin), and b) an effective amount of ABI2099. In some embodiments, the hedgehog inhibitor is provided in the form of nanoparticles (such as nanoparticles comprising a hedgehog inhibitor and a carrier protein (such as albumin)).

In some embodiments, the invention provides a method of treating a proliferative disease (such as cancer) in an individual comprising administering to the individual a) an effective amount of a composition comprising nanoparticles comprising paclitaxel and an albumin (such as Abraxane®), and b) an effective amount of a hedgehog inhibitor. In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of an isoquinoline or quinazoline compound, such as a compound according to any of the general formulas (I), (Ia), (Ib), or (Ic). In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of a hedgehog inhibitor, wherein the hedgehog inhibitor is a tetrazine compound, such as a compound according to any of the general formulas (II), (IIa), (IIb), or (IIc). In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of a hedgehog inhibitor selected from the compounds provided in Table 1 and/or Table 4. In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of a hedgehog inhibitor selected from the compounds provided in Table 2 and/or Table 5. In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of a hedgehog inhibitor selected from the compounds provided in Table 3.

In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of a hedgehog inhibitor, wherein the hedgehog inhibitor is a cyclopamine or derivative thereof (such as IPI926). In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of a hedgehog inhibitor, wherein the hedgehog inhibitor is a pyridine or derivative thereof (such as GDC-0449 or NVP-LDE225). In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of a hedgehog inhibitor, wherein the hedgehog inhibitor is Jervine, GANT61, purmorphamine, SAG, SANT-2, tomatidine, zerumbone, or a derivative thereof. In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of a hedgehog inhibitor, wherein the hedgehog inhibitor is GDC-0449, XL139, IPI926, IPI609 (IPI269609), or LDE225. In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of a hedgehog inhibitor, wherein the hedgehog inhibitor is GDC-0449, IPI926, NVP-LDE225, or BMS-833923/XL139. In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of IPI926.

In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of ABI1C4. In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of ABI1C5. In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of ABI1C6. In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of ABI1C7. In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of ABI2C4. In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of ABI2C5. In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of ABI2C6. In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of ABI2C7. In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of ABI2012. In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of ABI1914. In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of ABI2088. In some embodiments, there is provided a method of treating a proliferative disease (such as cancer) in an individual comprising administering to the individual a) an effective amount of a composition comprising nanoparticles comprising paclitaxel and an albumin (such as Abraxane®), and b) an effective amount of ABI2099. In some embodiments, the hedgehog inhibitor is provided in the form of nanoparticles (such as nanoparticles comprising a hedgehog inhibitor and a carrier protein (such as albumin)).

In some embodiments, there is provided a method of treating pancreatic cancer, comprising administering to the individual a) an effective amount of a composition comprising nanoparticles comprising paclitaxel and albumin (such as Abraxane®), and b) an effective amount of an isoquinoline or quinazoline compound (such as a compound of any of general formulas (I), (Ia), (Ib), or (Ic), or a compound provided in Table 1, or a compound provided in Table 4). In some embodiments, there is provided a method of treating pancreatic cancer, comprising administering to the individual a) an effective amount of a composition comprising nanoparticles comprising paclitaxel and albumin (such as Abraxane®), and b) an effective amount of a tetrazine compound (such as a compound of any of general formulas (II), (IIa), (IIb), or (IIc), or a compound provided in Table 2, or a compound provided in Table 5). In some embodiments, there is provided a method of treating pancreatic cancer, comprising administering to the individual a) an effective amount of a composition comprising nanoparticles comprising paclitaxel and albumin (such as Abraxane®), and b) an effective amount of a compound provided in Table 3. In some embodiments, there is provided a method of treating pancreatic cancer, comprising administering to the individual a) an effective amount of a composition comprising nanoparticles comprising paclitaxel and albumin (such as Abraxane®), and b) an effective amount of a compound selected from Jervine, GANT61, purmorphamine, SAG, SANT-2, tomatidine, zerumbone, and derivatives thereof. In some embodiments, there is provided a method of treating pancreatic cancer, comprising administering to the individual a) an effective amount of a composition comprising nanoparticles comprising paclitaxel and albumin (such as Abraxane®), and b) an effective amount of a compound selected from GDC-0449, XL139, IPI926, IPI609 (IPI269609), and LDE225. In some embodiments, there is provided a method of treating pancreatic cancer, comprising administering to the individual a) an effective amount of a composition comprising nanoparticles comprising paclitaxel and albumin (such as Abraxane®), and b) an effective amount of a compound selected from GDC-0449, IPI926, NVP-LDE225, and BMS-833923/XL139. In some embodiments, there is provided a method of treating pancreatic cancer, comprising administering to the individual a) an effective amount of a composition comprising nanoparticles comprising paclitaxel and albumin (such as Abraxane®), and b) an effective amount of a cyclopamine or derivative thereof (such as IPI926). In some embodiments, there is provided a method of treating pancreatic cancer, comprising administering to the individual a) an effective amount of a composition comprising nanoparticles comprising paclitaxel and albumin (such as Abraxane®), and b) an effective amount of IPI926.

In some embodiments, there is provided a method of treating pancreatic cancer, comprising administering to the individual a) an effective amount of a composition comprising nanoparticles comprising paclitaxel and albumin (such as Abraxane®), and b) an effective amount of ABI1C4. In some embodiments, there is provided a method of treating pancreatic cancer, comprising administering to the individual a) an effective amount of a composition comprising nanoparticles comprising paclitaxel and albumin (such as Abraxane®), and b) an effective amount of ABI1C5. In some embodiments, there is provided a method of treating pancreatic cancer, comprising administering to the individual a) an effective amount of a composition comprising nanoparticles comprising paclitaxel and albumin (such as Abraxane®), and b) an effective amount of ABI1C6. In some embodiments, there is provided a method of treating pancreatic cancer, comprising administering to the individual a) an effective amount of a composition comprising nanoparticles comprising paclitaxel and albumin (such as Abraxane®), and b) an effective amount of ABI1C7. In some embodiments, there is provided a method of treating pancreatic cancer, comprising administering to the individual a) an effective amount of a composition comprising nanoparticles comprising paclitaxel and albumin (such as Abraxane®), and b) an effective amount of ABI2C4. In some embodiments, there is provided a method of treating pancreatic cancer, comprising administering to the individual a) an effective amount of a composition comprising nanoparticles comprising paclitaxel and albumin (such as Abraxane®), and b) an effective amount of ABI2C5. In some embodiments, there is provided a method of treating pancreatic cancer, comprising administering to the individual a) an effective amount of a composition comprising nanoparticles comprising paclitaxel and albumin (such as Abraxane®), and b) an effective amount of ABI2C6. In some embodiments, there is provided a method of treating pancreatic cancer, comprising administering to the individual a) an effective amount of a composition comprising nanoparticles comprising paclitaxel and albumin (such as Abraxane®), and b) an effective amount of ABI2C7. In some embodiments, there is provided a method of treating pancreatic cancer, comprising administering to the individual a) an effective amount of a composition comprising nanoparticles comprising paclitaxel and albumin (such as Abraxane®), and b) an effective amount of ABI2012. In some embodiments, there is provided a method of treating pancreatic cancer, comprising administering to the individual a) an effective amount of a composition comprising nanoparticles comprising paclitaxel and albumin (such as Abraxane®), and b) an effective amount of ABI1914. In some embodiments, there is provided a method of treating pancreatic cancer, comprising administering to the individual a) an effective amount of a composition comprising nanoparticles comprising paclitaxel and albumin (such as Abraxane®), and b) an effective amount of ABI2088. In some embodiments, there is provided a method of treating pancreatic cancer, comprising administering to the individual a) an effective amount of a composition comprising nanoparticles comprising paclitaxel and albumin (such as Abraxane®), and b) an effective amount of ABI2099. In some embodiments, the hedgehog inhibitor is provided in the form of nanoparticles (such as nanoparticles comprising a hedgehog inhibitor and a carrier protein (such as albumin)).

The composition comprising nanoparticles of taxane (also referred to as "nanoparticle taxane composition") and the hedgehog inhibitor can be administered simultaneously, either in the same composition or in separate compositions. Alternatively, the nanoparticle taxane composition and the hedgehog inhibitor are administered sequentially, i.e., the nanoparticle taxane composition is administered either prior to or after the administration of the hedgehog inhibitor. In some embodiments, the nanoparticle taxane composition is administered prior to the administration of the hedgehog inhibitor. In some embodiments, the nanoparticle taxane composition is administered after the administration of the hedgehog inhibitor.

In some embodiments, the administration of the nanoparticle taxane composition and the hedgehog inhibitor are concurrent, i.e., the administration period of the nanoparticle taxane composition and that of the hedgehog inhibitor overlap with each other. In some embodiments, the nanoparticle taxane composition is administered for at least one cycle (for example, at least any of 2, 3, or 4 cycles) prior to the administration of the hedgehog inhibitor. In some embodiments, the hedgehog inhibitor is administered for at least any of one, two, three, or four weeks.

In some embodiments, the administrations of the nanoparticle taxane composition and the hedgehog inhibitor are non-concurrent. For example, in some embodiments, the administration of the nanoparticle taxane composition is terminated before the hedgehog inhibitor is administered. In some embodiments, the administration of the hedgehog inhibitor is terminated before the nanoparticle taxane composition is administered.

In some embodiments, the method further comprises administering an effective amount of an antimetabolite (such as gemcitabine). Thus, for example, in some embodiments, there is provided a method of treating a proliferative disease (such as cancer), comprising administering to the individual a) an effective amount of a composition comprising nanoparticles comprising a taxane and a carrier protein (such as albumin), b) an effective amount of a hedgehog inhibitor; and c) an effective amount of an antimetabolite (such as gemcitabine). In some embodiments, there is provided a method treating a proliferative disease (such as cancer), comprising administering to the individual a) an effective amount of a composition comprising nanoparticles comprising paclitaxel and an albumin (such as Abraxane®), b) an effective amount of a hedgehog inhibitor, and c) an effective amount of gemcitabine.

In some embodiments, there is provided a method of treating pancreatic cancer, comprising administering to the individual a) an effective amount of a composition comprising nanoparticles comprising a taxane and a carrier protein (such as albumin), b) an effective amount of a hedgehog inhibitor; and c) an effective amount of an antimetabolite (such as gemcitabine). In some embodiments, there is provided a method of treating pancreatic cancer, comprising administering to the individual a) an effective amount of a composition comprising nanoparticles comprising paclitaxel and an albumin (such as Abraxane®), b) an effective amount of a hedgehog inhibitor, and c) an effective amount of gemcitabine.

In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of an isoquinoline or quinazoline compound (such as a compound of any of general formulas (I), (Ia), (Ib), or (Ic), or a compound provided in Table 1, or a compound provided in Table 4), and c) an effective amount of gemcitabine. In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of a tetrazine compound (such as a compound of any of general formulas (II), (IIa), (IIb), or (IIc), or a compound provided in Table 2, or a compound provided in Table 5), and c) an effective amount of gemcitabine. In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of a compound provided in Table 3, and c) an effective amount of gemcitabine.

In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of a compound selected from Jervine, GANT61, purmorphamine, SAG, SANT-2, tomatidine, zerumbone, and derivatives thereof, and c) an effective amount of gemcitabine. In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of a compound selected from GDC-0449, XL139, IPI926, IPI609 (IPI269609), and LDE225, and c) an effective amount of gemcitabine. In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of a compound selected from GDC-0449, IPI926, NVP-LDE225, and BMS-833923/XL139, and c) an effective amount of gemcitabine. In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of a cyclopamine or derivative thereof (such as IPI926), and c) an effective amount of gemcitabine. In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of IPI926, and c) an effective amount of gemcitabine.

In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of ABI1C4, and c) an effective amount of gemcitabine. In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of ABI1C5, and c) an effective amount of gemcitabine. In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of ABI1C6, and c) an effective amount of gemcitabine. In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of ABI1C7, and c) an effective amount of gemcitabine. In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of ABI2C4, and c) an effective amount of gemcitabine. In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of ABI2C5, and c) an effective amount of gemcitabine. In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of ABI2C6, and c) an effective amount of gemcitabine. In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of ABI2C7, and c) an effective amount of gemcitabine. In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of ABI2012, and c) an effective amount of gemcitabine. In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of ABI1914, and c) an effective amount of gemcitabine. In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of ABI2088, and c) an effective amount of gemcitabine. In some embodiments, there is provided a method of treating cancer (such as pancreatic cancer or colon cancer), comprising administering to the individual a) an effective amount of a composition comprising nanoparticles comprising paclitaxel and albumin (such as Abraxane®), b) an effective amount of ABI2099, and c) an effective amount of gemcitabine.

In some embodiments, there is provided a method of treating a cancer, comprising administering to the individual a) an effective amount of a composition comprising nanoparticles comprising paclitaxel and albumin (such as Abraxane®), and b) an effective amount of GDC-0449, wherein the cancer is a locally advanced solid tumor or metastatic solid tumor. In some embodiments, there is provided a method of treating a cancer, comprising administering to the individual a) an effective amount of a composition comprising nanoparticles comprising paclitaxel and albumin (such as Abraxane®), and b) an effective amount of GDC-0449, wherein the cancer is advanced basal cell carcinoma, metastatic colorectal cancer or advanced ovarian cancer. In some embodiments, there is provided a method of treating an advanced and/or metastatic solid tumor, comprising administering to the individual a) an effective amount of a composition comprising nanoparticles comprising paclitaxel and albumin (such as Abraxane®), and b) an effective amount of IPI926.

In some embodiments, there is provided a method of treating a locally advanced or a metastatic solid tumor, comprising administering to the individual a) an effective amount of a composition comprising nanoparticles comprising paclitaxel and albumin (such as Abraxane®), and b) an effective amount of NVP-LDE225. In some embodiments, there is provided a method of treating a locally advanced or metastatic solid tumor, comprising administering to the individual a) an effective amount of a composition comprising nanoparticles comprising paclitaxel and albumin (such as Abraxane®), and b) an effective amount of BMS-833923/XL139.

Also provided herein are methods of enhancing delivery of a taxane to a tumor comprising administering to an individual an effective amount of a hedgehog inhibitor and vice versa. In some embodiments, there is provided a method of enhancing delivery of a taxane to a tumor by administering to the individual a) a composition comprising nanoparticles comprising the taxane and a carrier protein (such as albumin), and b) a hedgehog inhibitor. In some embodiments, there is provided a method of enhancing delivery of paclitaxel to a tumor by administering to the individual a) a composition comprising nanoparticles comprising paclitaxel and albumin (such as Abraxane®), and b) a hedgehog inhibitor. In some embodiments, there is provided a method of enhancing delivery of a hedgehog inhibitor to a tumor by administering to the individual a) a composition comprising nanoparticles comprising the taxane and a carrier protein (such as albumin), and b) a hedgehog inhibitor. In some embodiments, there is provided a method of enhancing delivery of a hedgehog inhibitor to a tumor by administering to the individual a) a composition comprising nanoparticles comprising paclitaxel and albumin (such as Abraxane®), and b) a hedgehog inhibitor. In some embodiments, there is provided a method of enhancing delivery of a compound to a tumor by administering to the individual a) a composition comprising nanoparticles comprising the taxane and a carrier protein (such as albumin), b) a hedgehog inhibitor and c) an effective amount of the compound. In some embodiments, there is provided a method of enhancing delivery of a compound to a tumor by administering to the individual a) a composition comprising nanoparticles comprising paclitaxel and albumin (such as Abraxane®), b) an effective amount of a hedgehog inhibitor, and c) the compound.

In some embodiments, the tumor has extensive stroma. In some embodiments, the tumor is pancreatic, lung, colon, or melanoma. In some embodiments, the tumor is poorly perfused and/or poorly vascularized.

In some embodiments, there is provided a method of inhibiting tumor metastasis in an individual, comprising administering to the individual: a) an effective amount of a composition comprising nanoparticles comprising taxane and a carrier protein, and b) an effective amount of a hedgehog inhibitor. In some embodiments, the effective amounts of the taxane nanoparticle composition and the hedgehog inhibitor synergistically inhibit tumor metastasis. In some embodiments, at least about 10% (including for example at least about any of 20%, 30%, 40%, 60%, 70%, 80%, 90%, or 100%) metastasis is inhibited. In some embodiments, method of inhibiting metastasis to lymph node is provided. In some embodiments, method of inhibiting metastasis to the lung is provided. In some embodiments, the taxane is paclitaxel.

In some embodiments, there is provided a method of prolonging survival (such as disease free survival) in an individual having cancer, comprising administering to the individual a) an effective amount of a composition comprising nanoparticles comprising a taxane and a carrier protein (such as albumin), and b) an effective amount of a hedgehog inhibitor. In some embodiments, there is provided a method of prolonging survival (such as disease free survival) in an individual having cancer, comprising administering to the individual a) an effective amount of a composition comprising nanoparticles comprising paclitaxel and an albumin (such as Abraxane®), and b) an effective amount of a hedgehog inhibitor. In some embodiments, the survival is prolonged for at least about 2, 3, 4, 5, 6, 12, or 24 months.

In some embodiments, there is provided a method of causing disease remission (partial or complete) in an individual having cancer, comprising administering to the individual a) an effective amount of a composition comprising nanoparticles comprising a taxane and a carrier protein (such as albumin), and b) an effective amount of a hedgehog inhibitor. In some embodiments, there is provided a method of causing disease remission (partial or complete) in an individual having cancer, comprising administering to the individual a) an effective amount of a composition comprising nanoparticles comprising paclitaxel and an albumin (such as Abraxane®), and b) an effective amount of a hedgehog inhibitor.

In some embodiments, there is provided a method of improving quality of life in an individual having a proliferative disease (such as cancer), comprising administering to the individual a) an effective amount of a composition comprising nanoparticles comprising a taxane and a carrier protein (such as albumin), and b) an effective amount of a hedgehog inhibitor. In some embodiments, there is provided a method of improving quality of life in an individual having a proliferative disease (such as cancer), comprising administering to the individual a) an effective amount of a composition comprising nanoparticles comprising paclitaxel and an albumin (such as Abraxane®), and b) an effective amount of a hedgehog inhibitor. In some embodiments, the survival is prolonged for at least about 2, 3, 4, 5, 6, 12, or 24 months.

In some embodiments, there is provided a method of inhibiting stromal desmoplasia in an individual, comprising administering to the individual: a) an effective amount of a composition comprising nanoparticles comprising taxane and a carrier protein, and b) an effective amount of a hedgehog inhibitor.

In some embodiments, there is provided a method of inhibiting growth of cancer stem cells in an individual, comprising administering to the individual: a) an effective amount of a composition comprising nanoparticles comprising taxane and a carrier protein, and b) an effective amount of a hedgehog inhibitor.

In some embodiments, there is provided a method of treating cancer in an individual, comprising administering to the individual: a) an effective amount of a composition comprising nanoparticles comprising taxane and a carrier protein, and b) an effective amount of a hedgehog inhibitor, wherein the expression of Gli (such as Gli-1) is decreased (for example by at least about any one of 10%, 20%, 30%, 40%, 50%, 60%, 70%, 80%, 90%, 95%, 99%, or 100%) comparing to an untreated control individual.

In some embodiments, there is provided a method of treating cancer in an individual, comprising administering to the individual: a) an effective amount of a composition comprising nanoparticles comprising taxane and a carrier protein, and b) an effective amount of a hedgehog inhibitor, wherein the individual has increased tumor vasculature density as a result of the treatment. In some embodiments, there is provided a method of treating cancer in an individual, comprising administering to the individual: a) an effective amount of a composition comprising nanoparticles comprising taxane and a carrier protein, and b) an effective amount of a hedgehog inhibitor, wherein the individual has decreased tumor vasculature density as a result of the treatment.

In some embodiments, there is provided a method of treating cancer in an individual, comprising administering to the individual: a) an effective amount of a composition comprising nanoparticles comprising taxane and a carrier protein, and b) an effective amount of a hedgehog inhibitor, wherein the individual has increased angiogenesis as a result of the treatment. In some embodiments, there is provided a method of treating cancer in an individual, comprising administering to the individual: a) an effective amount of a composition comprising nanoparticles comprising taxane and a carrier protein, and b) an effective amount of a hedgehog inhibitor, wherein the individual has decreased angiogenesis as a result of the treatment.

In some embodiments, there is provided a method of treating cancer in an individual, comprising administering to the individual: a) an effective amount of a composition comprising nanoparticles comprising taxane and a carrier protein, and b) an effective amount of a hedgehog inhibitor, wherein the individual has collapsed stroma as a result of the treatment. In some embodiments, there is provided a method of treating cancer in an individual, comprising administering to the individual: a) an effective amount of a composition comprising nanoparticles comprising taxane and a carrier protein, and b) an effective amount of a hedgehog inhibitor, wherein the individual has increased microvessel density (for example by at least about any one of 10%, 20%, 30%, 40%, 50%, 60%, 70%, 80%, 90%, 95%, 99%, or 100%) in a tumor as a result of the treatment.

In some embodiments, there is provided a method of treating cancer in an individual, comprising administering to the individual: a) an effective amount of a composition comprising nanoparticles comprising a taxane and a carrier protein, b) an effective amount of a hedgehog inhibitor, wherein the individual has increased concentration of the taxane at the tumor site compared to an individual not administered an effective amount of the hedgehog inhibitor. In some embodiments, there is provided a method of treating cancer in an individual, comprising administering to the individual: a) an effective amount of a composition comprising nanoparticles comprising a taxane and a carrier protein, b) an effective amount of a hedgehog inhibitor, wherein the individual has increased concentration of the hedgehog inhibitor at the tumor site compared to an individual not administered an effective amount of the taxane.

In some embodiments, there is provided a method of treating cancer in an individual, comprising administering to the individual: a) an effective amount of a composition comprising nanoparticles comprising taxane and a carrier protein, b) an effective amount of a hedgehog inhibitor, and c) an effective amount of a third anti-cancer compound (such as gemcitabine), wherein the individual has increased concentration of the anti-cancer compound at the tumor site compared to an individual not administered an effective around of a composition comprising nanoparticles comprising taxane and a carrier protein and an effective amount of a hedgehog inhibitor.

In some embodiments, the amount of the drug (such as the hedgehog inhibitor or the nanoparticle taxane composition) may in some embodiments be sufficient to: (i) reduce the number of cancer cells; (ii) reduce tumor size; (iii) inhibit, retard, slow to some extent and preferably stop cancer cell infiltration into peripheral organs; (iv) inhibit (i.e., slow to some extent and preferably stop) tumor metastasis; (v) inhibit tumor growth; (vi) prevent or delay occurrence and/or recurrence of tumor; (vii) relieve to some extent one or more of the symptoms associated with the cancer; (viii) prolong overall survival; (ix) prolong disease free survival; (x) cause particle remission of the disease; and/or (xi) cause complete remission of the disease. In some embodiments, the amount of the hedgehog inhibitor is sufficient to: (i) inhibit stromal desmoplasia; (ii) inhibit growth of cancer stem cells; (iii) inhibit hedgehog autocrine signaling; (iv) inhibit hedgehog paracrine signaling; and/or (v) decrease the expression level of Gli (such as Gli-1)(for example by at least about any one of 10%, 20%, 30%, 40%, 50%, 60%, 70%, 80%, 90%, 95%, 99%, or 100%) comparing to an untreated control individual.

In some embodiments, the amount of the hedgehog inhibitor or nanoparticle taxane composition is effective to: (i) cause stromal collapse, (ii) increase the concentration of gemcitabine or another anti-cancer compound (for example by at least about any one of 10%, 20%, 30%, 40%, 50%, 60%, 70%, 80%, 90%, 95%, 99%, or 100%) in the tumor when administered in combination with gemcitabine or the other anti-cancer compound, and/or (iii) increase microvessel density (for example by at least about any one of 10%, 20%, 30%, 40%, 50%, 60%, 70%, 80%, 90%, 95%, 99%, or 100%) in the tumor. In some embodiments, the amount of the hedgehog inhibitor and nanoparticle taxane composition together are effective to: (i) cause stromal collapse, (ii) increase the concentration of gemcitabine or another anti-cancer compound (for example by at least about any one of 10%, 20%, 30%, 40%, 50%, 60%, 70%, 80%, 90%, 95%, 99%, or 100%) in the tumor when administered in combination with gemcitabine or the other anti-cancer compound, and/or (iii) increase microvessel density (for example by at least about any one of 10%, 20%, 30%, 40%, 50%, 60%, 70%, 80%, 90%, 95%, 99%, or 100%) in the tumor. In some embodiments, the amount of the hedgehog inhibitor and nanoparticle taxane composition are synergistically effective to: (i) cause stromal collapse, (ii) increase the concentration of gemcitabine or another anti-cancer compound (for example by at least about any one of 10%, 20%, 30%, 40%, 50%, 60%, 70%, 80%, 90%, 95%, 99%, or 100%) in the tumor when administered in combination with gemcitabine or the other anti-cancer compound, and/or (iii) increase microvessel density (for example by at least about any one of 10%, 20%, 30%, 40%, 50%, 60%, 70%, 80%, 90%, 95%, 99%, or 100%) in the tumor.

In some embodiments, the effective amounts of the taxane nanoparticle composition and the hedgehog inhibitor synergistically inhibit tumor growth. In some embodiments, at least about 10% (including for example at least about any of 20%, 30%, 40%, 60%, 70%, 80%, 90%, or 100%) tumor growth is inhibited. In some embodiments, the taxane is paclitaxel. In some embodiments, the taxane in the nanoparticle in the composition is administered by intravenous administration. In some embodiments, the hedgehog inhibitor is administered by intraperitoneal administration. In some embodiments, the hedgehog inhibitor is administered by oral administration. In some embodiments, the nanoparticle taxane composition is administered by intravenous administration and the hedgehog inhibitor is administered by oral administration.

In some embodiments, the effective amount of taxane in the nanoparticle taxane composition is between about 45 mg/m² to about 350 mg/m² and the effective amount of the hedgehog inhibitor is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane in the nanoparticle taxane composition is between about 80 mg/m² to about 150 mg/m² and the effective amount of the hedgehog inhibitor is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane (e.g., paclitaxel) in the nanoparticle taxane composition is about 100 mg/m². In some embodiments, the effective amount of taxane in the nanoparticle taxane composition is between about 170 mg/m² to about 200 mg/m² and the effective amount of the hedgehog inhibitor is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane in the nanoparticle taxane composition is between about 200 mg/m² to about 350 mg/m² and the effective amount of the hedgehog inhibitor is about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the effective amount of taxane (e.g., paclitaxel) in the nanoparticle taxane composition is about 260 mg/m². In some embodiments of any of the above methods, the effective amount of the hedgehog inhibitor is about 1-5 mg/kg, 5-10 mg/kg, 10-15 mg/kg, 15-20 mg/kg, 20-30 mg/kg, about 30-40 mg/kg, about 40-50 mg/kg, about 50-60 mg/kg, about 60-70 mg/kg, about 70-80 mg/kg, about 80-100 mg/kg, or about 100-120 mg/kg. In some embodiments, the taxane in the nanoparticle taxane composition is administered weekly. In some embodiments, the taxane in a nanoparticle taxane composition is administered every two weeks. In some embodiments, the taxane in the nanoparticle taxane composition is administered every three weeks. In some embodiments, the hedgehog inhibitor is administered 1x, 2x, 3x, 4x, 5x, 6x, or 7 times a week. In some embodiments, the hedgehog inhibitor is administered daily. In some embodiments, the hedgehog inhibitor is administered 2, 3, 4, 5, 6 times per week. In some embodiments, the hedgehog inhibitor is administered every two weeks or two out of three weeks.

In some embodiments, the methods further comprise administration of one or more additional agent. The additional agent can be another agent that inhibits a hedgehog signaling pathway, such as the agents described herein. Alternatively, the additional agent is a chemotherapeutic agent, such as chemotherapeutic agents described in U.S. Patent Application No. 2006/0263434, incorporated herein in its entirety. In some embodiments, the additional agent is any one of dexamethasone, bortezomib, imatinib, sorafenib, gemcitabine, lenalidomide, sunitinib, erlotinib, paclitaxel, and docetaxel.

For example, in some embodiments, there is provided a method of treating a proliferative disease, comprising: a) an effective amount of a composition comprising nanoparticles comprising a taxane (such as paclitaxel) and a carrier protein (such as albumin), b) an effective amount of a hedgehog inhibitor, and c) an effective amount of an additional agent selected from the group consisting of dexamethasone, bortezomib, imatinib, sorafenib, gemcitabine, lenalidomide, sunitinib, paclitaxel, and docetaxel.

In some embodiments, there is provided a method of treating a proliferative disease, comprising: a) an effective amount of a composition comprising nanoparticles comprising a taxane (such as paclitaxel) and a carrier protein (such as albumin), b) an effective amount of a hedgehog inhibitor, and c) an effective amount of an antimetabolite. In some embodiments, there is provided a method of treating a proliferative disease, comprising administering: a) an effective amount of a composition comprising nanoparticles comprising a taxane (such as paclitaxel) and a carrier protein (such as albumin), b) an effective amount of a hedgehog inhibitor, and c) an effective amount of gemcitabine.

In some embodiments, the gemcitabine is administered at a dose of about 500-5000 mg/m², including for example any of about 1000-2000 mg/m² (including for example about any of 1000-1200 mg/m², 1200-1400 mg/m², 1400-1600 mg/m², 1600-1800 mg/m², and 1800-2000 mg/m²). In some embodiments, gemcitabine is administered at a dose of about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). In some embodiments, the gemcitabine is administered weekly, biweekly, every three weeks, every four weeks, two out of three weeks, and three out of four weeks.

In some embodiments, there is provided a method of treating a patient having a proliferative disease, comprising administering: a) about 100 to about 150 mg/m² Abraxane® weekly, b) about 1000 to about 2000 mg/m² gemcitabine once every four weeks, and c) an effective amount of a hedgehog inhibitor. In some embodiments, the hedgehog inhibitor is administered orally, for example at a daily dose of about 4-120 mg/kg (for example 4-40 mg/kg, 40-75 mg/kg, or 75-100 mg/kg). The administration of gemcitabine can be simultaneous with that of the nanoparticle taxane composition, or sequential with that of the nanoparticle taxane composition. For example, the administration of the gemcitabine can be immediately after or before the administration of the nanoparticle taxane composition.

In some embodiments, there is provided a method of treating a patient having a proliferative disease, comprising administering: a) about 10 to about 200 mg/kg Abraxane® weekly (or about 10 to about 180 mg/kg once every four days, about 10 to about 30 mg/kg daily, or about 30 mg/kg once every three weeks), and b) about 30-100 mg/kg (such as about 30 to about 50 mg/kg or about 75-100 mg/kg) of a hedgehog inhibitor daily. In some embodiments, the Abraxane® is administered by intravenous injection. In some embodiments, the hedgehog inhibitor is administered orally. In some embodiments, the method further comprises administering about 80 to about 120 mg/kg gemcitabine twice weekly by intraperitoneal injection. The administration of gemcitabine can be simultaneous with that of the nanoparticle taxane composition, or sequential with that of the nanoparticle taxane composition. For example, the administration of the gemcitabine can be immediately after or before the administration of the nanoparticle taxane composition

The administration of gemcitabine can be simultaneous with that of the hedgehog inhibitor, or sequential with that of the hedgehog inhibitor. For example, the administration of the gemcitabine can be immediately after or before the administration of the hedgehog inhibitor.

In some embodiments, the method (with or without gemcitabine) further comprises administration of erlotinib. Erlotinib is administered (for example by intraperitoneal administration) at about 20-200 mg/kg/day (including for example about any one of 50 mg/kg/day, 80 mg/kg/day, 100 mg/kg/day, 120 mg/kg/day, 140 mg/kg/day, 180 mg/kg/day).

In some embodiments, the invention provides pharmaceutical compositions comprising nanoparticles comprising a taxane and a carrier protein (such as albumin) for use in the treatment of a proliferative disease (such as cancer), wherein said use comprises simultaneous and/or sequential administration of a hedgehog inhibitor. In some embodiments, the invention provides a pharmaceutical composition comprising a hedgehog inhibitor for use in the treatment of a proliferative disease (such as cancer), wherein said use comprises simultaneous and/or sequential administration of a composition comprising nanoparticles comprising a taxane and a carrier protein (such as albumin). In some embodiments, the invention provides taxane-containing nanoparticle taxane compositions and compositions comprising a hedgehog inhibitor for simultaneous, and/or sequential use for treatment of a proliferative disease (such as cancer).

In some embodiments, there is provided a method of treating pancreatic cancer, comprising administering to the individual a) an effective amount of a composition comprising paclitaxel-containing nanoparticles comprising paclitaxel and albumin (such as Abraxane®), and b) an effective amount of a composition comprising nanoparticles comprising a Hedgehog inhibitor (such as IPI926). In some embodiments, there is provided a method of treating pancreatic cancer, comprising administering to the individual a) an effective amount of a composition comprising paclitaxel-containing nanoparticles comprising paclitaxel and albumin (such as Abraxane®), b) an effective amount of a composition comprising nanoparticles comprising a Hedgehog inhibitor (such as IPI926), and c) an effective amount of gemcitabine.

In some embodiments, there is provided a method of treating a cancer in an individual comprising administering to the individual: a) an effective amount of a composition comprising nanoparticles comprising a taxane and an albumin, and b) an effective amount of a hedgehog inhibitor. In some embodiments, the hedgehog inhibitor inhibits the activity of Smoothened. In some embodiments, the hedgehog inhibitor is a cyclopamine or derivative thereof. In some embodiments, the hedgehog inhibitor is selected from the group consisting of GDC-0449, XL139, IPI926, IPI609, and LDE225. In some embodiments according to any of the methods described above, the method further comprises administering an effective amount of gemcitabine. In some embodiments according to any of the methods described above, the cancer to be treated is selected from the group consisting of basal cell carcinoma, medulloblastoma, glioblastoma, multiple myeloma, chronic myelogenous leukemia (CML), acute myelogenous leukemia, pancreatic cancer, lung cancer (small cell lung cancer and non-small cell lung cancer), esophageal cancer, stomach cancer, biliary cancer, prostate cancer, liver cancer, hepatocellular cancer, gastrointestinal cancer, gastric cancer, and ovarian and bladder cancer. In some embodiments, the cancer is pancreatic cancer. In some embodiments, the cancer is basal cell carcinoma.

In some embodiments according to any of the methods described above, the composition comprising nanoparticles comprising taxane and albumin and the hedgehog inhibitor are administered simultaneously. In some embodiments, the composition comprising nanoparticles of taxane comprising albumin and the hedgehog inhibitor are administered sequentially.

In some embodiments according to any of the methods described above, the taxane is paclitaxel. In some embodiments, the taxane is docetaxel.

In some embodiments according to any of the methods described above, the average diameter of the nanoparticles in the composition is no greater than about 200 nm. In some embodiments according to any of the methods described above, the weight ratio of the albumin and the taxane in the nanoparticle composition is less than about 1:1 to 9:1. In some embodiments according to any of the methods described above, the nanoparticle composition is substantially free of Cremophor. In some embodiments according to any of the methods described above, the individual is a human. In some embodiments according to any of the methods described above, the hedgehog inhibitor is administered orally. In some embodiments according to any of the methods described above, the hedgehog inhibitor is administered prior to the administration of the nanoparticle composition. In some embodiments, the hedgehog inhibitor is administered after the administration of the nanoparticle composition.

### Proliferative diseases in art individual to be treated

The methods described herein are useful for treating proliferative diseases, which generally comprise administration of the combination of the nanoparticle taxane composition and the hedgehog inhibitor to an individual.

In some embodiments, the individual is human. In some embodiments, the individual is a man. In some embodiments, the individual is a woman. In some embodiments, the individual is more than about 50 years old, such as more than about any of 55, 60, 65, 70 years old. In some embodiments, the individual has previously been diagnosed with diabetes.

The individual (such as human) may have advanced disease or lesser extent of disease, such as low tumor burden. In some embodiments, the individual is at an early stage of a proliferative disease (such as cancer). In some embodiments, the individual is at an advanced stage of a proliferative disease (such as an advanced cancer). In some embodiments, the individual is HER2 positive. In some embodiments, the individual is HER2 negative. In some embodiments, the individual is SPARC positive. In some embodiments, the individual is SPARC negative. In some embodiments, the individual has a mutation (for example in Patched or Smoothened) that leads to constitutive activation of a hedgehog signaling pathway. In some embodiments, the individual has increased expression in a Gli protein (such as Gli-1).

In some embodiments, the individual has a disease that is refractory to treatment of a nanoparticle composition of taxane alone and/or combination of a nanoparticle composition of taxane with an agent other than a hedgehog inhibitor. In some embodiments, the individual has a disease that is refractory to treatment of a hedgehog inhibitor alone and/or combination of a hedgehog inhibitor with an agent other than a nanoparticle composition of a taxane (such as a non-nanoparticle composition of taxane, for example Taxol®). In some embodiments, the individual has a disease that is refractory to treatment of gemcitabine alone and/or a combination of gemcitabine and an agent other than a hedgehog inhibitor or a nanoparticle composition of taxane.

In some embodiments, the individual is not responsive (or partially responsive) to the treatment of a nanoparticle composition of taxane alone and/or combination of a nanoparticle composition of taxane with an agent other than a hedgehog inhibitor. In some embodiments, the individual is not responsive (or partially responsive) to the treatment of a hedgehog inhibitor alone and/or combination of a hedgehog inhibitor with an agent other than a nanoparticle composition of a taxane. In some embodiments, there is provided a method of treating an individual having a disease that is not responsive to the treatment of gemcitabine and/or a combination of gemcitabine and an agent other than a hedgehog inhibitor or a nanoparticle composition of a taxane (such as a non-nanoparticle composition of taxane, for example Taxol®).

The methods may be practiced in an adjuvant setting. The methods provided herein may also be practiced in a neoadjuvant setting. In some embodiments, the individual has previously been treated. In some embodiments, the individual has not previously been treated. In some embodiments, the treatment is a first line therapy.

The methods described herein are useful for treating proliferative diseases. In some embodiments, there is provided a method of reducing cell proliferation and/or cell migration. In some embodiments, there is provided a method of treating any of the following diseases: restenosis, stenosis, fibrosis, angiogenesis, psoriasis, atherosclerosis, and proliferation of smooth muscle cells. The present invention also provides methods of delaying development of any of the proliferative diseases described herein.

In some embodiments, there is provided a method of treating a primary tumor. In some embodiments, there is provided a method of treating metastatic cancer (that is, cancer that has metastasized from the primary tumor). In some embodiments, there is provided a method of treating a proliferative disease (such as cancer) (and in broader aspect method of treating a proliferative disease) at advanced stage(s).

In some embodiments, the disease is a cancer of any one of the following: basal cell carcinoma, medulloblastoma, glioblastoma, multiple myeloma, chronic myelogenous leukemia (CML), acute myelogenous leukemia, pancreatic cancer, lung cancer (small cell lung cancer and non-small cell lung cancer), esophageal cancer, stomach cancer, biliary cancer, prostate cancer, liver cancer, hepatocellular cancer, gastrointestinal cancer, gastric cancer, and ovarian and bladder cancer. In some embodiments, the cancer is selected from the group consisting of pancreas ductal adenocarcinoma, colon adenocarcinoma, and ovary cystadenocarcinoma. In some embodiments, the cancer is pancreas ductal adenocarcinoma. In some embodiments, the cancer is a tumor that is poorly perfused and/or poorly vascularized.

In some embodiments, the cancer is pancreatic cancer, including for example pancreatic adenocarcinoma, pancreatic adenosquamous carcinoma, pancreatic squamous cell carcinoma, and pancreatic giant cell carcinoma. In some embodiments, the pancreatic cancer is exocrine pancreatic cancer. In some embodiments, the pancreatic cancer is endocrine pancreatic cancer (such as islet cell carcinoma). In some embodiments, the pancreatic cancer is advanced metastatic pancreatic cancer.

In some embodiments, the cancer is breast cancer (which may be HER2 positive or HER2 negative), including, for example, advanced breast cancer, stage IV breast cancer, locally advanced breast cancer, and metastatic breast cancer.

In some embodiments, the cancer is lung cancer, including, for example, non-small cell lung cancer (NSCLC, such as advanced NSCLC), small cell lung cancer (SCLC, such as advanced SCLC), and advanced solid tumor malignancy in the lung.

In some embodiments, the cancer is medulloblastoma, rhabdomyosarcoma, melanoma, basal cell carcinoma, colon cancer, breast cancer, lung cancer, liver cancer, stomach cancer, prostate cancer, or pancreatic cancer.

Other examples of cancers that may be treated bv the methods of the invention include, but are not limited to, adrenocortical carcinoma, angiogenic myeloid metaplasia, AIDS-related cancers (e.g., AIDS-related lymphoma), anal cancer, appendix cancer, astrocytoma (e.g., cerebellar and cerebral), basal cell carcinoma, bile duct cancer (e.g., extrahepatic), bladder cancer, bone cancer, (osteosarcoma and malignant fibrous histiocytoma), brain tumor (e.g., glioma, brain stem glioma, cerebellar or cerebral astrocytoma (e.g., pilocytic astrocytoma, diffuse astrocytoma, anaplastic (malignant) astrocytoma), malignant glioma, ependymoma, oligodendroglioma, meningioma, craniopharyngioma, haemangioblastomas, medulloblastoma, supratentorial primitive neuroectodermal tumors, visual pathway and hypothalamic glioma, and glioblastoma), breast cancer, bronchial adenomas/carcinoids, carcinoid tumor (e.g., gastrointestinal carcinoid tumor), carcinoma of unknown primary, central nervous system lymphoma, cervical cancer, colon cancer, colorectal cancer, chronic myeloproliferative disorders, endometrial cancer (e.g., uterine cancer), ependymoma, esophageal cancer, Ewing's family of tumors, eye cancer (e.g., intraocular melanoma and retinoblastoma), gallbladder cancer, gastric (stomach) cancer, gastrointestinal carcinoid tumor, gastrointestinal stromal tumor (GIST), germ cell tumor, (e.g., extracranial, extragonadal, ovarian), gestational trophoblastic tumor, head and neck cancer, hepatocellular (liver) cancer (e.g., hepatic carcinoma and hepatoma), hepatoma hypopharyngeal cancer, islet cell carcinoma (endocrine pancreas), laryngeal cancer, laryngeal cancer, leukemia, lip and oral cavity cancer, oral cancer, liver cancer, lung cancer (e.g., small cell lung cancer, non-small cell lung cancer, adenocarcinoma of the lung, and squamous carcinoma of the lung), lymphoid neoplasm (e.g., lymphoma), medulloblastoma, melanoma, mesothelioma, metastatic squamous neck cancer, mouth cancer, multiple endocrine neoplasia syndrome, myelodysplastic syndromes, myelodysplastic/myeloproliferative diseases, nasal cavity and paranasal sinus cancer, nasopharyngeal cancer, neuroblastoma, neuroendocrine cancer, oropharyngeal cancer, ovarian cancer (e.g., ovarian epithelial cancer, ovarian germ cell tumor, ovarian low malignant potential tumor), pancreatic cancer, parathyroid cancer, penile cancer, cancer of the peritoneal, pharyngeal cancer, pheochromocytoma, pineoblastoma and supratentorial primitive neuroectodermal tumors, pituitary tumor, pleuropulmonary blastoma, lymphoma, primary central nervous system lymphoma (microglioma), pulmonary lymphangiomyomatosis, rectal cancer, renal cancer, renal pelvis and ureter cancer (transitional cell cancer), rhabdomyosarcoma, salivary gland cancer, skin cancer (e.g., non-melanoma (e.g., squamous cell carcinoma), melanoma, and Merkel cell carcinoma), small intestine cancer, squamous cell cancer, testicular cancer, throat cancer, thymoma and thymic carcinoma, thyroid cancer, tuberous sclerosis, urethral cancer, vaginal cancer, vulvar cancer, Wilms' tumor, and post-transplant lymphoproliferative disorder (PTLD), abnormal vascular proliferation associated with phakomatoses, edema (such as that associated with brain tumors), and Meigs' syndrome.

In some embodiments, the cancer is a solid tumor (such as advanced solid tumor). Solid tumor includes, but is not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, Kaposi's sarcoma, soft tissue sarcoma, uterine synovial sarcoma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma (including for example adenocarcinoma, clear cell renal cell carcinoma, papillary renal cell carcinoma, chromophobe renal cell carcinoma, collecting duct renal cell carcinoma, granular renal cell carcinoma, mixed granular renal cell carcinoma, renal angiomyolipomas, or spindle renal cell carcinoma.), hepatoma, bile duct carcinoma, choriocarcinoma, seminoma, embryonal carcinoma, Wilms' tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma.

In some embodiments the lymphoid neoplasm (*e.g.,* lymphoma) is a B-cell neoplasm. Examples of B-cell neoplasms include, but are not limited to, precursor B-cell neoplasms (*e.g.,* precursor B-lymphoblastic leukemia/lymphoma) and peripheral B-cell neoplasms (*e.g*., B-cell chronic lymphocytic leukemia/prolymphocytic leukemia/small lymphocytic lymphoma (small lymphocytic (SL) NHL), lymphoplasmacytoid lymphoma/immunocytoma, mantel cell lymphoma, follicle center lymphoma, follicular lymphoma (*e.g.,* cytologic grades: I (small cell), II (mixed small and large cell), III (large cell) and/or subtype: diffuse and predominantly small cell type), low grade/follicular non-Hodgkin's lymphoma (NHL), intermediate grade/follicular NHL, marginal zone B-cell lymphoma (*e.g.,* extranodal (*e.g.,* MALT-type +/- monocytoid B cells) and/or Nodal *(e.g.,* +/monocytoid B cells)), splenic marginal zone lymphoma (*e.g.,* +/- villous lymphocytes), Hairy cell leukemia, plasmacytoma/plasma cell myeloma (*e.g.,* myeloma and multiple myeloma), diffuse large B-cell lymphoma (*e.g.,* primary mediastinal (thymic) B-cell lymphoma), intermediate grade diffuse NHL, Burkitt's lymphoma, High-grade B-cell lymphoma, Burkitt-like, high grade immunoblastic NHL, high grade lymphoblastic NHL, high grade small non-cleaved cell NHL, bulky disease NHL, AIDS-related lymphoma, and Waldenstrom's macroglobulinemia).

In some embodiments the lymphoid neoplasm (*e.g.,* lymphoma) is a T-cell and/or putative NK-cell neoplasm. Examples of T-cell and/or putative NK-cell neoplasms include, but are not limited to, precursor T-cell neoplasm (precursor T-lymphoblastic lymphoma/leukemia) and peripheral T-cell and NK-cell neoplasms (*e.g*., T-cell chronic lymphocytic leukemia/prolymphocytic leukemia, and large granular lymphocyte leukemia (LGL) (*e.g.,* T-cell type and/or NK-cell type), cutaneous T-cell lymphoma (*e.g.,* mycosis fungoides/Sezary syndrome), primary T-cell lymphomas unspecified (*e.g.*, cytological categories (*e.g.*, medium-sized cell, mixed medium and large cell), large cell, lymphoepitheloid cell, subtype hepatosplenic yd T-cell lymphoma, and subcutaneous panniculitic T-cell lymphoma), angioimmunoblastic T-cell lymphoma (AILD), angiocentric lymphoma, intestinal T-cell lymphoma (*e.g.*, +/- enteropathy associated), adult T-cell lymphoma/leukemia (ATL), anaplastic large cell lymphoma (ALCL) (*e.g.,* CD30+, T- and null-cell types), anaplastic large-cell lymphoma, and Hodgkin's like).

In some embodiments the lymphoid neoplasm (*e.g.,* lymphoma) is Hodgkin's disease. For example, the Hodgkin's disease may be lymphocyte predominance, nodular sclerosis, mixed cellularity, lymphocyte depletion, and/or lymphocyte-rich.

In some embodiments, the cancer is leukemia. In some embodiments, the leukemia is chronic leukemia. Examples of chronic leukemia include, but are not limited to, chronic myelocytic I (granulocytic) leukemia, chronic myelogenous, and chronic lymphocytic leukemia (CLL). In some embodiments, the leukemia is acute leukemia. Examples of acute leukemia include, but are not limited to, acute lymphoblastic leukemia (ALL), acute myeloid leukemia, acute lymphocytic leukemia, and acute myelocytic leukemia (e.g., myeloblastic, promyelocytic, myelomonocytic, monocytic, and erythroleukemia).

In some embodiments, the cancer is liquid tumor or plasmacytoma. Plasmacytoma includes, but is not limited to, myeloma. Myeloma includes, but is not limited to, an extramedullary plasmacytoma, a solitary myeloma, and multiple myeloma. In some embodiments, the plasmacytoma is multiple myeloma.

In some embodiments, the cancer is multiple myeloma. Examples of multiple myeloma include, but are not limited to, IgG multiple myeloma, IgA multiple myeloma, IgD multiple myeloma, IgE multiple myeloma, and nonsecretory multiple myeloma. In some embodiments, the multiple myeloma is IgG multiple myeloma. In some embodiments, the multiple myeloma is IgA multiple myeloma. In some embodiments, the multiple myeloma is a smoldering or indolent multiple myeloma. In some embodiments, the multiple myeloma is progressive multiple myeloma. In some embodiments, multiple myeloma may be resistant to a drug, such as, but not limited to, bortezomib, dexamethasone (Dex-), doxorubicin (Dox-), and melphalan (LR).

### Hedgehog inhibitors,

The methods described herein comprise administration of a hedgehog inhibitor, namely, an agent that inhibits a hedgehog signaling pathway, for example by affecting the activity of one or more components of the hedgehog signaling pathway, either directly or indirectly. In some embodiments, the hedgehog inhibitor is present in a nanoparticle composition, such as nanoparticle compositions described herein.

Generally, hedgehog signaling occurs through the interaction of hedgehog protein with hedgehog receptor, Patched and the co-receptor Smoothened. There are at least two mammalian homologues of Patched, Ptch-1 and Ptch-2, both of which are 12 transmembrane proteins containing a sterol sensing domain. The binding of hedgehog and Patched activates Smoothened, a seven transmembrane G-coupled protein, which in turn triggers a signaling cascade that results in the regulation of transcription by zinc-finger transcription factors of the Gli family (Gli-1, Gli-2, and Gli-3).

In the cancer context, the hedgehog signaling pathway may be activated in the absence of hedgehog through activation of a downstream component, e.g., by overexpression/activation of Smoothened and/or loss-of-function mutations in Patched, that result in constitutive activation of the hedgehog signaling in the absence of hedgehog.

In some embodiments, the hedgehog inhibitor inhibits the Sonic hedgehog signaling pathway. In some embodiments, the hedgehog inhibitor inhibits the Indian hedgehog signaling pathway. In some embodiments, the hedgehog inhibitor inhibits the Desert hedgehog signaling pathway. In some embodiments, the hedgehog inhibitor inhibits two or more of the Sonic hedgehog signaling pathway, the Indian hedgehog signaling pathway, and the Desert hedgehog signaling pathway.

Each hedgehog signaling component, depending on their biological function and effects on the final outcome of the downstream gene activation or expression, can be classified as either positive or negative regulators. A positive regulator is a hedgehog signaling component that positively affects the transmission of the hedgehog signal, i.e., stimulates downstream biological events when hedgehog is present. A negative regulator is a hedgehog signaling component that negative affects the transmission of the hedgehog signal, i.e. inhibits downstream biological events when hedgehog is present. The hedgehog inhibitors described herein can either act by suppressing a positive regulator of the hedgehog signaling pathway or by activating or enhancing a negative regulator of the hedgehog signaling pathway.

Thus, for example, the hedgehog inhibitor may inhibit the Hedgehog signaling pathway by any one or more of the following: 1) blocking the ability of hedgehog to transduce a signal, such as by blocking a native hedgehog ligand (e.g., Shh, Dhh, Ihh) from binding to a receptor; 2) blocking a hedgehog receptor (e.g., Ptc-1, Ptc-2, Smo, etc.) from transmitting signals to a downstream component in the hedgehog signaling pathway, 3) blocking the potentiating or stimulating activity of a positive regulatory hedgehog signaling component, or 4) activating or enhancing the repressive activity of a negative regulatory hedgehog signaling component.

In some embodiments, the hedgehog inhibitor blocks the interaction between hedgehog and its receptor (such as Ptc-1 or Ptc-2). In some embodiments, the hedgehog inhibitor inhibits the activity of Smoothened. In some embodiments, the hedgehog inhibitor affects the activity of a hedgehog signaling pathway component, including for example Patched (such as Ptc-1 or Pct-2), Smoothened, Gli (including Gli-1, Gli-2, and Gli-3), Bmi-1, Fused, Suppressor of Fused, Costal2 (Cos2), HIP1 (Hedgehog interacting protein), and Rab23.

In some embodiments, the hedgehog inhibitor inhibits the signaling event induced by the activation of Smoothened. In some embodiments, the hedgehog inhibitor directly targets Smoothened. In some embodiments, the hedgehog inhibitor inhibits one or more signaling component(s) of downstream of Smoothened. For example, in some embodiments, there is provided a method of treating a proliferative disease (such as cancer) in an individual, comprising administering to the individual: a) an effective amount of a composition comprising nanoparticles comprising a taxane and a carrier protein (such as albumin); and b) an effective amount of an agent that inhibits the activity of Smoothened. In some embodiments, there is provided a method of treating a proliferative disease (such as cancer) in an individual, comprising administering to the individual: a) an effective amount of a composition comprising nanoparticles comprising a taxane and a carrier protein (such as albumin); and b) an effective amount of an agent that directly target Smoothened.

Hedgehog inhibitors have been disclosed in PCT Patent Application Nos. PCT/US2010/037986 and PCT/US2010/037717, the content of each of which is incorporated by reference herein in their entirety.

Hedgehog inhibitors of the present invention include isoquinoline and quinazoline compounds of Formula (I): 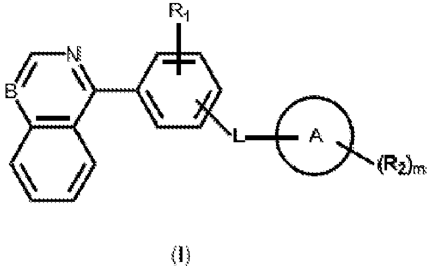 or a pharmaceutically acceptable salt thereof, wherein:
B is N or CH;
R₁ represents hydrogen, halogen, hydroxyl, amino, nitro, cyano, alkyl, alkenyl, alkoxy, alkoxycarbonyl, carbamoyl, alkylthio, sulfonyl, sulfinyl, cycloalkyl or a heterocycle;
L is oxygen, NR₃, NR₃CO, NR₃SO, NR₃SO₂, SO₂NR₃; NR₃CONH, NR₃CSNH, CONR₃, CSNR₃, NR₃CHR₄, NR₃PO or NR₃PO(OH);
Ring A is aryl, heterocycle, heteroaryl;
R₂ represents hydrogen, hydroxyl, halogen, amino, nitro, cyano, acyl, alkyl, alkenyl, alkynyl, alkylthio, sulfonyl, sulfinyl, alkoxy, alkoxycarbonyl, carbamoyl, acylamine, sulfamoyl or sulfonamide;
or R₂ is a aryl, heterocycle or heteroaryl that is optionally substituted with hydroxyl, halogen, amino, nitro, cyano, acyl, alkyl, alkanoyl, sulfonyl, sulfinyl, alkoxy, carbamoyl, acylamine, sulfamoyl and sulfonamide;
R₃ and R₄ are independently selected from hydrogen or an optionally substituted C₁-C₄ alkyl group; and
m is 0-4.

In a some embodiments, the hedgehog inhibitor is an isoquinoline compound of Formula (Ia): 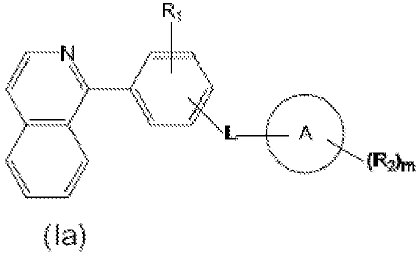 or a pharmaceutically acceptable salt, wherein L, R₁, R₂, R₃, R₄, and m are defined as for Formula (I).

In some embodiments, the hedgehog inhibitor is a quinazoline compound of Formula (Ib): 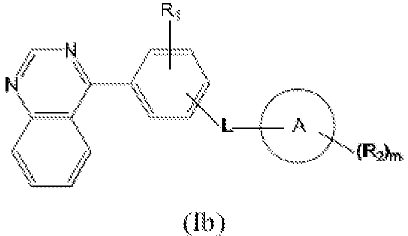 or a pharmaceutically acceptable salt, wherein L, R₁, R₂, R₃, R₄, and m are as defined for Formula (I).

In some embodiments, the hedgehog inhibitor is an isoquinoline or quinazoline compound of Formula (Ic): 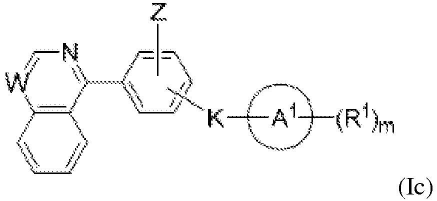 or a pharmaceutically acceptable salt thereof, wherein:
K is selected from NR³C(O), C(O)NR³, NR³S(O₂), S(O₂)NR³, and NR⁴C(O)NR⁵;
A¹ is selected from aryl, heterocyclyl, and heteroaryl;
R¹ is selected from H, halo, nitro, -OR⁴, C₁-C₆ alkyl, C₁-C₆ alkylsulfonyl, and C₁-C₆ haloalkyl;
m = 0-4;
R³, R⁴, and R⁵ are each independently selected from H and C₁-C₆ alkyl;
W is selected from CH and N;
Z is selected from H, halo, and C₁-C₆ alkyl, C₁-C₆ alkylthio, -NR⁴R⁵, -OR⁴, and cyano.

In some embodiments of Formula (IIc),
K is selected from NR³C(O), C(O)NR³, NR³S(O₂), S(O₂)NR³, and NR⁴C(O)NR⁵;
A¹ is selected from phenyl and pyridyl;
R¹ is selected from H, halo, nitro, C₁-C₆ alkylsulfonyl, and C₁-C₆ alkyl;
m = 0-4;
R³, R⁴, and R⁵ are each independently selected from H and C₁-C₆ alkyl;
W is selected from CH and N; and
Z is selected from H, halo, and C₁-C₆ alkyl.

In some embodiments, the hedgehog inhibitor is selected from the isoquinoline and quinazoline compounds provided in Table 1. In some embodiments, the hedgehog inhibitor is selected from the isoquinoline and quinazoline compounds provided in Table 4. Table 4 provides exemplary isoquinoline and quinazoline compounds of hedgehog inhibitors.

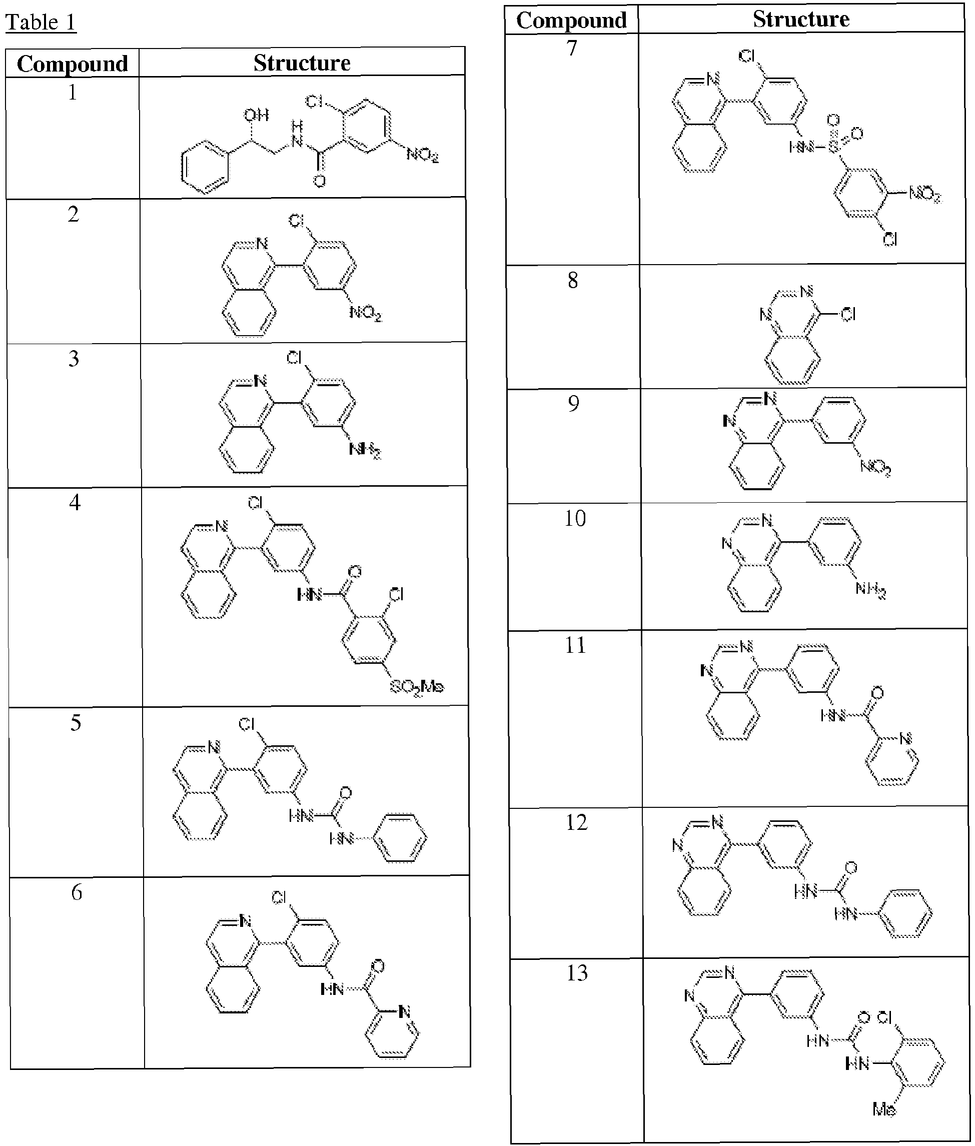

Hedgehog inhibitors of the present invention include triazine compounds of Formula (II): 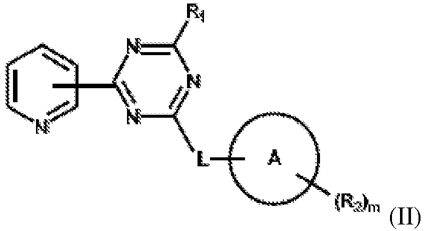 or a pharmaceutically acceptable salt thereof, wherein:
L is NR₃CO, NR₃SO₂, NR₃CONH, NR₃CSNH or NR₃CHR₄;
R₁ is selected from:
   (i) amino, alkyl amino, aryl amino, heteroaryl amino;
   (ii) Alkylthio, sulfinyl, sulfonyl, sulfamoyl;
   (iii) Alkyloxy, Alkanoyl, alkoxycarbonyl;
   (iv) Hydrogen, C₁-C₆ alkyl, cycloalkyl, C₂-C₆ alkenyl, C₂-C₆ alkynyl;
   (v) aryl, heterocyclic, heteroaryl;
   (vi) C₁-C₆ trifluoroalkyl, cyano and
   (vii) groups of the formula (a): 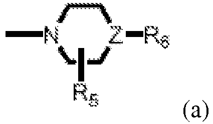 wherein:
      R₅ represents hydrogen, C₁-C₄ alkyl, oxo;
      Z is CH, when R₆ is hydrogen; or Z-R₆ is O; or Z is N, R₆ represents groups of hydrogen, C₁-C₆ alkyl, C₂-C₆ alkenyl, C₂-C₆ alkynyl, C₃-C₁₀ aryl or heteroaryl, (C₃-C₇ cycloalkyl)C₁-C₄ alkyl, C₁- C₆ haloalkyl, C₁-C₆ alkoxy, C₁- C₆ alkylthio, C₂-C₆ alkanoyl, C₁-C₆ alkoxycarbonyl, C₂- C₆ alkanoyloxy, mono- and di-(C₃-C₈ cycloalkyl)aminoC₀-C₄ alkyl, (4- to 7- membered heterocycle)C₀-C₄alkyl, C₁-C₆ alkylsulfonyl, mono- and di-(C₁- C₆ alkyl) sulfonamido, and mono- and di-(C₁- C₆ alkyl)aminocarbonyl, each of which is substituted with from 0 to 4 substituents independently chosen from halogen, hydroxy, cyano, amino, - COOH and oxo;
      Ring A is aryl, heterocycle, heteroaryl;
      R₂ is hydroxyl, halogen, amino, nitro, cyano, alkyl, alkenyl, alkynyl, alkanoyl, alkylthio, sulfonyl, sulfinyl, alkoxy, alkoxycarbonyl, carbamoyl, acylamine, sulfamoyl or sulfonamide;
      or R₂ is a aryl, heterocycle or heteroaryl that is optionally substituted with hydroxyl, halogen, amino, nitro, cyano, alkyl, acyl, sulfonyl, sulfinyl, alkoxy, carbamoyl, acylamine, sulfamoyl and sulfonamide;
      R₃ and R₄ are independently selected from hydrogen or an optionally substituted C₁-₄ alkyl group; and
      m is 0-4.

In some embodiments, the hedgehog inhibitor is a triazine compound of Formula (IIa): 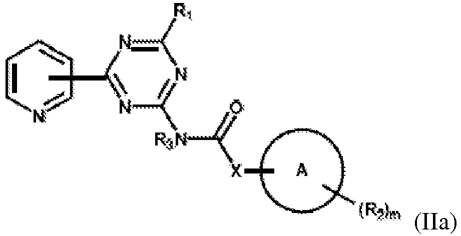
wherein A, R₁, R₂, R₃, R₄, and m are as defined for Formula (II), and
X is absent, O, CR₄R₇ or NR₃; and
R₇ is hydrogen or an optionally substituted C₁-C₄ alkyl group.

In some embodiments, the hedgehog inhibitor is a triazine compound of Formula (IIb): 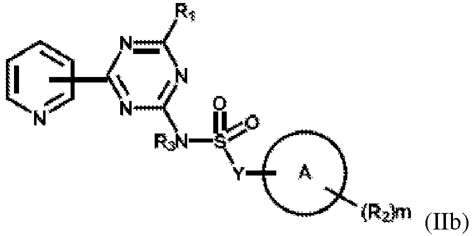
wherein A, R₁, R₂, R₃ and m are as defined for Formula (II), and
Y is absent or CR₄R₇; and
R₄ and R₇ are as defined for Formulas (II) and (IIa).

In some embodiments, the hedgehog inhibitor is a triazine compound of Formula (IIc): 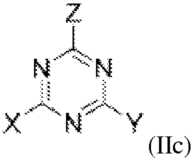 or a pharmaceutically acceptable salt thereof, wherein:
Y is selected from -K-A¹-R¹;
K is selected from NR³C(O) and NR⁴C(O)NR⁵;
A¹ is selected from aryl, heteroaryl, and heterocyclyl;
R¹ is one or more substituents independently selected from H, halo, nitro, C₁-C₆ alkylsulfonyl, -OR⁴, C₁-C₆ alkyl, and C₁-C₆ haloalkyl;
R³ is selected from H, C₁-C₆ alkyl, and -C(O)-A¹-R¹;
R⁴ and R⁵ are each independently selected from H and C₁-C₆ alkyl;
X is pyridinyl;
Z is selected from H, C₁-C₆ alkyl, C₁-C₆ alkylthio, C₁-C₆ alkoxy, C₁-C₆ haloalkyl, - NR⁴R⁵, and cyano.

In some embodiments of Formula (IIc),
Y is -K-A¹-R¹;
K is selected from NR³C(O) and NR⁴C(O)NR⁵;
A¹ is selected from phenyl and furanyl;
R¹ is one or more substituents independently selected from H, halo, nitro, C₁-C₆ alkylsulfonyl, -OR⁴, C₁-C₆ alkyl, and C₁-C₆ haloalkyl;
R³ is selected from H, C₁-C₆ alkyl, and -C(O)-A¹-R¹;
R⁴ and R⁵ are each independently selected from H and C₁-C₆ alkyl;
X is pyridinyl;
Z is selected from C₁-C₆ alkyl, C₁-C₆ alkylthio, and -NR⁴R⁵.

In some embodiments, the hedgehog inhibitor is selected from the triazine compounds provided in Table 2. In some embodiments, the hedgehog inhibitor is selected from the triazine compounds provided in Table 5. Table 5 provides exemplary tetrazine compounds of hedgehog inhibitors.

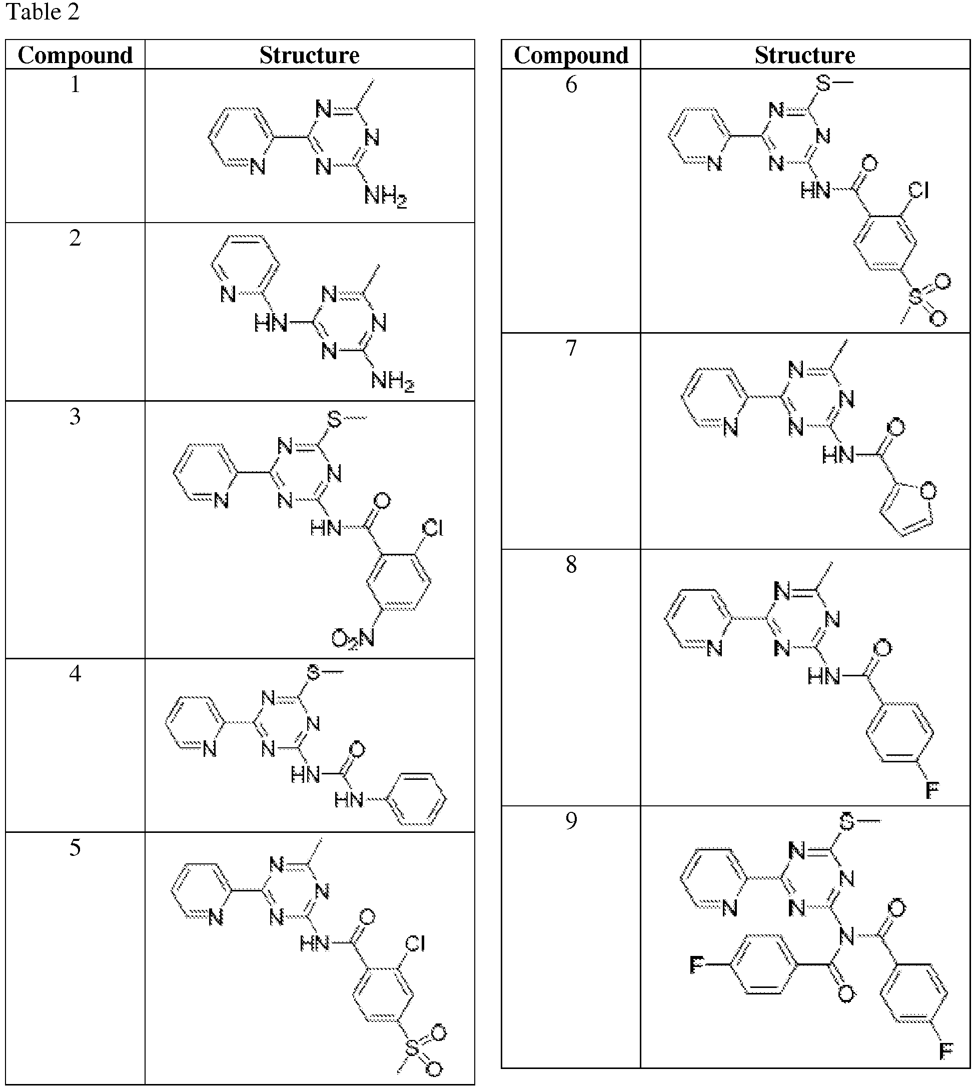

Other hedgehog inhibitors are known in the art, and include for example small molecule compounds, small peptides, antibodies, antisense oligonucleotides, siRNAs, and the like. In some embodiments, the hedgehog inhibitor is a small molecule compound. In some embodiments, the hedgehog inhibitor is a cyclopamine or derivative thereof. In some embodiments, the hedgehog inhibitor is Jervine, GANT61, purmorphamine, SAG, SANT-2, tomatidine, zerumbone, or derivatives thereof. In some embodiments, the hedgehog inhibitor is any of the following compounds: GDC-0449 (available from Genentech and/or Curis); XL139, IPI926 (available from Infinity Pharmaceuticals), IPI609 (IPI269609) or LDE225. In some embodiments, the hedgehog inhibitor is selected from the compounds provided in Table 3.

**Table 3**

| Compound | Name | Chemical Structure |
|---|---|---|
| 1 | GDC-0449 | 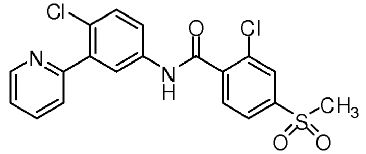 |
| 2 | IPI926 | 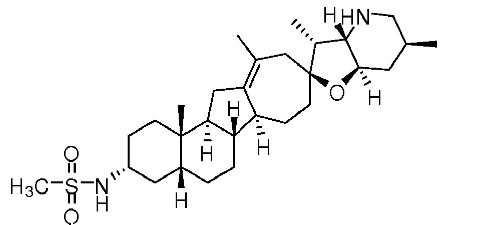 |
| 3 | Jervine | 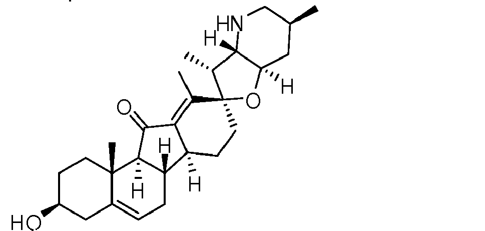 |
| 4 | GANT61 | 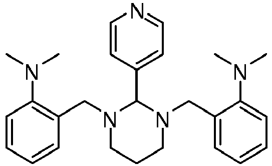 |
| 5 | Purmorphamine | 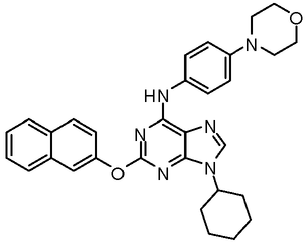 |
| 6 | SAG | 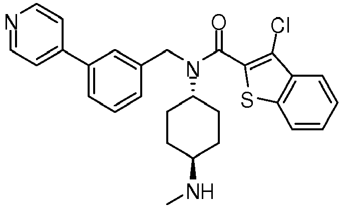 |
| 7 | SANT-2 | 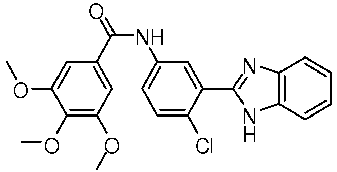 |
| 8 | Tomatidine | 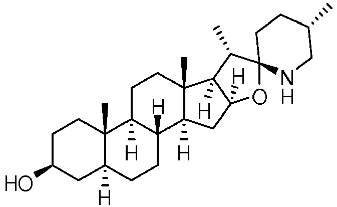 |
| 9 | Zerumbone | 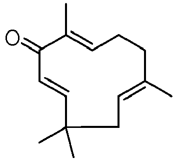 |
| 10 | IPI609 (IPI269609) | 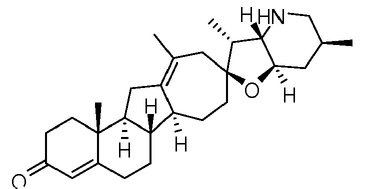 |
| 11 | XL139 | |
| 12 | LDE225 | |

In some embodiments, the hedgehog inhibitor is NVP-LDE225 (available from Novartis) or BMS-833923/XL139 (available from Bristol-Myers Squibb and/or Exelixis).

Additional hedgehog inhibitors are provided in Rubin et al., Nature Reviews Drug Discovery 5, 1026-1033 (2006); Brunton et al., J. Medicinal Chemistry, 51(5):1108-1110 (2008); Romer et al., Cancer Research 65:4975-4978 (2005); Chen et al., Proc. Nat. Acad. Sci. 99(22):14071-14076 (2002); Taipale et al., Nature 418: 892-897 (2002); Taipale et al., Nature 406:1005-1009 (2000); WO2009/086416, U.S. Pat. Pub. Nos. US20080019961, US20050112125; US20050222087; US20050085519; US20040038876; US20040127474; US20040110663, and US20030166543, all of which are herein incorporated by reference in their entireties.

The hedgehog inhibitors described herein can be the agents themselves, pharmaceutically acceptable salts thereof, pharmaceutically acceptable esters thereof, as well as stereoisomers, enantiomers, racemic mixtures, and the like. As discussed above, in some embodiments, the hedgehog inhibitor is provided in the form of a nanoparticle, comprising a hedgehog inhibitor and a carrier protein (such as albumin), such as the nanoparticle composition described herein.

Reference to an agent herein also applies to the agent or its derivatives and accordingly the invention contemplates and includes either of these embodiments (agent; agent or derivative(s)). "Derivatives" or "analogs" of an agent or other chemical moiety include, but are not limited to, compounds that are structurally similar to the hedgehog inhibitor or moiety or are in the same general chemical class as the hedgehog inhibitor or moiety. In some embodiments, the derivative or analog of the hedgehog inhibitor or moiety retains similar chemical and/or physical property (including, for example, functionality) of the hedgehog inhibitor or moiety.

### Nanoparticle compositions,

The methods described herein comprise administration of a nanoparticle composition of taxane and in some embodiments a nanoparticle composition of a hedgehog inhibitor. Nanoparticles of poorly water soluble drugs (such as taxane) have been disclosed in, for example, U.S. Pat. Nos. 5,916,596; 6,506,405; 6,749,868, and 6,537,579 and also in U.S. Pat. Pub. Nos. 2005/0004002 and 2007/0082838. Although the description provided below is specific to taxanes, it is understood that the same applies to the hedgehog inhibitors described herein. For examples, there are provided in some embodiments compositions comprising nanoparticles comprising (in various embodiments consisting essentially of) a hedgehog inhibitor (such as any one of the hedgehog inhibitors described herein) and a carrier protein (such as albumin). As discussed above, the nanoparticle compositions of the hedgehog inhibitor can be used in combination with a nanoparticle composition of the taxane.

In some embodiments, the composition comprises nanoparticles with an average or mean diameter of no greater than about 1000 nanometers (nm), such as no greater than about any of 900, 800, 700, 600, 500, 400, 300, 200, and 100 nm. In some embodiments, the average or mean diameters of the nanoparticles is no greater than about 200 nm. In some embodiments, the average or mean diameters of the nanoparticles is no greater than about 150 nm. In some embodiments, the average or mean diameters of the nanoparticles is no greater than about 100 nm. In some embodiments, the average or mean diameter of the nanoparticles is about 20 to about 400 nm. In some embodiments, the average or mean diameter of the nanoparticles is about 40 to about 200 nm. In some embodiments, the nanoparticles are sterile-filterable.

In some embodiments, the nanoparticles in the composition described herein have an average diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least about any one of 60%, 70%, 80%, 90%, 95%, or 99%) of all the nanoparticles in the composition have a diameter of no greater than about 200 nm, including for example no greater than about any one of 190, 180, 170, 160, 150, 140, 130, 120, 110, 100, 90, 80, 70, or 60 nm. In some embodiments, at least about 50% (for example at least any one of 60%, 70%, 80%, 90%, 95%, or 99%) of all the nanoparticles in the composition fall within the range of about 20 to about 200 nm, including for example any one of about 30 to about 180 nm, and any one of about 40 to about 150, about 50 to about 120, and about 60 to about 100 nm.

Particle size can be determined by methods known in the art. For example, the size of the particles can be determined using photon correlation spectroscopy or dynamic light scattering. Instruments used for measure the size of submicron particles are known in the art and include, for example, Malvern zetasizers, Malvern autosizers, Coutler N4, and Amtec. In some embodiments, the average particle size used herein refers to Z-average (for example a harmonic Z-average).

In some embodiments, the carrier protein has sulfhydryl groups that can form disulfide bonds. In some embodiments, at least about 5% (including for example at least about any one of 10%, 15%, 20%, 25%, 30%, 40%, 50%, 60%, 70%, 80%, or 90%) of the carrier protein in the nanoparticle portion of the composition are crosslinked (for example crosslinked through one or more disulfide bonds).

In some embodiments, the nanoparticles comprise the taxane (such as paclitaxel) coated with a carrier protein, such as albumin (e.g., human serum albumin). In some embodiments, the composition comprises taxane in non-nanoparticle form, wherein at least about any one of 50%, 60%, 70%, 80%, 90%, 95%, or 99% of the taxane in the composition are in nanoparticle form. In some embodiments, the taxane in the nanoparticles constitutes more than about any one of 50%, 60%, 70%, 80%, 90%, 95%, or 99% of the nanoparticles by weight. In some embodiments, the nanoparticles have a non-polymeric matrix. In some embodiments, the nanoparticles comprise a core of taxane that is substantially free of polymeric materials (such as polymeric matrix).

In some embodiments, the nanoparticle composition is substantially free (such as free) of surfactants (such as Cremophor®, Tween 80, or other organic solvents used for the administration of taxanes). In some embodiments, the nanoparticle composition contains less than about any one of 20%, 15%, 10%, 7.5%, 5%, 2.5%, or 1% organic solvent. In some embodiments, the weight ratio of carrier protein (such as albumin) and taxane in the nanoparticle composition is about 18:1 or less, such as about 15:1 or less, for example about 10:1 or less. In some embodiments, the weight ratio of carrier protein (such as albumin) and taxane in the composition falls within the range of any one of about 1:1 to about 18:1, about 2:1 to about 15:1, about 3:1 to about 13:1, about 4:1 to about 12:1, about 5:1 to about 10:1. In some embodiments, the weight ratio of carrier protein and taxane in the nanoparticle portion of the composition is about any one of 1:2, 1:3, 1:4, 1:5, 1:10, 1:15, or less.

In some embodiments, the nanoparticle composition comprises one or more of the above characteristics.

The nanoparticles described herein may be present in a dry formulation (such as lyophilized composition) or suspended in a biocompatible medium. Suitable biocompatible media include, but are not limited to, water, buffered aqueous media, saline, buffered saline, optionally buffered solutions of amino acids, optionally buffered solutions of proteins, optionally buffered solutions of sugars, optionally buffered solutions of vitamins, optionally buffered solutions of synthetic polymers, lipid-containing emulsions, and the like.

The proteins described herein may be naturally occurring, i.e., obtained or derived from a natural source (such as blood), or synthesized (such as chemically synthesized or by synthesized by recombinant DNA techniques).

Examples of suitable carrier proteins include proteins normally found in blood or plasma, which include, but are not limited to, albumin, immunoglobulin including IgA, lipoproteins, apolipoprotein B, alpha-acid glycoprotein, beta-2-macroglobulin, thyroglobulin, transferin, fibronectin, factor VII, factor VIII, factor IX, factor X, and the like. In some embodiments, the carrier protein is non-blood protein, such as casein, a-lactalbumin, and ß-lactoglobulin. The carrier proteins may either be natural in origin or synthetically prepared. In some embodiments, the pharmaceutically acceptable carrier comprises albumin, such as human serum albumin. Human serum albumin (HSA) is a highly soluble globular protein of Mᵣ 65K and consists of 585 amino acids. HSA is the most abundant protein in the plasma and accounts for 70-80 % of the colloid osmotic pressure of human plasma. The amino acid sequence of HSA contains a total of 17 disulphide bridges, one free thiol (Cys 34), and a single tryptophan (Trp 214). Intravenous use of HSA solution has been indicated for the prevention and treatment of hypovolemic shock (see, e.g., Tullis, JAMA, 237, 355-360, 460-463, (1977)) and Houser et al., Surgery, Gynecology and Obstetrics, 150, 811-816 (1980)) and in conjunction with exchange transfusion in the treatment of neonatal hyperbilirubinemia (see, e.g., Finlayson, Seminars in Thrombosis and Hemostasis, 6, 85-120, (1980)). Other albumins are contemplated, such as bovine serum albumin. Use of such non-human albumins could be appropriate, for example, in the context of use of these compositions in non-human mammals, such as the veterinary (including domestic pets and agricultural context).

Human serum albumin (HSA) has multiple hydrophobic binding sites (a total of eight for fatty acids, an endogenous ligand of HSA) and binds a diverse set of taxanes, especially neutral and negatively charged hydrophobic compounds (Goodman et al., The Pharmacological Basis of Therapeutics, 9th ed, McGraw-Hill New York (1996)). Two high affinity binding sites have been proposed in subdomains IIA and IIIA of HSA, which are highly elongated hydrophobic pockets with charged lysine and arginine residues near the surface which function as attachment points for polar ligand features (see, e.g., Fehske et al., Biochem. Pharmcol., 30, 687-92 (198a), Vorum, Dan. Med. Bull., 46, 379-99 (1999), Kragh-Hansen, Dan. Med. Bull.,1441, 131-40 (1990), Curry et al., Nat. Struct. Biol., 5, 827-35 (1998), Sugio et al., Protein. Eng., 12, 439-46 (1999), He et al., Nature, 358, 209-15 (199b), and Carter et al., Adv. Protein. Chem., 45, 153-203 (1994)). Paclitaxel and propofol have been shown to bind HSA (see, e.g., Paal et al., Eur. J. Biochem., 268(7), 2187-91 (200a), Purcell et al., Biochim. Bioplys. Acta, 1478(a), 61-8 (2000), Altmayer et al., Arzneimittelƒorschung, 45, 1053-6 (1995), and Garrido et al., Rev. Esp. Anestestiol. Reanim., 41, 308-12 (1994)). In addition, docetaxel has been shown to bind to human plasma proteins (see, e.g., Urien et al., Invest. New Drugs, 14(b), 147-51 (1996)).

The carrier protein (such as albumin) in the composition generally serves as a carrier for the taxane, i.e., the carrier protein in the composition makes the taxane more readily suspendable in an aqueous medium or helps maintain the suspension as compared to compositions not comprising a carrier protein. This can avoid the use of toxic solvents (or surfactants) for solubilizing the taxane, and thereby can reduce one or more side effects of administration of the taxane into an individual (such as a human). Thus, in some embodiments, the composition described herein is substantially free (such as free) of surfactants, such as Cremophor (including Cremophor EL^{®} (BASF)). In some embodiments, the nanoparticle composition is substantially free (such as free) of surfactants.

The amount of carrier protein in the composition described herein will vary depending on other components in the composition. In some embodiments, the composition comprises a carrier protein in an amount that is sufficient to stabilize the taxane in an aqueous suspension, for example, in the form of a stable colloidal suspension (such as a stable suspension of nanoparticles). In some embodiments, the carrier protein is in an amount that reduces the sedimentation rate of the taxane in an aqueous medium. For particle-containing compositions, the amount of the carrier protein also depends on the size and density of nanoparticles of the taxane.

A taxane is "stabilized" in an aqueous suspension if it remains suspended in an aqueous medium (such as without visible precipitation or sedimentation) for an extended period of time, such as for at least about any of 0.1, 0.2, 0.25, 0.5, 1, 2, 3, 4, 5, 6, 7, 8, 9, 10, 11, 12, 24, 36, 48, 60, or 72 hours. The suspension is generally, but not necessarily, suitable for administration to an individual (such as human). Stability of the suspension is generally (but not necessarily) evaluated at a storage temperature (such as room temperature (such as 20-25 °C) or refrigerated conditions (such as 4°C)). For example, a suspension is stable at a storage temperature if it exhibits no flocculation or particle agglomeration visible to the naked eye or when viewed under the optical microscope at 1000 times, at about fifteen minutes after preparation of the suspension. Stability can also be evaluated under accelerated testing conditions, such as at a temperature that is higher than about 40°C.

In some embodiments, the carrier protein is present in an amount that is sufficient to stabilize the taxane in an aqueous suspension at a certain concentration. For example, the concentration of the taxane in the composition is about 0.1 to about 100 mg/ml, including for example any of about 0.1 to about 50 mg/ml, about 0.1 to about 20 mg/ml, about 1 to about 10 mg/ml, about 2 mg/ml to about 8 mg/ml, about 4 to about 6 mg/ml, about 5 mg /ml. In some embodiments, the concentration of the taxane is at least about any of 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, and 50 mg/ml. In some embodiments, the carrier protein is present in an amount that avoids use of surfactants (such as Cremophor), so that the composition is free or substantially free of surfactant (such as Cremophor).

In some embodiments, the composition, in liquid form, comprises from about 0.1% to about 50% (w/v) (e.g. about 0.5% (w/v), about 5% (w/v), about 10% (w/v), about 15% (w/v), about 20% (w/v), about 30% (w/v), about 40% (w/v), or about 50% (w/v)) of carrier protein. In some embodiments, the composition, in liquid form, comprises about 0.5% to about 5% (w/v) of carrier protein.

In some embodiments, the weight ratio of carrier protein, e.g., albumin, to the taxane in the nanoparticle taxane composition is such that a sufficient amount of taxane binds to, or is transported by, the cell. While the weight ratio of carrier protein to taxane will have to be optimized for different carrier protein and taxane combinations, generally the weight ratio of carrier protein, e.g., albumin, to taxane (w/w) is about 0.01:1 to about 100:1, about 0.02:1 to about 50:1, about 0.05:1 to about 20:1, about 0.1:1 to about 20:1, about 1:1 to about 18:1, about 2:1 to about 15:1, about 3:1 to about 12:1, about 4:1 to about 10:1, about 5:1 to about 9:1, about 1:1 to about 9:1, or about 9:1. In some embodiments, the carrier protein to taxane weight ratio is about any of 18:1 or less, 15:1 or less, 14:1 or less, 13:1 or less, 12:1 or less, 11:1 or less, 10:1 or less, 9:1 or less, 8:1 or less, 7:1 or less, 6:1 or less, 5:1 or less, 4:1 or less, and 3:1 or less.

In some embodiments, the carrier protein allows the composition to be administered to an individual (such as human) without significant side effects. In some embodiments, the carrier protein (such as albumin) is in an amount that is effective to reduce one or more side effects of administration of the taxane to a human. The term "reducing one or more side effects of administration of the taxane" refers to reduction, alleviation, elimination, or avoidance of one or more undesirable effects caused by the taxane, as well as side effects caused by delivery vehicles (such as solvents that render the taxanes suitable for injection) used to deliver the taxane. Such side effects include, for example, myelosuppression, neurotoxicity, hypersensitivity, inflammation, venous irritation, phlebitis, pain, skin irritation, peripheral neuropathy, neutropenic fever, anaphylactic reaction, venous thrombosis, extravasation, and combinations thereof. These side effects, however, are merely exemplary and other side effects, or combination of side effects, associated with taxanes can be reduced.

In some embodiments, the composition comprises Abraxane®. Abraxane® is a formulation of paclitaxel stabilized by human albumin USP, which can be dispersed in directly injectable physiological solution. When dispersed in a suitable aqueous medium such as 0.9% sodium chloride injection or 5% dextrose injection, Abraxane® forms a stable colloidal suspension of paclitaxel. The mean particle size of the nanoparticles in the colloidal suspension is about 130 nanometers. Since HSA is freely soluble in water, Abraxane® can be reconstituted in a wide range of concentrations ranging from dilute (0.1 mg/ml paclitaxel) to concentrated (20 mg/ml paclitaxel), including for example about 2 mg/ml to about 8 mg/ml, about 5 mg/ml.

Methods of making nanoparticle compositions are known in the art. For example, nanoparticles containing taxanes (such as paclitaxel) and carrier protein (such as albumin) can be prepared under conditions of high shear forces (e.g., sonication, high pressure homogenization, or the like). These methods are disclosed in, for example, U.S. Pat. Nos. 5,916,596; 6,506,405; 6,749,868, and 6,537,579 and also in U.S. Pat. Pub. No. 2005/0004002, 2007/0082838, 2006/0263434and PCT Application WO08/137148.

Briefly, the taxane (such as paclitaxel) is dissolved in an organic solvent, and the solution can be added to a human serum albumin solution. The mixture is subjected to high pressure homogenization. The organic solvent can then be removed by evaporation. The dispersion obtained can be further lyophilized. Suitable organic solvent include, for example, ketones, esters, ethers, chlorinated solvents, and other solvents known in the art. For example, the organic solvent can be methylene chloride or chloroform/alcohol (such as ethanol), for example with a ratio of 1:9, 1:8, 1:7, 1:6, 1:5, 1:4, 1:3, 1:2, 1:1, 2:1, 3:1, 4:1, 5:1, 6:1, 7:1, 8:1, or 9:1.

### Modes of administration

As discussed above, the composition comprising nanoparticles comprising taxane (also referred to as "nanoparticle taxane composition") and the hedgehog inhibitor can be administered simultaneously (i.e., simultaneous administration) and/or sequentially (i.e., sequential administration). The mode of administration for the various components is further discussed below in more detail. The mode of administration discussed herein thus may be applicable to all methods described herein.

In some embodiments, the nanoparticle taxane composition and the hedgehog inhibitor (including the specific agents described herein) are administered simultaneously. The term "simultaneous administration," as used herein, means that the nanoparticle taxane composition and the hedgehog inhibitor are administered with a time separation of no more than about 15 minute(s), such as no more than about any of 10, 5, or 1 minutes. When the drugs are administered simultaneously, the taxane in the nanoparticles and the hedgehog inhibitor may be contained in the same composition (e.g., a composition comprising both the nanoparticles and the hedgehog inhibitor) or in separate compositions (e.g., the nanoparticles are contained in one composition and the hedgehog inhibitor is contained in another composition).

In some embodiments, the nanoparticle taxane composition and the hedgehog inhibitor are administered sequentially. The term "sequential administration" as used herein means that the taxane in the nanoparticle taxane composition and the hedgehog inhibitor are administered with a time separation of more than about 15 minutes, such as more than about any of 20, 30, 40, 50, 60 or more minutes. Either the nanoparticle taxane composition or the hedgehog inhibitor may be administered first. The nanoparticle taxane composition and the hedgehog inhibitor are contained in separate compositions, which may be contained in the same or different packages.

In some embodiments, the administration of the nanoparticle taxane composition and the hedgehog inhibitor are concurrent, i.e., the administration period of the nanoparticle taxane composition and that of the hedgehog inhibitor overlap with each other. In some embodiments, the administration of the nanoparticle taxane composition and the hedgehog inhibitor are non-concurrent. For example, in some embodiments, the administration of the nanoparticle taxane composition is terminated before the hedgehog inhibitor is administered. In some embodiments, the administration of the hedgehog inhibitor is terminated before the nanoparticle taxane composition is administered. The time period between these two non-concurrent administrations can range from about two to eight weeks, such as about four weeks.

The dosing frequency of the drug-containing nanoparticle taxane composition and the hedgehog inhibitor may be adjusted over the course of the treatment, based on the judgment of the administering physician. When administered separately, the drug-containing nanoparticle taxane composition and the hedgehog inhibitor can be administered at different dosing frequency or intervals. For example, the drug-containing nanoparticle taxane composition can be administered weekly, while a hedgehog inhibitor can be administered more or less frequently. In some embodiments, sustained continuous release formulation of the taxane-containing nanoparticle and/or hedgehog inhibitor may be used. Various formulations and devices for achieving sustained release are known in the art.

The nanoparticle taxane composition and the hedgehog inhibitor can be administered using the same route of administration or different routes of administration. In some embodiments (for both simultaneous and sequential administrations), the taxane in the nanoparticle taxane composition and the hedgehog inhibitor are administered at a predetermined ratio. For example, in some embodiments, the ratio by weight of the taxane in the nanoparticle taxane composition and the hedgehog inhibitor is about 1 to 1. In some embodiments, the weight ratio may be between about 0.001 to about 1 and about 1000 to about 1, or between about 0.01 to about 1 and 100 to about 1. In some embodiments, the ratio by weight of the taxane in the nanoparticle taxane composition and the hedgehog inhibitor is less than about any of 100:1, 50:1, 30:1, 10:1, 9:1, 8:1, 7:1, 6:1, 5:1, 4:1, 3:1, 2:1, and 1:1 In some embodiments, the ratio by weight of the taxane in the nanoparticle taxane composition and the hedgehog inhibitor is more than about any of 1:1, 2:1, 3:1, 4:1, 5:1, 6:1, 7:1, 8:1, 9:1, 30:1, 50:1, 100:1. Other ratios are contemplated.

The doses required for the taxane and/or the hedgehog inhibitor may (but not necessarily) be lower than what is normally required when each agent is administered alone. Thus, in some embodiments, a subtherapeutic amount of the taxane in the nanoparticle taxane composition and/or the hedgehog inhibitor are administered. "Subtherapeutic amount" or "subtherapeutic level" refer to an amount that is less than therapeutic amount, that is, less than the amount normally used when the taxane in the nanoparticle taxane composition and/or the hedgehog inhibitor are administered alone. The reduction may be reflected in terms of the amount administered at a given administration and/or the amount administered over a given period of time (reduced frequency).

In some embodiments, enough hedgehog inhibitor is administered so as to allow reduction of the normal dose of the taxane in the nanoparticle taxane composition required to effect the same degree of treatment by at least about any of 5%, 10%, 20%, 30%, 50%, 60%, 70%, 80%, 90%, or more. In some embodiments, enough taxane in the nanoparticle taxane composition is administered so as to allow reduction of the normal dose of the hedgehog inhibitor required to effect the same degree of treatment by at least about any of 5%, 10%, 20%, 30%, 50%, 60%, 70%, 80%, 90%, or more.

In some embodiments, the dose of both the taxane in the nanoparticle taxane composition and the hedgehog inhibitor are reduced as compared to the corresponding normal dose of each when administered alone. In some embodiments, both the taxane in the nanoparticle taxane composition and the hedgehog inhibitor are administered at a subtherapeutic, i.e., reduced, level. In some embodiments, the dose of the nanoparticle taxane composition and/or the hedgehog inhibitor is substantially less than the established maximum toxic dose (MTD) of the corresponding agent when administered alone. For example, the dose of the nanoparticle taxane composition and/or the hedgehog inhibitor is less than about 50%, 40%, 30%, 20%, or 10% of the MTD of the corresponding agent when administered alone.

In some embodiments, the dose of taxane and/or the dose of the hedgehog inhibitor is higher than what is normally required when each agent is administered alone. For example, in some embodiments, the dose of the nanoparticle taxane composition and/or the hedgehog inhibitor is substantially higher than the established maximum toxic dose (MTD) of the corresponding agent when administered alone. For example, the dose of the nanoparticle taxane composition and/or the hedgehog inhibitor is more than about 50%, 40%, 30%, 20%, or 10% of the MTD of the corresponding agent when administered alone.

In some embodiments, the amount of a taxane (e.g., paclitaxel) in the composition is included in any of the following ranges: about 0.5 to about 5 mg, about 5 to about 10 mg, about 10 to about 15 mg, about 15 to about 20 mg, about 20 to about 25 mg, about 20 to about 50 mg, about 25 to about 50 mg, about 50 to about 75 mg, about 50 to about 100 mg, about 75 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg. In some embodiments, the amount of a taxane (e.g., paclitaxel) or derivative thereof in the effective amount of the composition (e.g., a unit dosage form) is in the range of about 5 mg to about 500 mg, such as about 30 mg to about 300 mg or about 50 mg to about 200 mg. In some embodiments, the concentration of the taxane (e.g., paclitaxel) in the composition is dilute (about 0.1 mg/ml) or concentrated (about 100 mg/ml), including for example any of about 0.1 to about 50 mg/ml, about 0.1 to about 20 mg/ml, about 1 to about 10 mg/ml, about 2 mg/ml to about 8 mg/ml, about 4 to about 6 mg/ml, about 5 mg/ml. In some embodiments, the concentration of the taxane (e.g., paclitaxel) is at least about any of 0.5 mg/ml, 1.3 mg/ml, 1.5 mg/ml, 2 mg/ml, 3 mg/ml, 4 mg/ml, 5 mg/ml, 6 mg/ml, 7 mg/ml, 8 mg/ml, 9 mg/ml, 10 mg/ml, 15 mg/ml, 20 mg/ml, 25 mg/ml, 30 mg/ml, 40 mg/ml, or 50 mg/ml.

Exemplary effective amounts of a taxane (e.g., paclitaxel) in the nanoparticle taxane composition include, but are not limited to, about any of 25 mg/m², 30 mg/m², 50 mg/m², 60 mg/m², 75 mg/m², 80 mg/m², 90 mg/m², 100 mg/m², 120 mg/m², 125 mg/m², 150 mg/m², 160 mg/m², 175 mg/m², 180 mg/m², 200 mg/m², 210 mg/m², 220 mg/m², 250 mg/m², 260 mg/m², 300 mg/m², 350 mg/m², 400 mg/m², 500 mg/m², 540 mg/m², 750 mg/m², 1000 mg/m², or 1080 mg/m² of a taxane (e.g., paclitaxel). In various embodiments, the composition includes less than about any of 350 mg/m², 300 mg/m², 250 mg/m², 200 mg/m², 150 mg/m², 120 mg/m², 100 mg/m², 90 mg/m², 50 mg/m², or 30 mg/m² of a taxane (e.g., paclitaxel). In some embodiments, the amount of the taxane (e.g., paclitaxel) per administration is less than about any of 25 mg/m², 22 mg/m², 20 mg/m², 18 mg/m², 15 mg/m², 14 mg/m², 13 mg/m², 12 mg/m², 11 mg/m², 10 mg/m², 9 mg/m², 8 mg/m², 7 mg/m², 6 mg/m², 5 mg/m², 4 mg/m², 3 mg/m², 2 mg/m², or 1 mg/m². In some embodiments, the effective amount of a taxane (e.g., paclitaxel) in the composition is included in any of the following ranges: about 1 to about 5 mg/m², about 5 to about 10 mg/m², about 10 to about 25 mg/m², about 25 to about 50 mg/m², about 50 to about 75 mg/m², about 75 to about 100 mg/m², about 100 to about 125 mg/m², about 125 to about 150 mg/m², about 150 to about 175 mg/m², about 175 to about 200 mg/m², about 200 to about 225 mg/m², about 225 to about 250 mg/m², about 250 to about 300 mg/m², about 300 to about 350 mg/m², or about 350 to about 400 mg/m². Preferably, the effective amount of a taxane (e.g., paclitaxel) in the composition is about 5 to about 300 mg/m², such as about 100 to about 150 mg/m², about 120 mg/m², about 130 mg/m², or about 140 mg/m².

In some embodiments of any of the above aspects, the effective amount of a taxane (e.g., paclitaxel) in the composition includes at least about any of 1 mg/kg, 2.5 mg/kg, 3.5 mg/kg, 5 mg/kg, 6.5 mg/kg, 7.5 mg/kg, 10 mg/kg, 15 mg/kg, or 20 mg/kg. In various embodiments, the effective amount of a taxane (e.g., paclitaxel) in the composition includes less than about any of 350 mg/kg, 300 mg/kg, 250 mg/kg, 200 mg/kg, 150 mg/kg, 100 mg/kg, 50 mg/kg, 25 mg/kg, 20 mg/kg, 10 mg/kg, 7.5 mg/kg, 6.5 mg/kg, 5 mg/kg, 3.5 mg/kg, 2.5 mg/kg, or 1 mg/kg of a taxane (e.g., paclitaxel) .

Exemplary dosing frequencies for the nanoparticle taxane composition (and as indicated below for the hedgehog inhibitor) include, but are not limited to, weekly without break; weekly, three out of four weeks; once every three weeks; once every two weeks; weekly, two out of three weeks. In some embodiments, the composition is administered about once every 2 weeks, once every 3 weeks, once every 4 weeks, once every 6 weeks, or once every 8 weeks. In some embodiments, the composition is administered at least about any of 1x, 2x, 3x, 4x, 5x, 6x, or 7x (i.e., daily) a week. In some embodiments, the intervals between each administration are less than about any of 6 months, 3 months, 1 month, 20 days, 15, days, 12 days, 10 days, 9 days, 8 days, 7 days, 6 days, 5 days, 4 days, 3 days, 2 days, or 1 day. In some embodiments, the intervals between each administration are more than about any of 1 month, 2 months, 3 months, 4 months, 5 months, 6 months, 8 months, or 12 months. In some embodiments, there is no break in the dosing schedule. In some embodiments, the interval between each administration is no more than about a week.

The administration of the nanoparticle taxane composition (and for the hedgehog inhibitor) can be extended over an extended period of time, such as from about a month up to about seven years. In some embodiments, the composition is administered over a period of at least about any of 2, 3, 4, 5, 6, 7, 8, 9, 10, 11, 12, 18, 24, 30, 36, 48, 60, 72, or 84 months. In some embodiments, the taxane (e.g., paclitaxel) is administered over a period of at least one month, wherein the interval between each administration is no more than about a week, and wherein the dose of the taxane (e.g., paclitaxel) at each administration is about 0.25 mg/m² to about 75 mg/m², such as about 0.25 mg/m² to about 25 mg/m² or about 25 mg/m² to about 50 mg/m².

In some embodiments, the dosage of a taxane (e.g., paclitaxel) in a nanoparticle taxane composition can be in the range of 5-400 mg/m² when given on a 3 week schedule, or 5-250 mg/m² when given on a weekly schedule. For example, the amount of a taxane (e.g., paclitaxel) is about 60 to about 300 mg/m² (e.g., about 260 mg/m²).

Other exemplary dosing schedules for the administration of the nanoparticle taxane composition (e.g., paclitaxel/albumin nanoparticle taxane composition) include, but are not limited to, 100 mg/m², weekly, without break; 75 mg/m² weekly, 3 out of four weeks; 100 mg/m², weekly, 3 out of 4 weeks; 125 mg/m², weekly, 3 out of 4 weeks; 125 mg/m², weekly, 2 out of 3 weeks; 130 mg/m², weekly, without break; 175 mg/m², once every 2 weeks; 260 mg/m², once every 2 weeks; 260 mg/m², once every 3 weeks; 180-300 mg/m², every three weeks; 60-175 mg/m², weekly, without break; 20-150 mg/m² twice a week; and 150-250 mg/m² twice a week. The dosing frequency of the composition may be adjusted over the course of the treatment based on the judgment of the administering physician.

In some embodiments, the individual is treated for at least about any of one, two, three, four, five, six, seven, eight, nine, or ten treatment cycles. The compositions described herein allow infusion of the composition to an individual over an infusion time that is shorter than about 24 hours. For example, in some embodiments, the composition is administered over an infusion period of less than about any of 24 hours, 12 hours, 8 hours, 5 hours, 3 hours, 2 hours, 1 hour, 30 minutes, 20 minutes, or 10 minutes. In some embodiments, the composition is administered over an infusion period of about 30 minutes.

Other exemplary dose of the taxane (in some embodiments paclitaxel) in the nanoparticle taxane composition include, but is not limited to, about any of 50 mg/m², 60 mg/m², 75 mg/m², 80 mg/m², 90 mg/m², 100 mg/m², 120 mg/m², 160 mg/m², 175 mg/m², 200 mg/m², 210 mg/m², 220 mg/m², 260 mg/m², and 300 mg/m². For example, the dosage of paclitaxel in a nanoparticle taxane composition can be in the range of 100-400 mg/m² when given on a 3 week schedule, or 50-250 mg/m² when given on a weekly schedule.

Other exemplary dosing schedules for the administration of the nanoparticle taxane composition (such as paclitaxel/albumin nanoparticle taxane composition, for example Abraxane®) include, but are not limited to, 100 mg/m², weekly, without break; 75 mg/m² weekly, 3 out of four weeks; 100 mg/m², weekly, 3 out of 4 weeks; 125 mg/m², weekly, 3 out of 4 weeks; 125 mg/m², weekly, 2 out of 3 weeks; 130 mg/m², weekly, without break; 175 mg/m², once every 2 weeks; 260 mg/m², once every 2 weeks; 260 mg/m², once every 3 weeks; 180-300 mg/m², every three weeks; 60-175 mg/m², weekly, without break. In addition, the taxane (alone or in combination therapy) can be administered by following a metronomic dosing regime described herein. In some embodiments, the nanoparticle taxane composition (for example Abraxane®) is administered weekly at a dose of about 10-200 mg/kg (including, for example, any of about 10-40 mg/kg, 40-60 mg/kg, 60-80 mg/kg, 80-100 mg/kg, 100-120 mg/kg, 120-140 mg/kg, 140-160 mg/kg, 160-180 mg/kg, and 180-200 mg/kg) per administration. In some embodiments, the nanoparticle taxane composition (for example Abraxane®) is administered once every four days at a dose of about 10-200 mg/kg (including, for example, any of about 10-40 mg/kg, 40-60 mg/kg, 60-80 mg/kg, 80-100 mg/kg, 100-120 mg/kg, 120-140 mg/kg, 140-160 mg/kg, 160-180 mg/kg, and 180-200 mg/kg) per administration. In some embodiments, the nanoparticle taxane composition (for example Abraxane®) is administered daily (for example for any of 2, 3, 4, 5, 6, 7, 8, 9, or 10 days) at a dose of about 10-30 mg/kg per administration. In some embodiments, the nanoparticle taxane composition (for example Abraxane®) is administered weekly at a dose of about 50-150 mg/m² (including, for example, any of about 50-100 mg/m² and 100-150 mg/m²) per administration. In some embodiments, the nanoparticle taxane composition (for example Abraxane®) is administered once every three weeks at a dose of about 50-300 mg/m² (including, for example, any of about 50-100 mg/m², 100-150 mg/m², 150-200 mg/m², 200-250 mg/m², and 250-300 mg/m²) per administration.

The dosing frequency of the hedgehog inhibitor can be the same or different from that of the nanoparticle taxane composition. For example, the hedgehog inhibitor can be administered daily, 6 times a week, 5 times a week, 4 times a week, 3 times a week, two times a week, or weekly. Exemplary amounts of the hedgehog inhibitor include, but are not limited to, any of the following ranges: about 0.5 to about 5 mg, about 5 to about 10 mg, about 10 to about 15 mg, about 15 to about 20 mg, about 20 to about 25 mg, about 20 to about 50 mg, about 25 to about 50 mg, about 50 to about 75 mg, about 50 to about 100 mg, about 75 to about 100 mg, about 100 to about 125 mg, about 125 to about 150 mg, about 150 to about 175 mg, about 175 to about 200 mg, about 200 to about 225 mg, about 225 to about 250 mg, about 250 to about 300 mg, about 300 to about 350 mg, about 350 to about 400 mg, about 400 to about 450 mg, or about 450 to about 500 mg. For example, the hedgehog inhibitor can be administered at a dose of about 1 mg/kg to about 200 mg/kg (including for example about 1 mg/kg to about 20 mg/kg, about 20 mg/kg to about 40 mg/kg, about 40 mg/kg to about 60 mg/kg, about 60 mg/kg to about 80 mg/kg, about 80 mg/kg to about 100 mg/kg, about 100 mg/kg to about 120 mg/kg, about 120 mg/kg to about 140 mg/kg, about 140 mg/kg to about 200 mg/kg). For example, in some embodiments, a cyclopamine is administered (for example by oral administration) at about any of 1-20 mg/kg, 20-40 mg/kg, 40-60 mg/kg, 60-80 mg/kg, 80-100 mg/kg, 100-120 mg, daily.

The nanoparticle taxane composition (and the hedgehog inhibitor) described herein can be administered to an individual (such as human) via various routes, including, for example, intravenous, intra-arterial, intraperitoneal, intrapulmonary, oral, inhalation, intravesicular, intramuscular, intra-tracheal, subcutaneous, intraocular, intrathecal, transmucosal, and transdermal. In some embodiments, sustained continuous release formulation of the composition may be used. In one variation of the invention, nanoparticles (such as albumin nanoparticles) of the inventive compounds can be administered by any acceptable route including, but not limited to, orally, intramuscularly, transdermally, intravenously, through an inhaler or other air borne delivery systems and the like.

A combination of the administration configurations described herein can be used. The combination therapy methods described herein may be performed alone or in conjunction with another therapy, such as surgery, radiation, chemotherapy, immunotherapy, gene therapy, and the like. Additionally, a person having a greater risk of developing the proliferative disease may receive treatments to inhibit or and/or delay the development of the disease.

As will be understood by those of ordinary skill in the art, the appropriate doses of hedgehog inhibitors will be approximately those already employed in clinical therapies wherein the hedgehog inhibitor are administered alone or in combination with hedgehog inhibitors. Variation in dosage will likely occur depending on the condition being treated. As described above, in some embodiments, the hedgehog inhibitors may be administered at a reduced level.

### Other components in the nanoparticle compositions

The nanoparticles described herein can be present in a composition that include other agents, excipients, or stabilizers. For example, to increase stability by increasing the negative zeta potential of nanoparticles, certain negatively charged components may be added. Such negatively charged components include, but are not limited to bile salts of bile acids consisting of glycocholic acid, cholic acid, chenodeoxycholic acid, taurocholic acid, glycochenodeoxycholic acid, taurochenodeoxycholic acid, litocholic acid, ursodeoxycholic acid, dehydrocholic acid and others; phospholipids including lecithin (egg yolk) based phospholipids which include the following phosphatidylcholines:
palmitoyloleoylphosphatidylcholine, palmitoyllinoleoylphosphatidylcholine,
stearoyllinoleoylphosphatidylcholine stearoyloleoylphosphatidylcholine, stearoylarachidoylphosphatidylcholine, and dipalmitoylphosphatidylcholine. Other phospholipids including L-a-dimyristoylphosphatidylcholine (DMPC), dioleoylphosphatidylcholine (DOPC), distearyolphosphatidylcholine (DSPC), hydrogenated soy phosphatidylcholine (HSPC), and other related compounds. Negatively charged surfactants or emulsifiers are also suitable as additives, e.g., sodium cholesteryl sulfate and the like.

In some embodiments, the composition is suitable for administration to a human. In some embodiments, the composition is suitable for administration to a mammal such as, in the veterinary context, domestic pets and agricultural animals. There are a wide variety of suitable formulations of the nanoparticle composition (see, e.g., U.S. Pat. Nos. 5,916,596 and 6,096,331). The following formulations and methods are merely exemplary and are in no way limiting. Formulations suitable for oral administration can consist of (a) liquid solutions, such as an effective amount of the compound dissolved in diluents, such as water, saline, or orange juice, (b) capsules, sachets or tablets, each containing a predetermined amount of the active ingredient, as solids or granules, (c) suspensions in an appropriate liquid, and (d) suitable emulsions. Tablet forms can include one or more of lactose, mannitol, corn starch, potato starch, microcrystalline cellulose, acacia, gelatin, colloidal silicon dioxide, croscarmellose sodium, talc, magnesium stearate, stearic acid, and other excipients, colorants, diluents, buffering agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

Examples of suitable carriers, excipients, and diluents include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline solution, syrup, methylcellulose, methyl- and propylhydroxybenzoates, talc, magnesium stearate, and mineral oil. The formulations can additionally include lubricating agents, wetting agents, emulsifying and suspending agents, preserving agents, sweetening agents or flavoring agents.

Formulations suitable for parenteral administration include aqueous and non-aqueous, isotonic sterile injection solutions, which can contain anti-oxidants, buffers, bacteriostats, and solutes that render the formulation compatible with the blood of the intended recipient, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described. Injectable formulations are preferred.

In some embodiments, the composition is formulated to have a pH range of about 4.5 to about 9.0, including for example pH ranges of any of about 5.0 to about 8.0, about 6.5 to about 7.5, and about 6.5 to about 7.0. In some embodiments, the pH of the composition is formulated to no less than about 6, including for example no less than about any of 6.5, 7, or 8 (such as about 8). The composition can also be made to be isotonic with blood by the addition of a suitable tonicity modifier, such as glycerol.

### Compositions and Kits

Provided herein are compositions for use in treating a proliferative disease (such as cancer, for example pancreatic cancer or colon cancer) comprising nanoparticles comprising a taxane and a carrier protein (such as an albumin), wherein the composition is used in conjunction with a Hedgehog inhibitor. In some embodiments, there is provided a composition for use in treating a proliferative disease (such as cancer, for example pancreatic cancer or colon cancer) comprising nanoparticles comprising paclitaxel and an albumin (such as Abraxane ®), wherein the composition is used in conjunction with a Hedghog inhibitor. In some embodiments, there is provided a composition for use in treating a proliferative disease (such as cancer, for example pancreatic cancer or colon cancer) comprising a hedgehog inhibitor, wherein the hedgehog inhibitor is used in conjunction with a composition comprising nanoparticles comprising a taxane and a carrier protein (such as an albumin), for example a composition comprising nanoparticles comprising paclitaxel and an albumin (such as Abraxane ®). Also provided herein are compositions for use in treating a proliferative disease (such as cancer, for example pancreatic cancer or colon cancer) comprising (a) nanoparticles comprising a taxane and a carrier protein (such as an albumin), and (b) a Hedghog inhibitor. In some embodiments, there is provided a composition for use in treating a proliferative disease (such as cancer, for example pancreatic cancer or colon cancer) comprising (a) nanoparticles comprising paclitaxel and an albumin (such as Abraxane ®), and (b) a Hedghog inhibitor.

In some embodiments, there is provided use of a composition comprising nanoparticles comprising a taxane and a carrier protein (such as albumin) for the manufacture of medicament for treating a proliferative disease (such as cancer, for example pancreatic cancer or colon cancer), wherein the nanoparticle is used in conjunction with a hedgehog inhibitor. In some embodiments, there is provided use of a hedgehog inhibitor for the manufacture of a medicament for treating a proliferative disease (such as cancer, for example pancreatic cancer or colon cancer), wherein the hedgehog inhibitor is used in combination with a composition comprising nanoparticles comprising a taxane and a carrier protein (such as an albumin), for example a composition comprising nanoparticles comprising paclitaxel and an albumin (such as Abraxane ®). Also provided herein are uses of a composition comprising 1) nanoparticles comprising a taxane and a carrier protein (such as an albumin) and 2) a Hedghog inhibitor for the manufacture of medicaments for treating a proliferative disease (such as cancer, for example pancreatic cancer or colon cancer).

The invention also provides kits for use in the instant methods and/or kits comprising a composition described herein. Kits of the invention include one or more containers comprising taxane-containing nanoparticle compositions (or unit dosage forms and/or articles of manufacture) and/or an agent that inhibits the hedgehog signaling pathway and in some embodiments, further comprise instructions for use in accordance with any of the methods described herein. The kit may further comprise a description of selection an individual suitable or treatment. Instructions supplied in the kits of the invention are typically written instructions on a label or package insert (e.g., a paper sheet included in the kit), but machine-readable instructions (e.g., instructions carried on a magnetic or optical storage disk) are also acceptable.

In some embodiments, the kit comprises a) a composition comprising nanoparticles comprising a taxane and a carrier protein (such as albumin), b) an effective amount of an agent that inhibits the hedgehog signaling pathway and c) instructions for administering the nanoparticles and the hedgehog inhibitors simultaneously and/or sequentially, for treatment of a proliferative disease (such as cancer). In some embodiments, the taxane is any of paclitaxel, docetaxel, and ortataxel. In some embodiments, the kit comprises nanoparticles comprising a) a composition comprising nanoparticles comprising paclitaxel and an albumin (such as Abraxane®), b) an effective amount of an agent that inhibits the hedgehog signaling pathway, and c) instructions for administering the nanoparticles and the hedgehog inhibitors simultaneously and/or sequentially, for the effective treatment of a proliferative disease (such as cancer).

In some embodiments, the kit further comprises an effective amount of an additional therapeutic agent, such as gemcitabine. For example, in some embodiments, the kit comprises a) a composition comprising nanoparticles comprising a taxane and a carrier protein (such as albumin), b) an effective amount of an agent that inhibits the hedgehog signaling pathway, c) an effective amount of gemcitabine, and d) instructions for administering the nanoparticles, the hedgehog inhibitors, and the gemcitabine for treatment of a proliferative disease (such as cancer for example pancreatic cancer). In some embodiments, the taxane is any of paclitaxel, docetaxel, and ortataxel. In some embodiments, the kit comprises nanoparticles comprising a) a composition comprising nanoparticles comprising paclitaxel and an albumin (such as Abraxane®), b) an effective amount of an agent that inhibits the hedgehog signaling pathway, c) an effective amount of gemcitabine, and d) instructions for administering the nanoparticles, the hedgehog inhibitors, and the gemcitabine for the effective treatment of a proliferative disease (such as cancer for example pancreatic cancer).

The nanoparticles and the hedgehog inhibitors can be present in separate containers or in a single container. It is understood that the kit may comprise one distinct composition or two or more compositions wherein one composition comprises nanoparticles and one composition comprises a hedgehog inhibitor.

The kits of the invention are in suitable packaging. Suitable packaging include, but is not limited to, vials, bottles, jars, flexible packaging (e.g., sealed Mylar or plastic bags), and the like. Kits may optionally provide additional components such as buffers and interpretative information.

The instructions relating to the use of the nanoparticle taxane compositions generally include information as to dosage, dosing schedule, and route of administration for the intended treatment. The containers may be unit doses, bulk packages (e.g., multi-dose packages) or sub-unit doses. For example, kits may be provided that contain sufficient dosages of the taxane (such as taxane) as disclosed herein to provide effective treatment of an individual for an extended period, such as any of a week, 2 weeks, 3 weeks, 4 weeks, 6 weeks, 8 weeks, 3 months, 4 months, 5 months, 7 months, 8 months, 9 months, or more. Kits may also include multiple unit doses of the taxane and pharmaceutical compositions and instructions for use and packaged in quantities sufficient for storage and use in pharmacies, for example, hospital pharmacies and compounding pharmacies.

Those skilled in the art will recognize that several embodiments are possible within the scope and spirit of this invention. The invention will now be described in greater detail by reference to the following non-limiting examples. The following examples further illustrate the invention but, of course, should not be construed as in any way limiting its scope.

### EXAMPLES

### Example 1. Treatment of pancreatic cancer with Abraxane® and a hedgehog pathway inhibitor

This example describes treatment of pancreatic cancer using Abraxane in combination with a hedgehog pathway inhibitor (compound X) and optionally with gemcitabine is tested in KPC mice (for KPC mice, see S. R. Hingorani et al., Cancer Cell 7, 469 (2005)). KPC mice conditionally express endogenous mutant Kras and p53 alleles in pancreatic cells and develop pancreatic tumors whose pathophysiological and molecular features resemble those of human pancreatic ductal adenocarcinoma (PDA).

The hedgehog pathway inhibitor (compound X) is dissolved in a 5% aqueous solution of hydroxypropyl-β-cyclodextrin (HPBCD) to a concentration of 5 mg/mL, with sonication and vortexing, and then sterile filtered. The solution is stored at 4°C for up to one week. Compound X is administered daily by oral gavage at the indicated dose. Gemcitabine powder is resuspended in sterile normal saline at 5 mg/mL. Gemcitabine is administered by intraperitoneal injection twice weekly at the indicated dose. Abraxane® is prepared as a 5 mg/mL suspension and administered intravenously at the indicated dose.

Mice are divided into five treatment groups, as follows: 1) vehicle; 2) Abr-Abraxane (10-200 mg/kg weekly, 10-180 mg/kg once every four days, 10-30 mg/kg daily, or 30 mg/kg once every three weeks); 3) X/gem - 40 mg/kg compound X + 100 mg/kg gemcitabine; 4) X/Abr - 40 mg/kg compound X + Abraxane (10-200 mg/kg weekly, 10-180 mg/kg once every four days, 10-30 mg/kg daily, or 30 mg/kg once every three weeks); 5) X/Abr/gem - 40 mg/kg compound X + 100 mg/kg gemcitabine + Abraxane (10-200 mg/kg weekly, 10-180 mg/kg once every four days, 10-30 mg/kg daily, or 30 mg/kg once every three weeks).

The effects on tumor histopathology and perfusion are investigated after 8 to 12 days of treatment by high resolution ultrasound, contrast ultrasound, MRI, and ¹⁹fluorine nuclear magnetic resonance. Additionally, tissue samples are harvested at various time points, snap frozen in liquid nitrogen, and stored at -80°C. Tissue samples are analyzed by LC/MS, immunofluorescence, phospho-histone H3 (PH3) proliferation analysis, and cleaved caspase 3 (CC3) apoptosis analysis. Mean vascular density and vascular-tumor distance of tissue samples are measured. A survival study is also carried out, during which tumor growth and tumor volume are measured by ultrasound twice weekly until endpoint criteria are met. Endpoint criteria include development of abdominal ascites, severe cachexia significant weight loss exceeding 20% of initial weight, or extreme weakness or inactivity.

### Example 2. Treatment of pancreatic cancer with Abraxane® and gemcitabine

Surgically resected human gemcitabine-resistant pancreatic tumors are xenografted onto nude mice, thus generating an in vivo platform for assessing histology and drug levels that closely resembles the biology of human pancreatic cancer.

Gemcitabine powder is resuspended in sterile normal saline at 5 mg/mL. Gemcitabine is administered by intraperitoneal injection twice weekly at the indicated dose. Abraxane® is prepared as a 5 mg/mL suspension and administered at the indicated dose.

Mice are divided into four treatment groups, as follows: 1) vehicle - 20 µL/g 0.85% NaCl + 8 µL/g 5% HPBCD; 2) gem - 100 mg/kg gemcitabine + 8 µL/g 5% HPBCD; 3) Abr - Abraxane (10-200 mg/kg weekly, 10-180 mg/kg once every four days, 10-30 mg/kg daily, or 30 mg/kg once every three weeks) + 20 µL/g 0.85% NaCl; 4) Abr/gem - 100 mg/kg gemcitabine + Abraxane (10-200 mg/kg weekly, 10-180 mg/kg once every four days, 10-30 mg/kg daily, or 30 mg/kg once every three weeks).

The effects on tumor histopathology and perfusion are investigated after 8 to 12 days of treatment by high resolution ultrasound, contrast ultrasound, MRI, and ¹⁹fluorine nuclear magnetic resonance. In addition, tissue samples are collected and analyzed by LC/MS to determine the levels of Abraxane® and/or gemcitabine in the tumors.

Abraxane® penetrates the tumor, causing stromal collapse and an increase in CD31+ vasculature, thereby increasing micro vessel density in the tumor. Furthermore, Abraxane® and gemcitabine exhibit synergy, wherein Abraxane® treatment increases gemcitabine concentration in the tumor, thereby allowing for more effective treatment by gemcitabine in otherwise gemcitabine-resistant tumors.

### Example 3. Treatment of pancreatic xenograft models with the combination of IPI-926 and Abraxane®.

IPI-926 is a potent and selective Smoothened ("Smo") inhibitor. The effects of the combination of IPI-926 with Abraxane® (also described as "nab-paclitaxel" herein) were studied in pancreatic cancer xenograft models.

The abilities of IPI-926 to regulate Gli1 expressions were examined in pancreatic cancer xenograft models of L3.6p1 and ASPC-1. L3.6pl and ASPC-1 human pancreatic cell lines were implanted subcutaneously into mice. IPI-926 was administered orally at 40 mg/kg and tumors were collected 24 hours later. Q-RT-PCR analysis showed the inhibition of murine Gli1 mRNA expression with the IPI-926 treatment (p<0.005, student T test). *See* Figure 1. Human Hh ligand expression was detected and human Gli1 mRNA levels were not modulated with the treatment (data not shown). The data shows that Hedgehog (Hh) signaling can occur in a paracrine manner in pancreatic xenograft models, where the human tumor cells provide Hedgehog ligand and activate murine Gli1 in the stromal cells. IPI-926 treatment inhibited murine Gli1 expression in the stromal cells.

The effects of IPI-926 on tumor perfusion were examined in the L3.6pl pancreatic cancer xenograft model. The L3.6pl tumor cell line was injected subcutaneously and treatment with IPI-926 was initiated. IPI-926 or vehicle was administered orally at 40 mg/kg for seven consecutive days. Mice were subjected to ultrasound image analysis using perfusion contrast enhancement (microbubbles) during the imaging procedure. The IPI-926 treated animals imaged via ultrasound showed more contrast agent in the tumors than the vehicle-treated animals did. The time to reach peak contrast was measured and there was a decrease in the IPI-926 treated animals compared to vehicle-treated animals. The table below shows the time to peak results with treatment of vehicle or IPI-926.

| Mouse # | Treatment | *Time to Peak(s) |
|---|---|---|
| C1M1 | Control | 16 |
| C1M2 | Control | 13 |
| C1M3 | Control | 8 |
| C1M4 | Control | 7 |
| C2M1 | IPI-926 | 3 |
| C2M2 | IPI-926 | 4 |
| C2M3 | IPI-926 | 6 |
| C2M4 | IPI-926 | 6 |

On average, the peak time for contrast agent levels decreased from 11.0 seconds to 4.75 seconds in the vehicle versus IPI-926 treated animals, respectively, (p=0.0321). The data thus show that inhibition of the Hedgehog pathway in tumor stroma with IPI-926 resulted in increased tumor perfusion in the subcutaneous L3.6pl pancreatic cancer xenograft model.

The effects of IPI-926 in combination with Abraxane® on tumor growth in the L3.6pl pancreatic xenograft model were examined. The L3.6pl human pancreatic cell line was implanted subcutaneously into mice and treatment was initiated on Day 10 after implant. IPI-926 was administered orally at 40mg/kg every other day ("QOD") and Abraxane® was administered *i*.*v*. at 20mg/kg once a week (QW1). On day 26, compared to the vehicle control, the Abraxane® alone group showed 61 % tumor growth inhibition, while the combination of IPI-926 and Abraxane® resulted in an 83% tumor growth inhibition (p=0.0048). *See* Figure 2A. Mice remained on treatment and time to reach 1000 mm³ was recorded. Once tumors reached 1000 mm³, mice were taken off study. Figure 2B shows the percentage of mice remaining on study as a function of days post implant. As shown in Figure 2B, the combination treatment group showed an increase in median % of mice on study (day 54), versus Abraxane® alone (day 35) (p<0.001), while IPI-926 had no effect as a single agent. The data thus demonstrate that the treatment with IPI-926 in combination with Abraxane® resulted in increased tumor growth inhibition, and the effects of the two drugs are synergistic.

The effects of IPI-926 on the level of paclitaxel in L3.6pl tumors and the effects on cell cycle were also studied. On day 27, 24 hours after the last dose of IPI-926 and Abraxane®, tumors were collected for pharmacokinetic analysis and phosphorylated histone H3 ("PH3") immunostaining. As shown in Figure 3A, the combination treatment of IPI-926 and Abraxane® resulted in 28% higher paclitaxel levels in the tumors compared to the tumors treated with Abraxane® alone (p<0.001). PH3 quantitative whole section analysis revealed a 33% increase of tumor cells accumulating in the late G₂/M phase in the combination treatment group versus the Abraxane® alone group (p=0.0014). *See* Figures 3B and 3C. Therefore, the combination of IPI-926 and Abraxane® increased paclitaxel tumor levels and increased late G₂/M tumor cell accumulation. These data, along with the data regarding the increase of tumor perfusion by IPI-926 show that IPI-926 enhanced the tumor drug delivery in a subcutaneous pancreatic tumor model.

The effects of the combination of IPI-926 and Abraxane® were also studied in ASPC-1 human pancreatic tumor model. The ASPC-1 human pancreatic cell line was implanted subcutaneously and treatment was initiated on day 20 after implant. IPI-926 was administered orally at 40 mg/kg QOD and Abraxane® was dosed *i.v.* at 20 mg/kg QW1. The final *i.v.* dose of Abraxane® was administered on day 34 and the final dose of IPI-926 was administered on day 41. Re-growth was monitored after the treatment stopped. Figure 4 shows that the combination of IPI-926 and Abraxane® resulted in increased tumor growth inhibition and delayed tumor re-growth in ASPC-1 tumor bearing mice. On day 41, compared to the vehicle control, both IPI-926 and Abraxane® showed single agent activity resulting in 38% and 34% tumor growth inhibition, respectively. The combination of IPI-926 and Abraxane® resulted in a 77% tumor growth inhibition (p=0.0048). *See* Figure 4.

### Example 4. Treatment of colon cancer with the combination of Abraxane® and various Hedgehog inhibitors.

The structures of various Hedgehog ("Hh") inhibitors, namely, GDC-0449 (also known as ABI2012), ABI1914, ABI2088, and ABI2099 are shown below. The effects of ABI2012, ABI1914, ABI2088, and ABI2099 in a Gli-Bla cell-based assay were studied and the EC50 values are shown below. EC50 is an index for response.

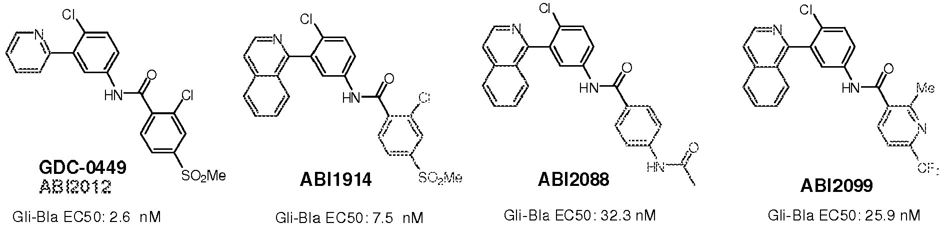

The activities of various hedgehog inhibitors (ABI2012, ABI1914, ABI2008, and ABI1915) on kinase inhibition were studied. The table below shows the % of kinase inhibition when the cells were treated with 1 µM of each compound. None of the tested compounds showed significant kinase inhibition. The hedgehog inhibitors thus do not inhibit a kinase activity. The compounds were stable in microsomes.

| | **ABI1914** | **ABI1915** | **ABI2008** | **ABI2012** |
|---|---|---|---|---|
| **Ab1**(**h**) | -18 | -21 | 6 | 13 |
| **Aurora**-**A**(**h**) | 1 | 5 | 9 | 7 |
| **cKit(h)** | -4 | 1 | 7 | -4 |
| **cSRC(h)** | 3 | 0 | 15 | -1 |
| **EGFR(h)** | -3 | 3 | 6 | -9 |
| **EphB4(h)** | -12 | -11 | -7 | -33 |
| **Flt3(h)** | -14 | -17 | 4 | 1 |
| **GSK3ß**(**h**) | 3 | 12 | -14 | -4 |
| **GF**-**1R**(**h**), **activate** | -5 | 4 | 8 | 8 |
| **MEK1**(**h**) | -9 | -3 | 1 | 2 |
| **Met(h)** | 3 | 1 | 0 | -19 |
| **mTOR(h)** | 6 | -4 | 5 | -6 |
| **PDGFRa(h)** | -3 | -3 | 4 | 0 |
| **PKBa(h)** | 5 | -8 | -5 | -19 |
| **Ret(h)** | -3 | -4 | 7 | 1 |
| **TrkA**(**h**) | -12 | -14 | -4 | -7 |

The effects of each of ABI2012, ABI1914, ABI2088, and ABI2099 on tumor growth were studied in human colon adenocarcinoma HT29 xenograft model. Mice were implanted with HT29 cells and ABI2012, ABI1914, ABI2088, and ABI209 were administered by intraperitoneal route (*"i.p."*) to the mice at two dosages: 75 mg/kg qdx12 and 100 mg/kg qdx12. As shown in Figures 5A and 5C, none of ABI2012, ABI1914, ABI2088, and ABI2099 as single agent showed significant tumor inhibition effects in HT29 xenograft model. The body weights of the mice with HT 29 xenograft treated with ABI2012, ABI1914, ABI2088, or ABI2099 were monitored, as shown in Figures 5B and 5D.

The effects of the combination of various Hedgehog pathway inhibitors (ABI2012, ABI1914, ABI2088, or ABI2099) and Abraxane® were studied in HT29 xenograft model. Each of ABI2012, ABI1914, ABI2088, and ABI2099 was administered at 75 mg/kg qdx12, *i.p.* Abraxane® was administered at 10 mg/kg q4dx3, *i*.*v*. The tumor volumes were measured. As shown in Figure 6A, each of the compounds tested showed the inhibition on tumor growth in combination with Abraxane®. Both ABI2088 and ABI2099 significantly enhanced the antitumor activity of Abraxane®. *See* Figure 6A. ABI2088 showed about 100% tumor growth inhibition ("TGI") in combination with Abraxane® in the HT29 xenograft model on day 22. The body weights of the mice with HT 29 xenograft treated with each of ABI2012, ABI1914, ABI2088, and ABI2099 in combination with Abraxane® were monitored, as shown in Figure 6B.

The drug metabolism and pharmacokinetics of ABI2012 (GDC-0449) and ABI2088 were studied, as shown in the table below.

| | **GDC**-**0449** | **ABI2088** |
|---|---|---|
| **Dosing** | IV | IV |
| **Vehicle** | 30%PEG 400 | 10%DMA :40%Propylene glycol :30%PEG400 |
| **CL (mL/min/kg)** | 4.65 ± 1.81 | 12.11 ± 1.59 |
| **AUC_{inf} (ng*h/mL)** | 3980 ± 1540 | 1392 ± 164 |
| **T_{1/2} (h)** | 1.32 ± 0.258 | 1.31 ± 0.14 |
| **Vss (L/kg)** | 0.490 ± 0.065 | 1.02 ± 0.11 |
| | | |

| **Dosing** | Oral | Oral |
|---|---|---|
| **Vehicle** | 0.5%Methlycellulose :0.2%Tween 80 | 10%DMA :40%Propylene glycol :30%PEG400 |
| **Cₘₐₓ (ng/mL)** | 2760 ± 1020 | 1005 ± 659 |
| **tₘₐₓ (h)** | 0.667 ± 0.289 | 0.5 ± 0.0 |
| **AUC_{inf} (ng*h/mL)** | 10500 ± 3150 | 4313 ± 1200 |
| **F(%)** | 53% | 62% |

### Example 5. Treatment of colon cancer with the combination of Abraxane® and various Hedgehog inhibitors.

The structures of various Hedgehog ("Hh") inhibitors, namely, ABI1C4, ABI1C5, ABI1C6, and ABI1C7 are shown below. The effects of these compounds in a Gli-Bla cell-based assay are studied.

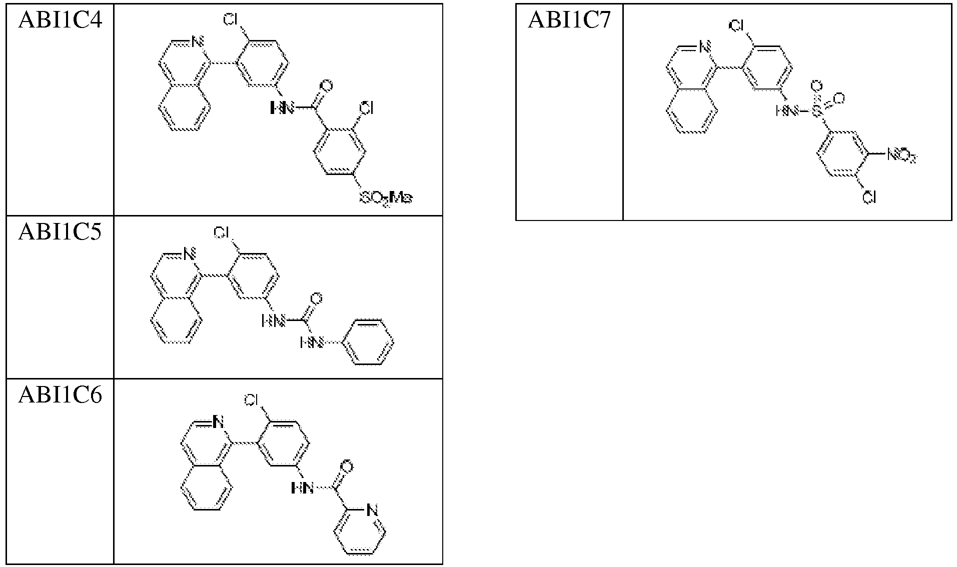

The activities of compounds ABI1C4, ABI1C5, ABI1C6, and ABI1C7 on kinase inhibition are studied. The effects of each compound on tumor growth are studied in human colon adenocarcinoma HT29 xenograft model. Mice are implanted with HT29 cells and ABI1C4, ABI1C5, ABI1C6, and ABI1C7 are administered by intraperitoneal route ("*i*.*p*.") to the mice at two dosages: 75 mg/kg qdx12 and 100 mg/kg qdx12, either as a single agent or in combination with Abraxane® administered at 10 mg/kg q4dx3, *i*.*v*. The tumor volumes are measured.

### Example 6. Treatment of colon cancer with the combination of Abraxane® and various Hedgehog inhibitors.

The structures of various Hedgehog ("Hh") inhibitors, namely, ABI2C4, ABI2C5, ABI2C6, and ABI2C7 are shown below. The effects of these compounds in a Gli-Bla cell-based assay are studied.

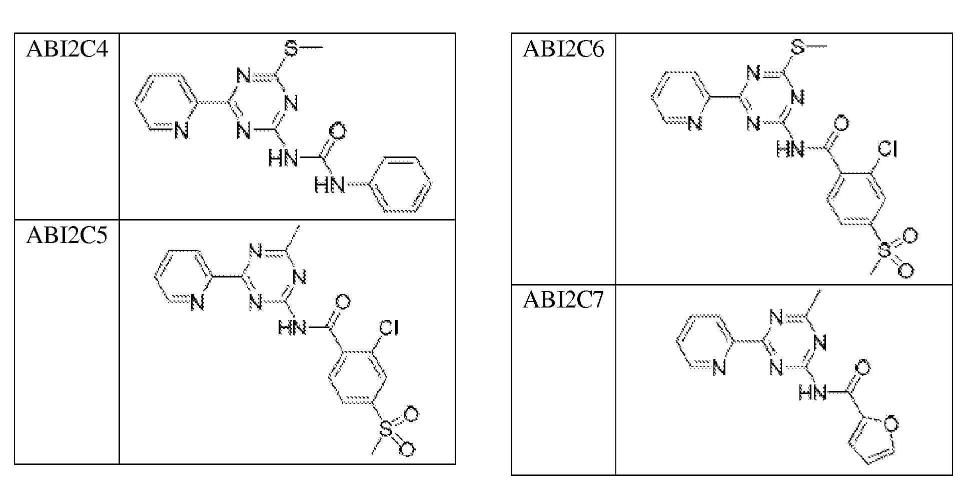

The activities of compounds ABI2C4, ABI2C5, ABI2C6, and ABI2C7 on kinase inhibition are studied. The effects of each compound on tumor growth are studied in human colon adenocarcinoma HT29 xenograft model. Mice are implanted with HT29 cells and ABI1C4, ABI2C5, ABI2C6, and ABI2C7 are administered by intraperitoneal route ("*i*.*p*.") to the mice at two dosages: 75 mg/kg qdx12 and 100 mg/kg qdx12, either as a single agent or in combination with Abraxane® administered at 10 mg/kg q4dx3, *i*.*v*. The tumor volumes are measured.

Although the foregoing invention has been described in some detail by way of illustration and example for purposes of clarity of understanding, it is apparent to those skilled in the art that certain minor changes and modifications will be practiced. Therefore, the description and examples should not be construed as limiting the scope of the invention.

All references, including publications, patent applications, and patents, cited herein are hereby incorporated by reference to the same extent as if each reference were individually and specifically indicated to be incorporated by reference and were set forth in its entirety herein.

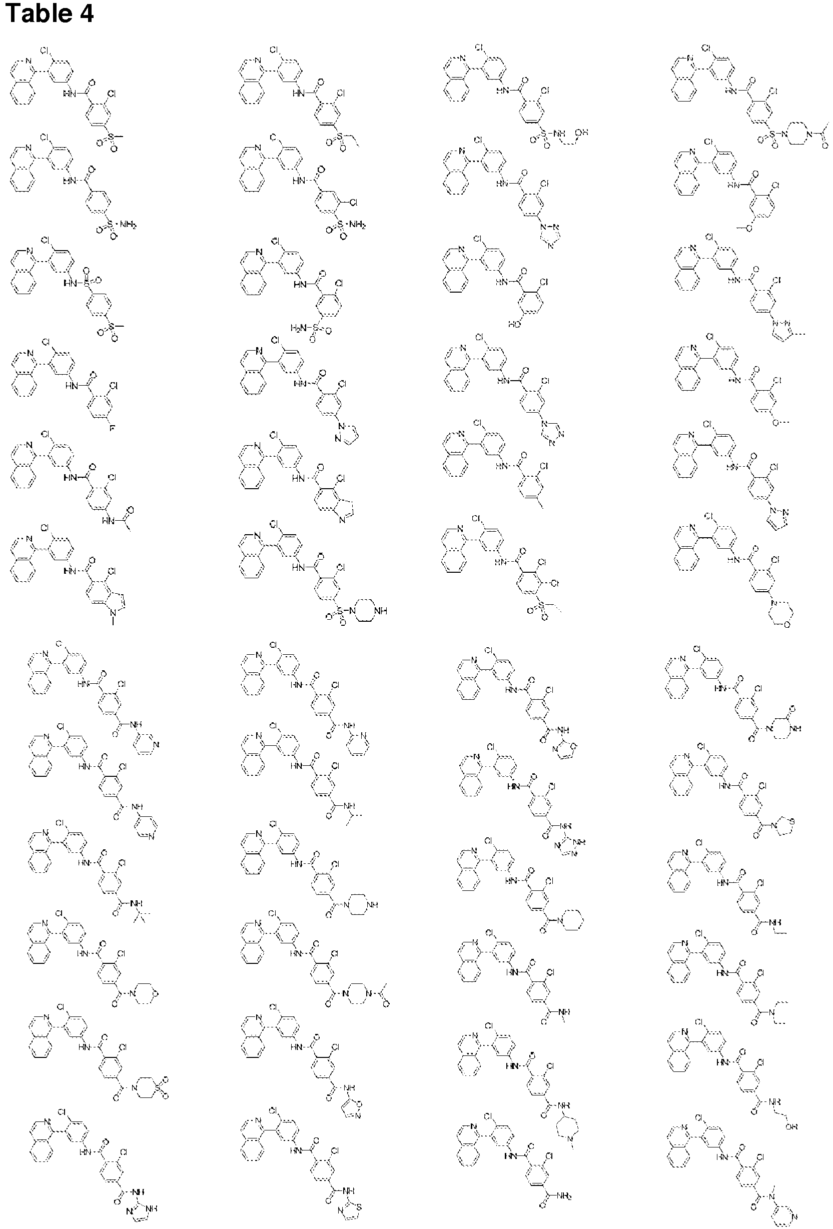

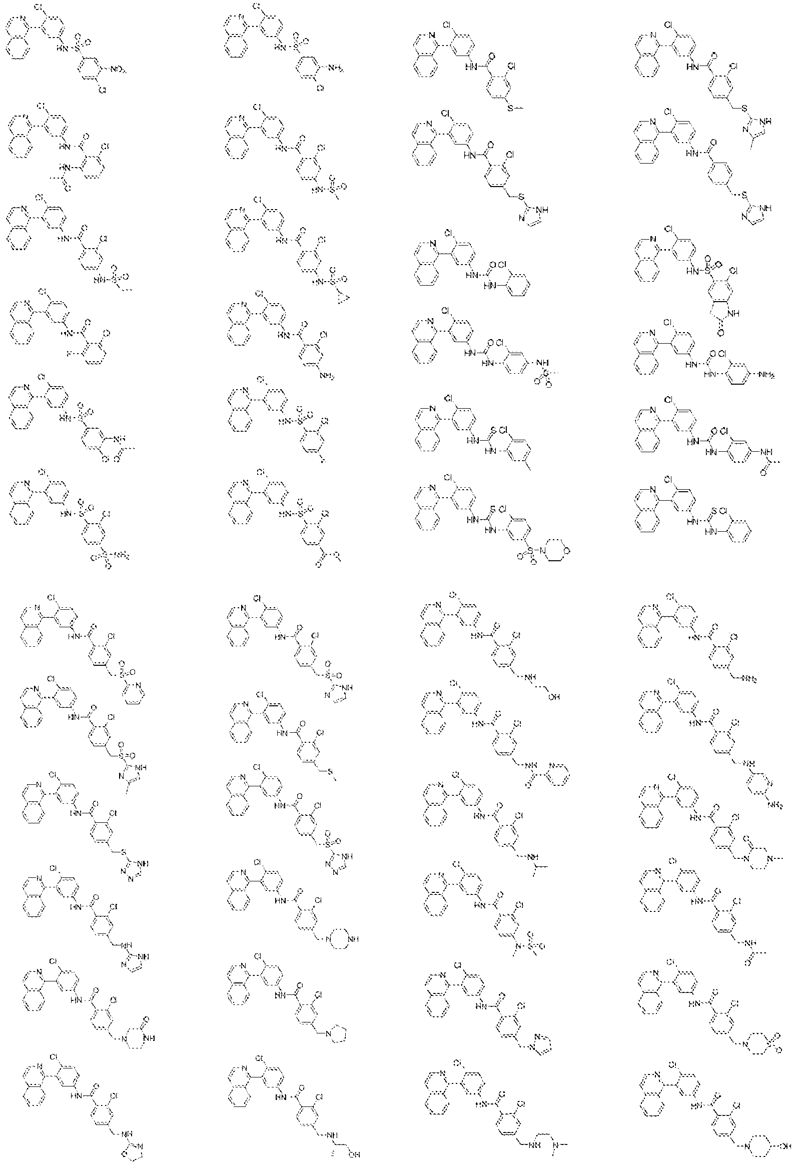

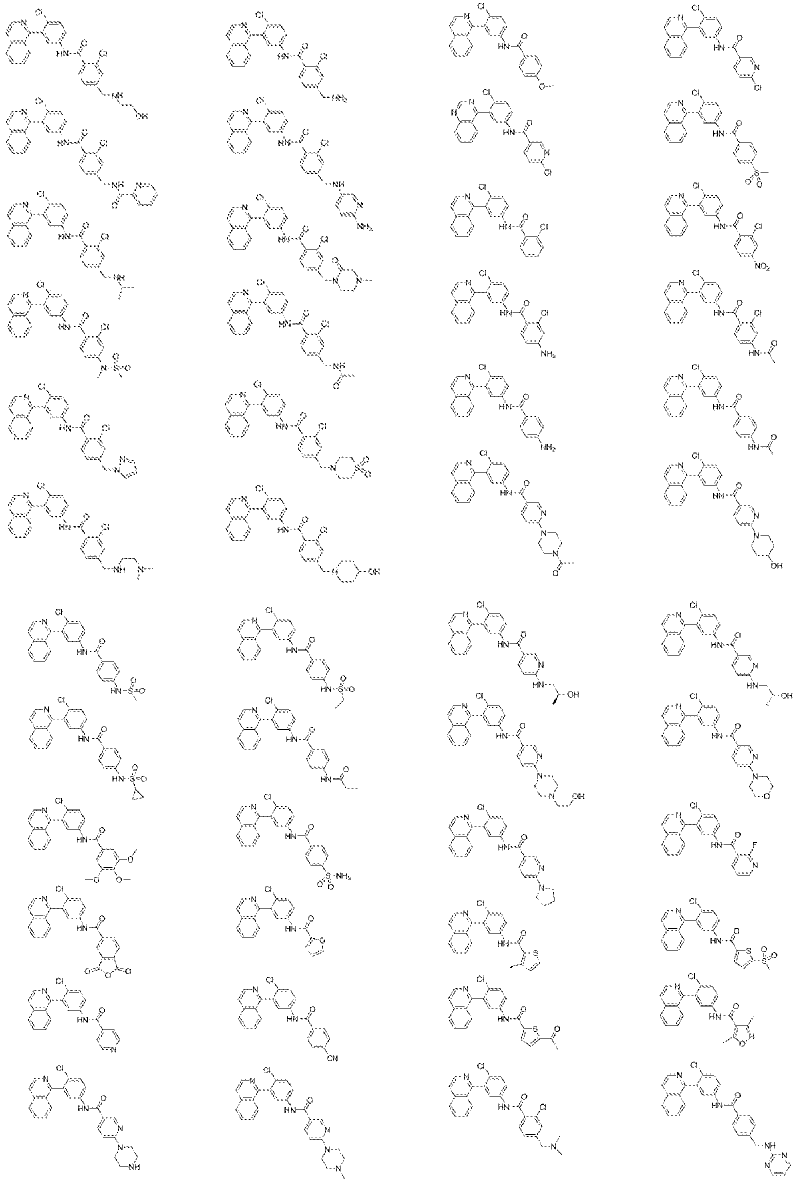

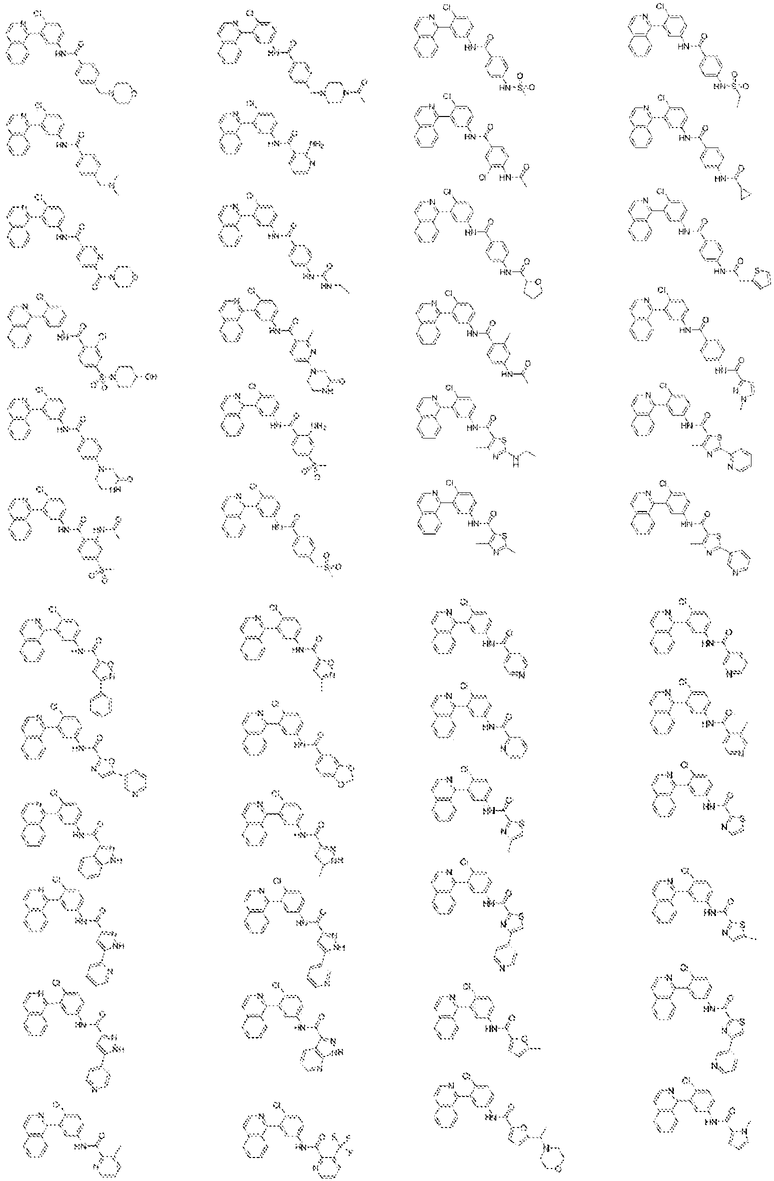

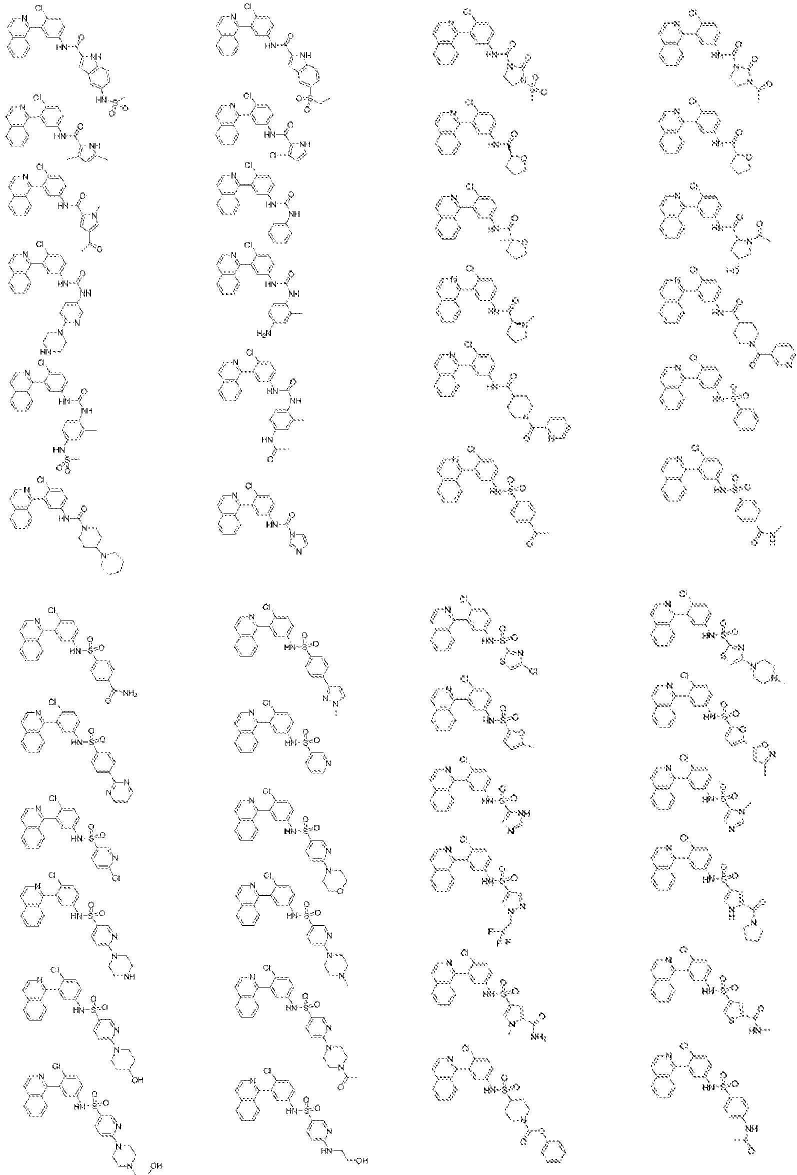

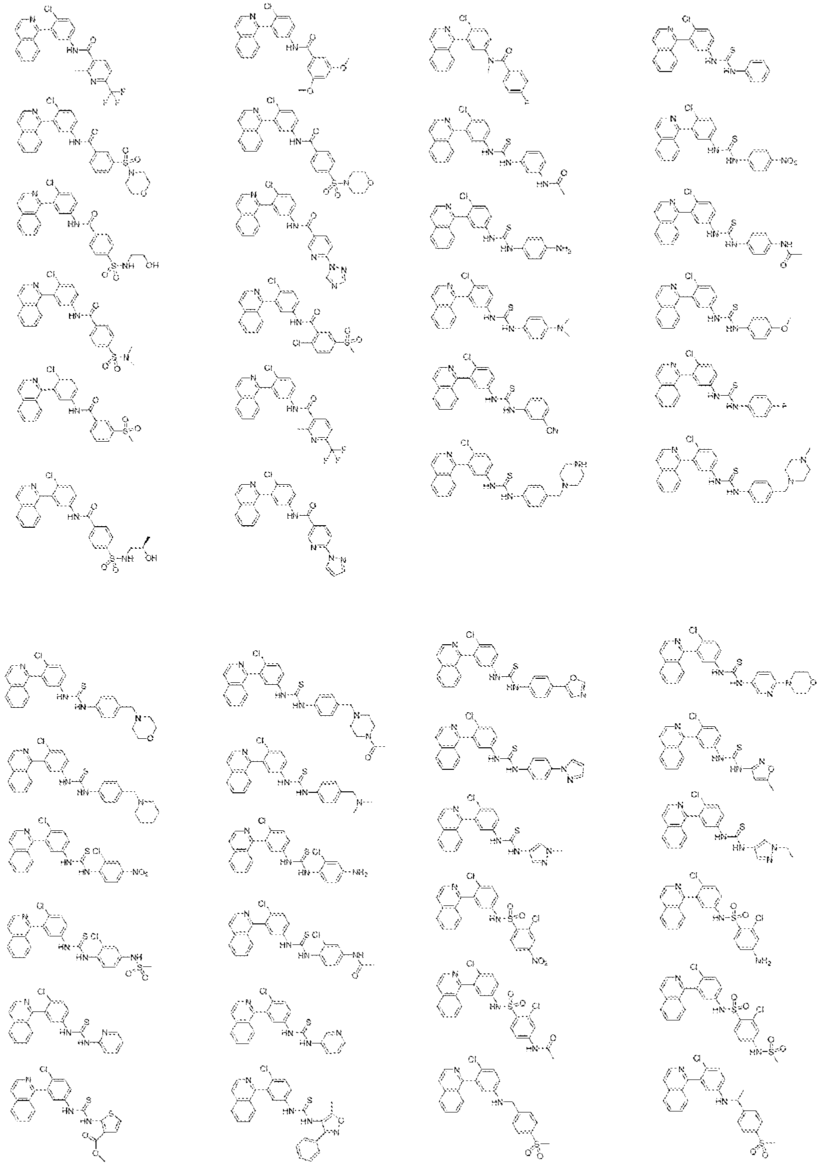

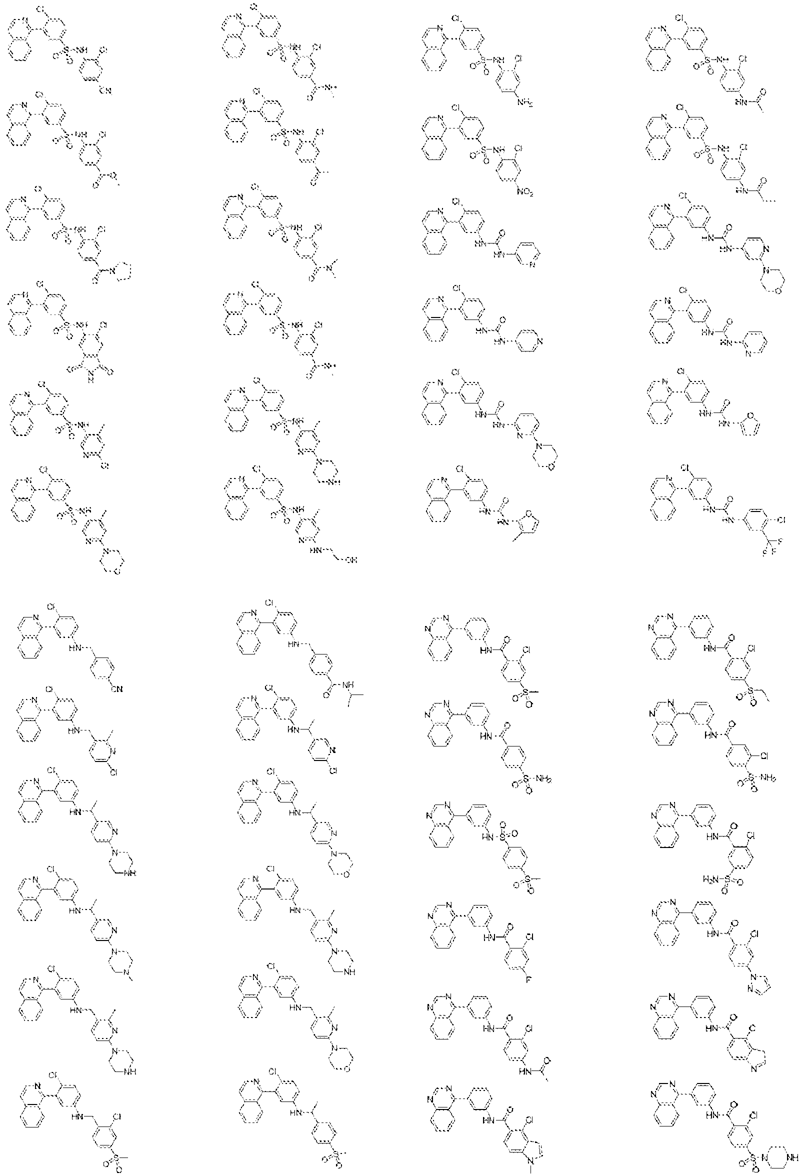

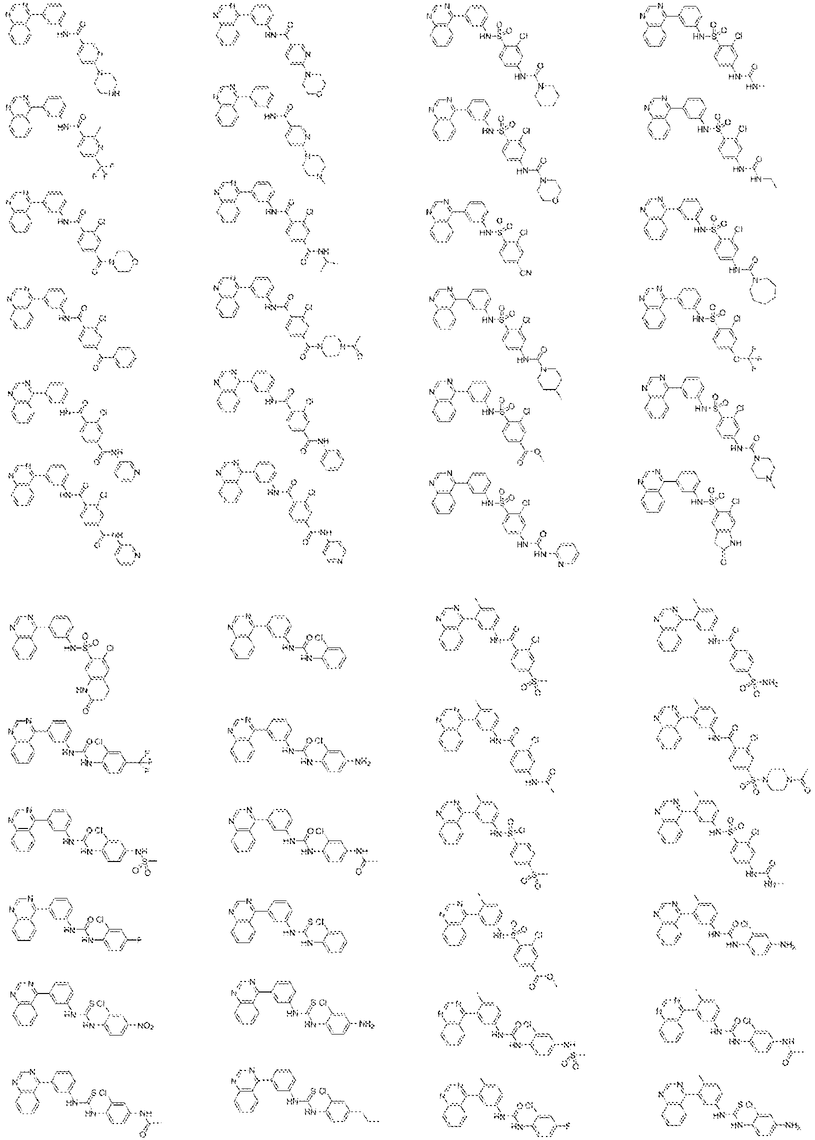

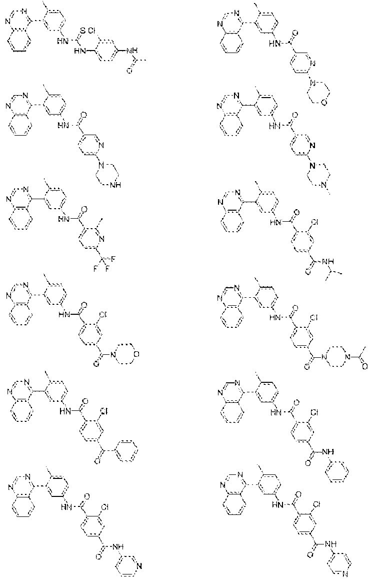

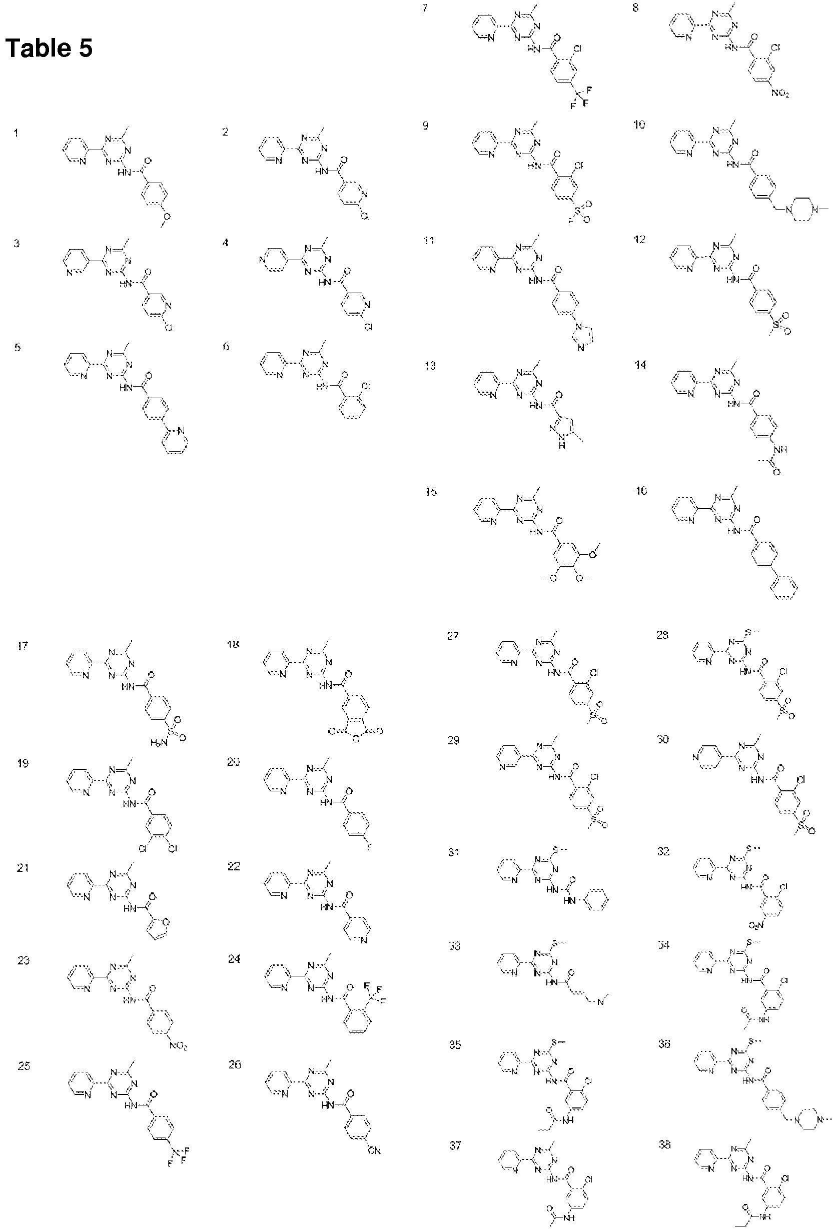

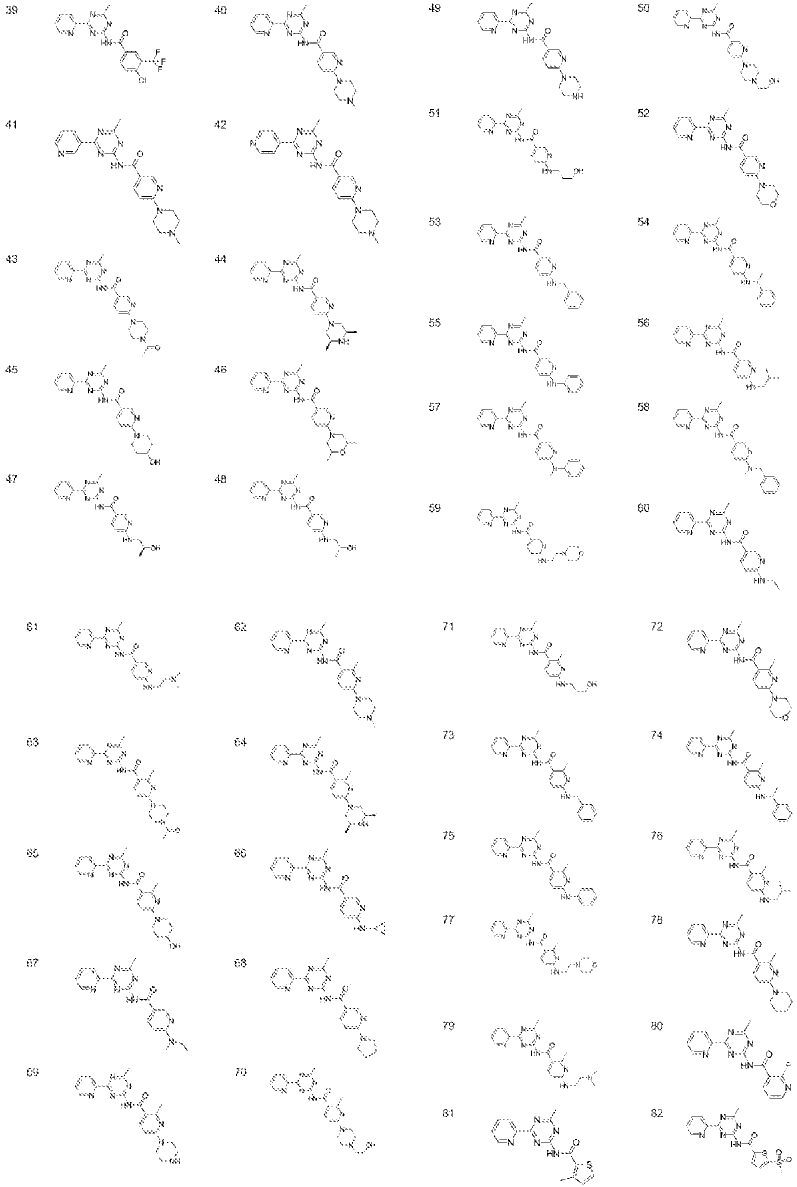

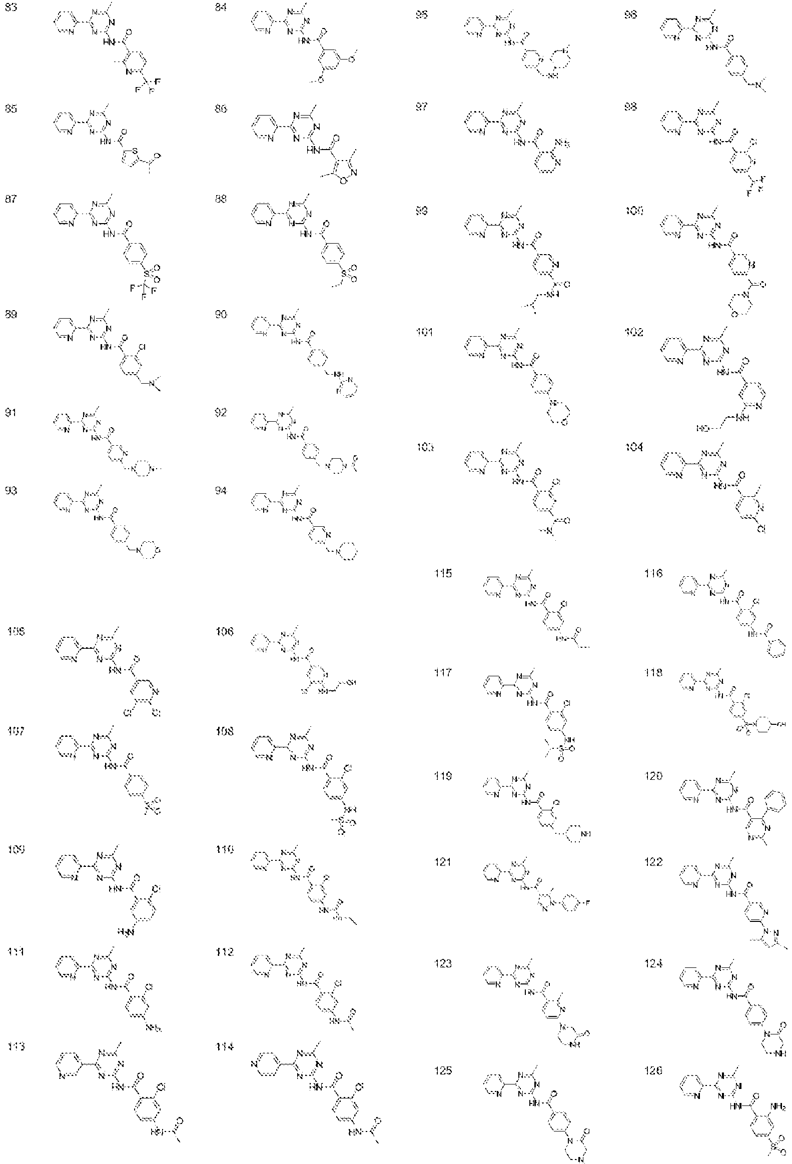

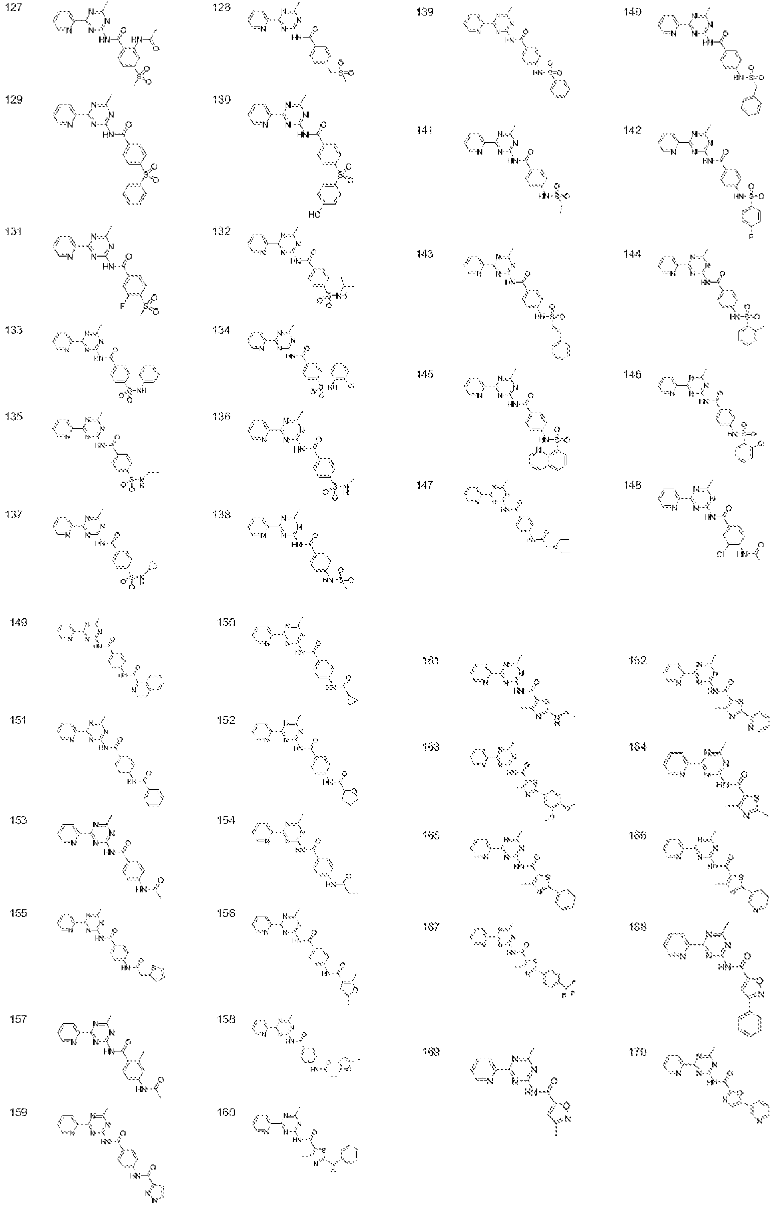

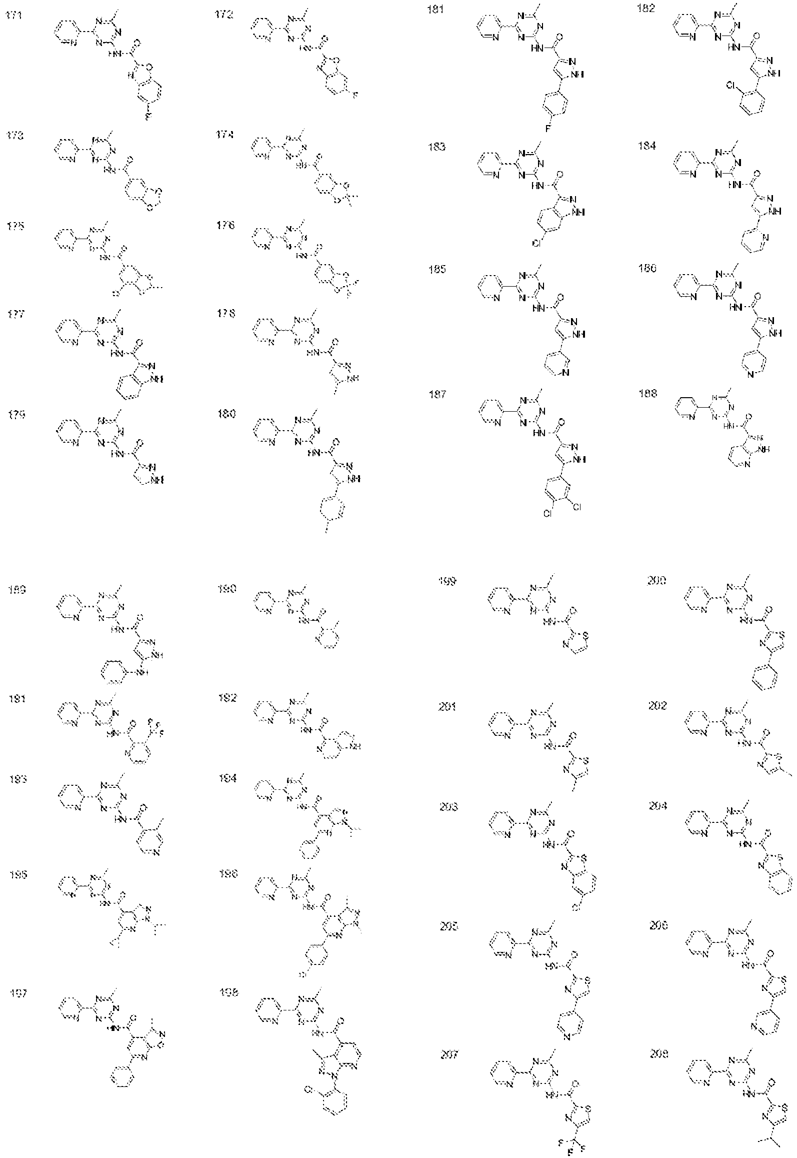

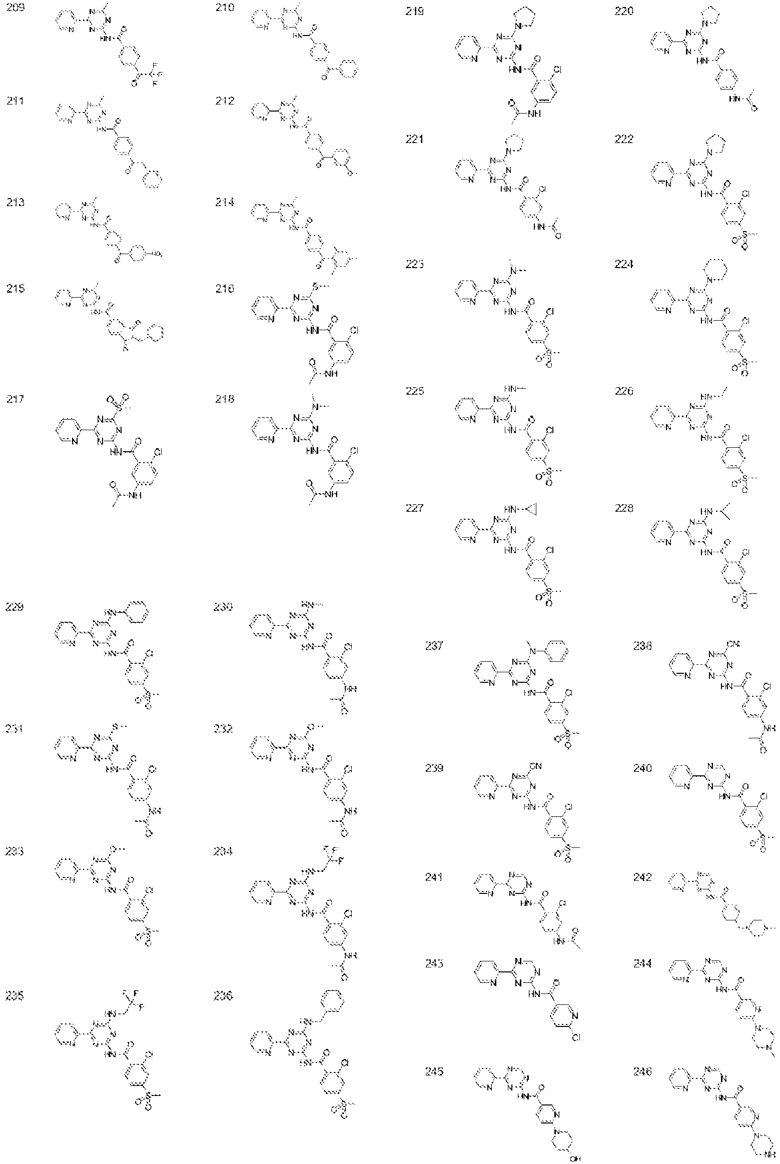

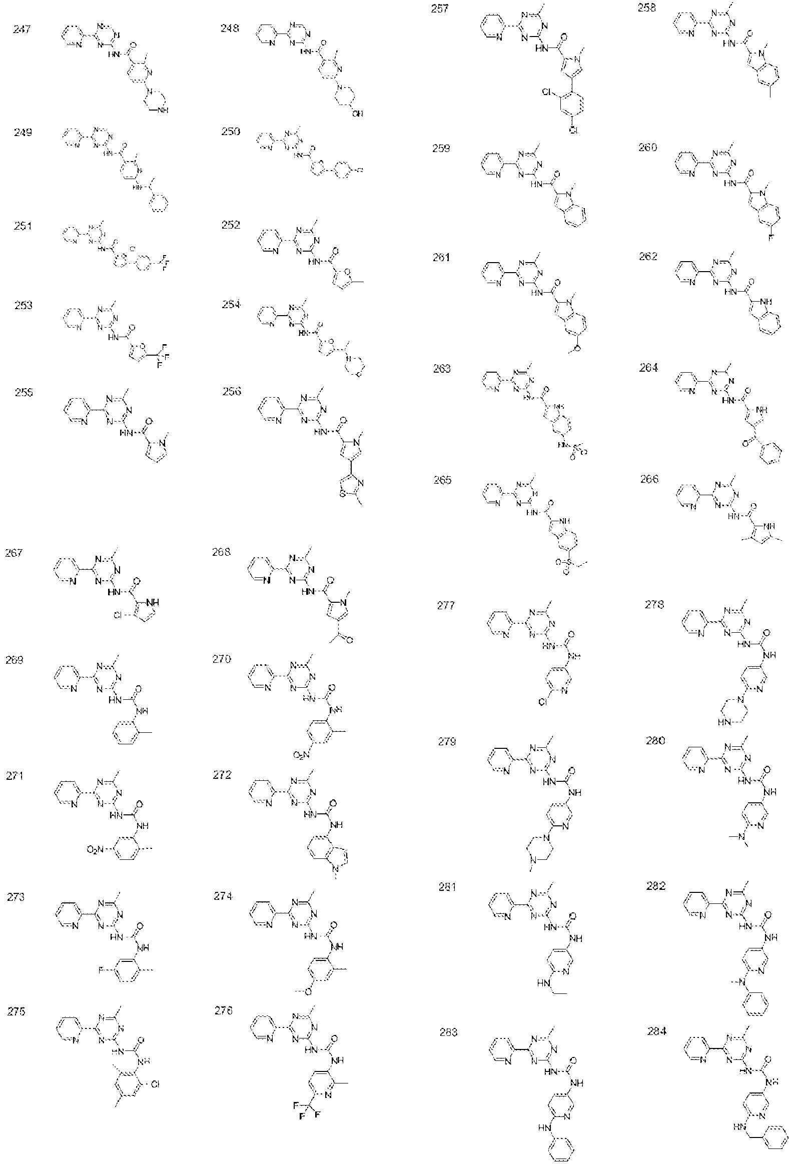

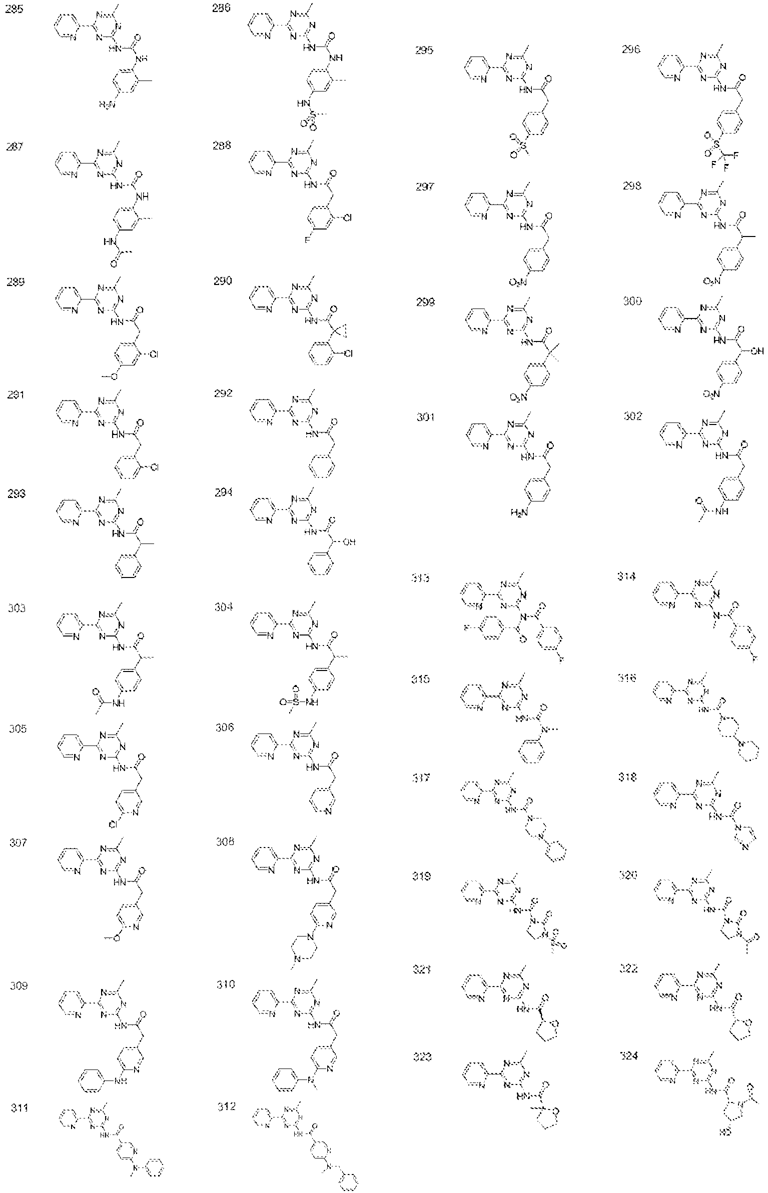

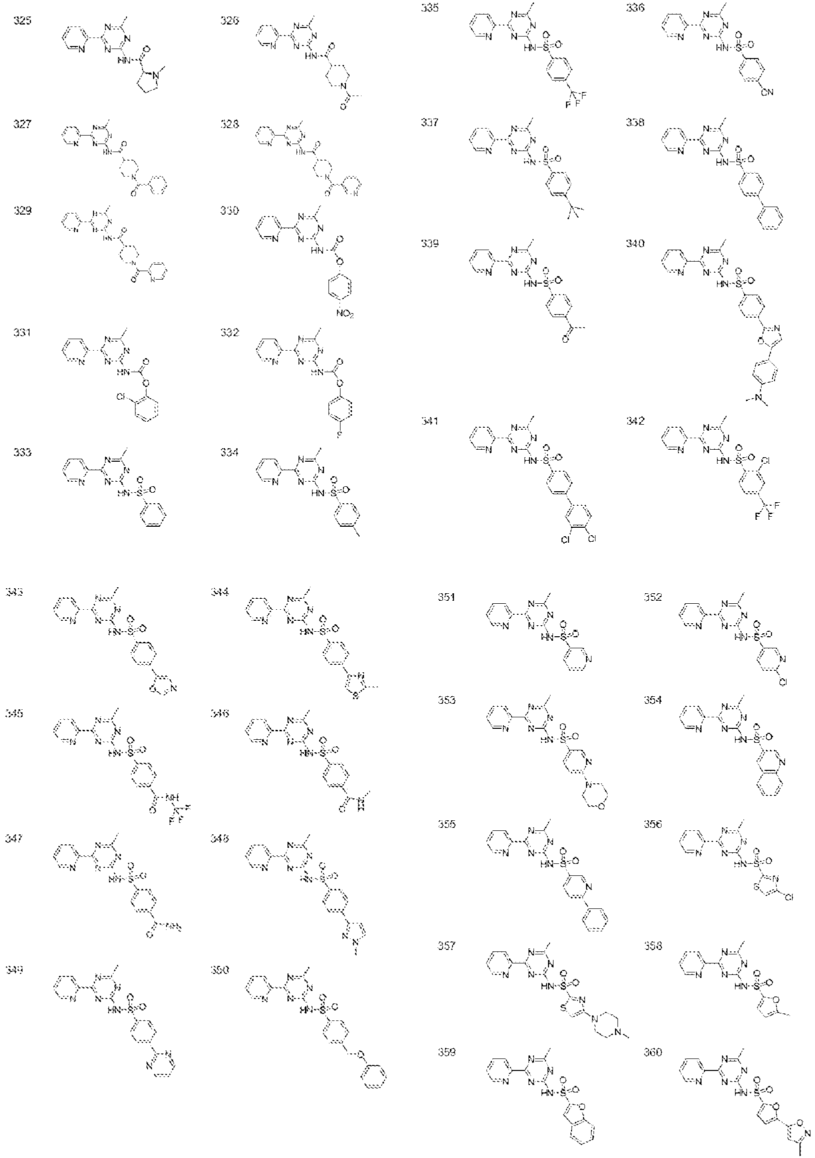

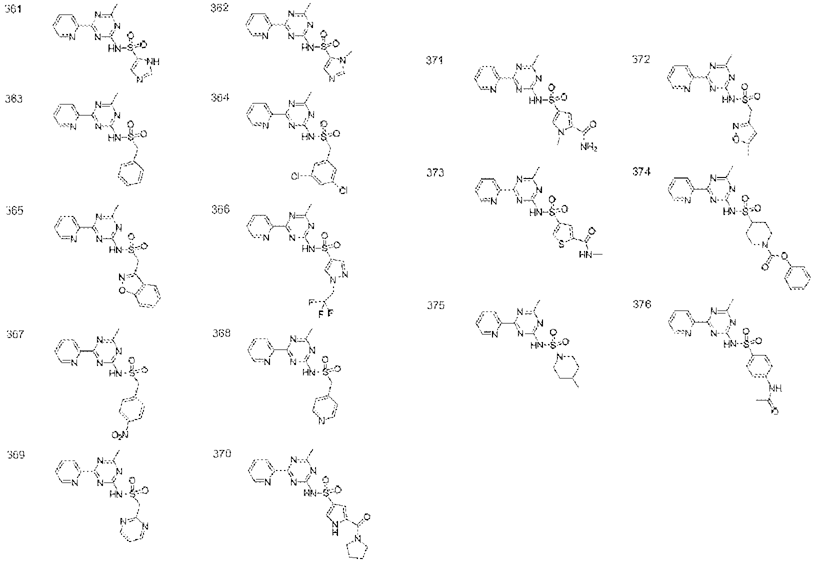

## Claims

1. A composition comprising nanoparticles comprising a taxane and an albumin for use in a method of treating a cancer in an individual, wherein the method further comprises administering a hedgehog inhibitor.

2. The composition for use according to claim 1, wherein said hedgehog inhibitor inhibits the activity of Smoothened.

3. The composition for use according to claim 1 or 2, wherein said hedgehog inhibitor is a cyclopamine or derivative thereof.

4. The composition for use according to claim 1, wherein said hedgehog inhibitor is selected from the group consisting of GDC-0449, XL139, IPI926, IPI609, and LDE225.

5. The composition for use according to any one of claims 1-4, further comprising administering an effective amount of gemcitabine.

6. The composition for use according to any one of claims 1-5, wherein the cancer is selected from the group consisting of basal cell carcinoma, medulloblastoma, glioblastoma, multiple myeloma, chronic myelogenous leukemia (CML), acute myelogenous leukemia, pancreatic cancer, lung cancer (small cell lung cancer and non-small cell lung cancer), esophageal cancer, stomach cancer, biliary cancer, prostate cancer, liver cancer, hepatocellular cancer, gastrointestinal cancer, gastric cancer, ovarian cancer, and bladder cancer.

7. The composition for use according to any one of claims 1-6, wherein the method comprises simultaneously administering the composition comprising nanoparticles comprising a taxane and an albumin and the hedgehog inhibitor.

8. The composition for use according to any one of claims 1-6, wherein the method comprises sequentially administering the composition comprising nanoparticles comprising a taxane and an albumin and the hedgehog inhibitor.

9. The composition for use according to any one of claims 1-8, wherein the taxane is paclitaxel or docetaxel.

10. The composition for use according to any one of claims 1-9, wherein the average diameter of the nanoparticles is no greater than about 200 nm.

11. The composition for use according to any one of claims 1-10, wherein the weight ratio of the albumin and the taxane in the nanoparticles is less than about 1:1 to 9:1.

12. The composition for use according to any one of claims 1-11, wherein the composition comprising nanoparticles comprising a taxane and an albumin is substantially free of Cremophor.

13. The composition for use according to any one of claims 1-12, wherein the individual is a human.

14. The composition for use according to any one of claims 1-13, wherein the hedgehog inhibitor is administered orally.

15. The composition for use according to any one of claims 1-14, wherein the hedgehog inhibitor is administered prior to the administration of the composition comprising nanoparticles comprising a taxane and an albumin or after the administration of the composition comprising nanoparticles comprising a taxane and an albumin.

## Patentansprüche

1. Zusammensetzung umfassend Nanopartikel umfassend ein Taxan und ein Albumin für die Verwendung in einem Verfahren zur Behandlung von Krebs in einem Individuum, wobei das Verfahren weiter das Verabreichen eines Hedgehog Inhibitors umfasst.

2. Zusammensetzung für die Verwendung gemäß Anspruch 1, wobei der Hedgehog Inhibitor die Aktivität von Smoothened inhibiert.

3. Zusammensetzung für die Verwendung gemäß Anspruch 1 oder 2, wobei der Hedgehog Inhibitor ein Cyclopamin oder Derivat davon ist.

4. Zusammensetzung für die Verwendung gemäß Anspruch 1, wobei der Hedgehog Inhibitor ausgewählt ist aus der Gruppe bestehend aus GDC-0449, XL139, IPI926, IPI609 und LDE225.

5. Zusammensetzung für die Verwendung gemäß einem der Ansprüche 1-4, weiter umfassend das Verabreichen einer wirksamen Mengen von Gemcitabin.

6. Zusammensetzung für die Verwendung gemäß einem der Ansprüche 1-5, wobei der Krebs ausgewählt ist aus der Gruppe bestehend aus basalem Zellkarzinom, Medulloblastom, Glioblastom, multiplem Myelom, chronischer myelogener Leukämie (CML), akuter myelogener Leukämie, Bauchspeicheldrüsenkrebs, Lungenkrebs (kleinzelliges Bronchialkarzinom und nicht-kleinzelliges Bronchialkarzinom), Speiseröhrenkrebs, Magenkarzinom, Gallenkrebs, Prostatakrebs, Leberkrebs, Leberzellkrebs, Magen-Darm-Krebs, Magenkrebs, Eierstockkrebs und Blasenkrebs.

7. Zusammensetzung für die Verwendung gemäß einem der Ansprüche 1-6, wobei das Verfahren das gleichzeitige Verabreichen der Nanopartikel umfassend Taxan und Albumin und des Hedgehog Inhibitors umfasst.

8. Zusammensetzung für die Verwendung gemäß einem der Ansprüche 1-6, wobei das Verfahren das sequentielle Verabreichen der Nanopartikeln umfassend Taxan und Albumin und den Hedgehog Inhibitor umfasst.

9. Zusammensetzung für die Verwendung gemäß einem der Ansprüche 1-8, wobei das Taxan Paclitaxel oder Docetaxel ist.

10. Zusammensetzung für die Verwendung gemäß einem der Ansprüche 1-9, wobei der durchschnittliche Durchmesser der Nanopartikel in der Zusammensetzung nicht größer als ungefähr 200 nm ist.

11. Zusammensetzung für die Verwendung gemäß einem der Ansprüche 1-10, wobei das Gewichtsverhältnis des Albumins und des Taxans in der Nanopartikelzusammensetzung kleiner als ungefähr 1:1 bis 9:1 ist.

12. Zusammensetzung für die Verwendung gemäß einem der Ansprüche 1-11, wobei die Nanopartikelzusammensetzung im Wesentlichen frei von Cremophor ist.

13. Zusammensetzung für die Verwendung gemäß einem der Ansprüche 1-12, wobei das Individuum ein Mensch ist.

14. Zusammensetzung für die Verwendung gemäß einem der Ansprüche 1-13, wobei der Hedgehog Inhibitor oral verabreicht wird.

15. Zusammensetzung für die Verwendung gemäß einem der Ansprüche 1-14, wobei der Hedgehog Inhibitor vor der Verabreichung der Nanopartikelzusammensetzung oder nach der Verabreichung der Nanopartikelzusammensetzung verabreicht wird.

## Revendications

1. Composition comprenant des nanoparticules comprenant un taxane et une albumine pour son utilisation dans un procédé de traitement d'un cancer dans un individu, le procédé comprenant en outre l'administration d'un inhibiteur de la voie Hedgehog.

2. Composition pour son utilisation selon la revendication 1, dans laquelle ledit inhibiteur de la voie Hedgehog inhibe l'activité de la protéine Smoothened.

3. Composition pour son utilisation selon la revendication 1 ou 2, dans laquelle ledit inhibiteur de la voie Hedgehog est une cyclopamine ou un de ses dérivés.

4. Composition pour son utilisation selon la revendication 1, dans laquelle ledit inhibiteur de la voie Hedgehog est choisi parmi le groupe constitué par GDC-0449, XL139, IPI926, IPI609 et LDE225.

5. Composition pour son utilisation selon l'une quelconque des revendications 1 à 4, comprenant en outre l'administration d'une quantité efficace de gemcitabine.

6. Composition pour son utilisation selon l'une quelconque des revendications 1 à 5, dans laquelle le cancer est choisi parmi le groupe constitué par le carcinome basocellulaire, le médulloblastome, le glioblastome, le myélome multiple, la leucémie myélogène chronique (CML), la leucémie myélogène aiguë, le cancer du pancréas, le cancer du poumon (cancer du poumon à petites cellules et cancer du poumon non à petites cellules), le cancer de l'oesophage, le cancer de l'estomac, le cancer biliaire, le cancer de la prostate, le cancer du foie, le cancer hépatocellulaire, le cancer gastrointestinal, le cancer gastrique, le cancer des ovaires et le cancer de la vessie.

7. Composition pour son utilisation selon l'une quelconque des revendications 1 à 6, dans laquelle le procédé comprend l'administration simultanée de nanoparticules comprenant du taxane et de l'albumine et de l'inhibiteur de la voie Hedgehog.

8. Composition pour son utilisation selon l'une quelconque des revendications 1 à 6, dans laquelle le procédé comprend l'administration séquentielle de nanoparticules de taxane comprenant de l'albumine et de l'inhibiteur de la voie Hedgehog.

9. Composition pour son utilisation selon l'une quelconque des revendications 1 à 8, dans laquelle le taxane est le paclitaxel ou le docetaxel.

10. Composition pour son utilisation selon l'une quelconque des revendications 1 à 9, dans laquelle le diamètre moyen des nanoparticules dans la composition n'est pas supérieur à environ 200 nm.

11. Composition pour son utilisation selon l'une quelconque des revendications 1 à 10, dans laquelle le rapport pondéral de l'albumine et du taxane dans la composition de nanoparticules est inférieur à environ 1:1 à 9:1.

12. Composition pour son utilisation selon l'une quelconque des revendications 1 à 11, dans laquelle la composition de nanoparticules est essentiellement exempte de Chremophor.

13. Composition pour son utilisation selon l'une quelconque des revendications 1 à 12, dans laquelle l'individu est un être humain.

14. Composition pour son utilisation selon l'une quelconque des revendications 1 à 13, dans laquelle l'inhibiteur de la voie Hedgehog est administré par voie orale.

15. Composition pour son utilisation selon l'une quelconque des revendications 1 à 14, dans laquelle l'inhibiteur de la voie Hedgehog est administré avant l'administration de la composition de nanoparticules ou après l'administration de la composition de nanoparticules.
